# EUROPEAN PATENT APPLICATION

(11) **EP 4 516 778 A1**
(43) Date of publication of application: **05.03.2025**
(21) Application number: 23796339.2
(22) Date of filing: 24.04.2023
(51) Int. Cl.: C07D 239/26, A61K 31/415, A61K 31/421, A61K 31/426, A61K 31/437, A61K 31/44, A61K 31/4965, A61K 31/505, A61P 11/00, A61P 13/12, A61P 19/02, A61P 35/00, A61P 35/02, A61P 43/00, C07D 213/61, C07D 213/65, C07D 213/82, C07D 231/12, C07D 239/42

(54) **COMPOUND SERVING AS DDR1 KINASE INHIBITOR, AND MEDICINE**

(30) Priority: 25.04.2022 JP 2022071524
(71) Applicant: Nippon Shinyaku Co., Ltd., Kyoto-shi Kyoto 601-8550 (JP)
(72) Inventor: ZAIMOKU, Hisaaki, Kyoto-shi, Kyoto 601-8550 (JP); ASADA, Junshi, Kyoto-shi, Kyoto 601-8550 (JP); HASHIMOTO, Kosuke, Kyoto-shi, Kyoto 601-8550 (JP); HONDA, Yohei, Kyoto-shi, Kyoto 601-8550 (JP); TSUZUKI, Yota, Kyoto-shi, Kyoto 601-8550 (JP); KOSUGI, Keiji, Kyoto-shi, Kyoto 601-8550 (JP)
(74) Representative: Vossius & Partner Patentanwälte Rechtsanwälte mbB
(86) International application number: PCT/JP2023/016163
(87) International publication number: WO 2023/210599

(57) **Abstract**

The present invention provides a compound represented by general formula [1] and having a DDR1 kinase inhibitory activity, a pharmaceutically acceptable salt of the compound, or a solvate of the compound or the pharmaceutically acceptable salt (in the formula, R¹ represents a hydrogen atom, a halogen atom, or an optionally substituted alkyl group; Het represents a 5- to 10-membered heteroaryl group; L¹ represents -CO-NH- or -CR^{a}=CR^{b}-; X represents C-R^{c}; R² represents a C₁-C₆ haloalkoxy group or a C₁-C₆ haloalkyl group; R³, R⁴ and carbon atoms to which these residues are bound together form an optionally substituted C₃-C₆ cycloalkyl group or an optionally substituted heterocycloalkyl group).

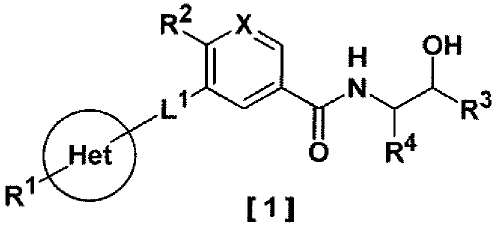

## Description

### Technical Field

The present invention relates to compounds as a DDR1 kinase inhibiting agent and medicines.

### Background

Discoidin Domain Receptor 1 (also referred to as DDR1) is a tyrosine kinase receptor belonging to the discoidin domain receptor family and is known to be involved in cell proliferation and differentiation as well as migration, invasion and adhesion. It is mainly expressed on epithelial cells in lung, kidney, mammary gland and gastrointestinal tract.

DDR1 transduces collagen signals into the cell by binding to the ligand collagen via the discoidin domain. DDR1 is unique in that it recognizes not only fibrous collagen such as type I collagen but also basement membrane collagen such as type IV collagen, as such ligand. Once DDR1 binds to collagen, specific tyrosine residues in the intracellular kinase domain of the receptor are phosphorylated, and binding of molecules having Src homology 2 and phosphotyrosine binding domains activates various downstream signaling pathways.

Activation of DDR1 is involved in various renal diseases. Activation of DDR1 is involved in pathologies of renal diseases such as Alport syndrome caused by mutations of type IV collagen gene (Non-Patent Documents 1 and 2), IgA nephropathy (Non-Patent Document 3), Goodpasture syndrome (Non-Patent Document 4), lupus nephritis (Non-Patent Document 4), ANCA (Anti-neutrophil cytoplasmic antibody)-related nephritis (Non-Patent Document 5), and diabetic nephropathy (Non-Patent Document 6).

Activation of DDR1 has also been reported to be involved in various fibrosis diseases. For example, pulmonary fibrosis (Non-Patent Document 7) and liver cirrhosis (Non-Patent Document 8) are known as such diseases.

Furthermore, DDR1 has been reported to be involved in the function of Th17 cells, which are involved in autoimmune diseases and allergies (Non-Patent Document 9). Th17 cells are involved in the pathogenesis of diseases such as rheumatoid arthritis, multiple sclerosis, ulcerative colitis and Crohn's disease.

It has also been reported to be involved in pathogenesis of cancer-related diseases. For example, acute myeloid leukemia (Non-Patent Document 10), acute lymphocytic leukemia (Non-Patent Document 11), chronic lymphocytic leukemia (Non-Patent Document 12), Hodgkin's lymphoma (Non-Patent Document 13), glioma (Non-Patent Document 14), non-small cell lung cancer (Non-Patent Document 15), breast cancer (Non-Patent Document 16), ovarian cancer (Non-Patent Document 17), prostate cancer (Non-Patent Document 18) and colorectal cancer (Non-Patent Document 19) are known as such diseases.

DDR1 has also been reported to be involved in other pathological conditions such as systemic lupus erythematosus (Non-Patent Document 5), osteoarthritis (Non-Patent Document 20), heparin-induced thrombocytopenia (Non-Patent Document 21), atherosclerosis (Non-Patent Document 22) and vitiligo vulgaris (Non-Patent Document 23).

Most of the treatments for these diseases are limited to symptomatic treatment, suppression of disease progression, and improvement of quality of life, and efficient treatment is extremely difficult. Therefore, DDR1 inhibitors can provide a new treatment for these DDR1-related diseases.

### Prior Art Document

### Non-Patent Document

Non-Patent Document 1: Gross et al., Matrix Biol. 2010 Jun;29(5):346-56.
Non-Patent Document 1: Richter et al., ACS Chem Biol. 2019 Jan 18;14(1):37-49.
Non-Patent Document 1: Hahn et al., Int J Mol Med. 2010 May;25(5):785-91.
Non-Patent Document 1: Kerroch et al., FASEB J. 2012 Oct;26(10):4079-91.
Non-Patent Document 1: Moll et al., J Transl Med. 2018 Jun 1;16(1):148.
Non-Patent Document 1: Salem et al., J Am Soc Nephrol. 2019 Oct;30(10):2000-2016.
Non-Patent Document 1: Matsuyama et al., J Immunol. 2005 May 15;174(10):6490-8.
Non-Patent Document 1: Shackel et al., Am J Pathol. 2002 Feb;160(2):641-54.
Non-Patent Document 1: Azreq et al., Oncotarget. 2016 Jul 19;7(29):44975-44990.
Non-Patent Document 1: Favreau et al., Cancer Med. 2014 Apr;3(2):265-72.
Non-Patent Document 1: Guo et al., Exp Ther Med. 2019 Mar;17(3):1593-1600.
Non-Patent Document 1: Barisione et al., Blood Cancer J. 2017 Jan 6;6(1):e513.
Non-Patent Document 1: Cader et al., Blood. 2013 Dec 19;122(26):4237-45.
Non-Patent Document 1: Ram et al., J Neurooncol. 2006 Feb;76(3):239-48.
Non-Patent Document 1: Miao et al., Med Oncol. 2013;30(3):626.
Non-Patent Document 1: Zhong et al., Oncol Rep. 2019 Dec;42(6):2844-2854.
Non-Patent Document 1: Heinzelmann-Schwarz et al., Clin Cancer Res. 2004 Jul 1;10(13):4427-36.
Non-Patent Document 1: Azizi et al., J Cell Physiol. 2019 Nov;234(11):19539-19552.
Non-Patent Document 1: Le et al., Front Cell Dev Biol. 2020 Jun 3;8:412.
Non-Patent Document 1: Han et al., PLoS One. 2020 Apr 24;15(4):e0231501.
Non-Patent Document 1: Witten et al., J Mol Med. 2018 Aug;96(8):765-775.
Non-Patent Document 1: Franco et al., Circ Res. 2008 May 23;102(10):1202-11.
Non-Patent Document 1: Almasi-Nasrabadi et al., Gene. 2019 Jun 5;700:17-22

### Summary of the Invention

### Problem to be Solved by the Invention

The invention provides a compound having DDR1 kinase inhibitory activity, or a pharmaceutically acceptable salt thereof, or a solvate thereof.

### Means for Solving the Problem

The inventors have discovered that the compounds described below, or pharmaceutically acceptable salts or solvates thereof, have excellent DDR1 kinase inhibitory activity, and have accomplished the invention.

The present invention may include the following Items (1) to (16).

### (Item 1)

A compound of the formula [1]: 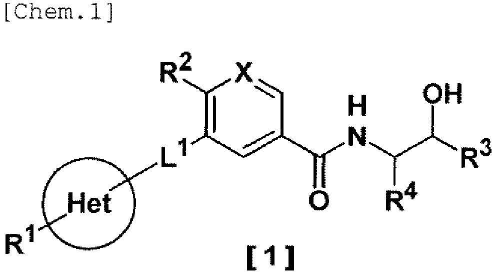 or a pharmaceutically acceptable salt thereof, or a solvate thereof, wherein
R¹ is one or two groups selected from the group consisting of hydrogen, halogen, an optionally-substituted alkyl, an optionally-substituted amino, an optionally-substituted alkoxy, an optionally-substituted aryl, haloalkyl, cycloalkyl, an optionally-substituted sulfonyl, cyano, an optionally-substituted carbamoyl, heteroaryl and dialkylphosphoryl;
Het is a 5- to 10-membered heteroaryl;
L¹ is a bond, -CO-NH-, -CR^{a}=CR^{b}- or -C≡C-, and the carbonyl group (C=O) in -CO-NH- is bonded to Het and -CR^{a} in -CR^{a}=CR^{b}-is bonded to Het;
R^{a} is hydrogen or halogen;
R^{b} is hydrogen or halogen;
X is N or C-R^{c} wherein R^{c} is hydrogen or halogen;
R² is hydrogen, ethyl, C₁-C₆ haloalkoxy or C₁-C₆ haloalkyl; and
R³ is hydrogen, and R⁴ is hydrogen, an optionally-substituted phenyl or an optionally-substituted alkyl, or
R³ and R⁴ are taken together with the carbon atoms to which they are attached to form an optionally-substituted C₃-C₈ cycloalkyl.

### (Item 2)

The compound or a pharmaceutically acceptable salt thereof, or a solvate thereof, according to Item 1
wherein
R¹ is one or two groups selected from the group consisting of hydrogen, halogen, an optionally-substituted alkyl, an optionally-substituted amino, an optionally-substituted alkoxy, an optionally-substituted aryl, haloalkyl and an optionally-substituted carbamoyl;
Het is a 5- to 10-membered heteroaryl;
L¹ is a bond, -CR^{a}=CR^{b}-, -CO-NH- or -C=C-, and the carbonyl group (C=O) in -CO-NH- is bonded to Het and -CR^{a} in -CR^{a}=CR^{b}-is bonded to Het;
R^{a} is hydrogen or halogen;
R^{b} is hydrogen or halogen;
X is N or C-R^{c} wherein R^{c} is hydrogen or halogen;
R² is ethyl, C₁-C₆ haloalkoxy or C₁-C₆ haloalkyl; and
R³ is hydrogen, and R⁴ is hydrogen, an optionally-substituted phenyl or an optionally-substituted alkyl, or
R³ and R⁴ are taken together with the carbon atoms to which they are attached to form an optionally-substituted C₃-C₈ cycloalkyl.

### (Item 3)

The compound or a pharmaceutically acceptable salt thereof, or a solvate thereof, according to Item 1
wherein
R¹ is one or two groups selected from the group consisting of hydrogen, an optionally-substituted alkyl, an optionally-substituted amino and an optionally-substituted carbamoyl;
Het is a 5- to 10-membered heteroaryl;
L¹ is a bond, -CR^{a}=CR^{b}- or -C=C-, and -CR^{a} in -CR^{a}=CR^{b}- is bonded to Het;
R^{a} is hydrogen;
R^{b} is halogen;
X is N or C-R^{c} wherein R^{c} is hydrogen;
R² is C₁-C₆ haloalkoxy or C₁-C₆ haloalkyl; and
R³ and R⁴ are taken together with the carbon atoms to which they are attached to form an optionally-substituted C₃-C₈ cycloalkyl.

### (Item 4)

The compound or a pharmaceutically acceptable salt thereof, or a solvate thereof, according to Item 1
wherein
R¹ is one or two groups selected from the group consisting of hydrogen, alkyl, amino and carbamoyl;
Het is pyrimidinyl, pyridyl, pyrazinyl, pyrazolyl, pyridinyl, imidazo[1,5-a]pyridyl, oxazolyl, thiazolyl or pyrazyl;
L¹ is a bond, -CR^{a}=CR^{b}- or -C=C-, and -CR^{a} in -CR^{a}=CR^{b}- is bonded to Het;
R^{a} is hydrogen;
R^{b} is halogen;
X is N or C-R^{c} wherein R^{c} is hydrogen;
R² is C₁-C₆ fluoroalkoxy, C₁-C₆ difluoroalkoxy, C₁-C₆ trifluoroalkoxy, C₁-C₆ fluoroalkyl, C₁-C₆ difluoroalkyl or C₁-C₆ trifluoroalkyl; and
R³ and R⁴ are taken together with the carbon atoms to which they are attached to form an optionally-substituted cyclopentyl, an optionally-substituted cyclohexyl, or an optionally-substituted spiro[2.4]heptanyl.

### (Item 5)

The compound or a pharmaceutically acceptable salt thereof, or a solvate thereof, according to Item 1
wherein
R¹ is a group selected from the group consisting of hydrogen, alkyl, amino and carbamoyl;
Het is pyrimidinyl, pyridyl, pyrazinyl, pyrazolyl, pyridinyl, imidazo[1,5-a]pyridyl, oxazolyl, thiazolyl or pyrazyl;
L¹ is a bond, -CR^{a}=CR^{b}- or -C≡C-, and -CR^{a} in -CR^{a}=CR^{b}- is bonded to Het;
R^{a} is hydrogen;
R^{b} is halogen;
X is N or C-R^{c} wherein R^{c} is hydrogen;
R² is C₁-C₆ fluoroalkoxy, C₁-C₆ difluoroalkoxy, C₁-C₆ trifluoroalkoxy, C₁-C₆ fluoroalkyl, C₁-C₆ difluoroalkyl or C₁-C₆ trifluoroalkyl; and
R³ and R⁴ are taken together with the carbon atoms to which they are attached to form cyclopentyl substituted with one or two groups selected from the group consisting of halogen and haloalkoxy, cyclohexyl substituted with one or two groups selected from the group consisting of halogen and haloalkoxy, or spiro[2.4]heptanyl substituted with one or two groups selected from the group consisting of halogen and haloalkoxy.

### (Item 6)

The compound according to Item 1 selected from the group consisting of the following compounds (1) to (47):
(1) 4-(difluoromethoxy)-N-[(1S,2S,4S)-2-hydroxy-4-(trifluoromethoxy)cyclopentyl]-3-[(pyrimidin-5-yl)ethynyl]benzamide,
(2) 4-(difluoromethoxy)-3-[2-(5-fluoropyridin-3-yl)ethynyl]-N-[(1S,2S,4S)-2-hydroxy-4-(trifluoromethoxy)cyclopentyl]benzamide,
(3) 3-[2-(2-aminopyrimidin-5-yl)ethynyl]-4-(difluoromethoxy)-N-[(1S,2S,4S)-2-hydroxy-4-(trifluoromethoxy)cyclopentyl]benzamide,
(4) 3-[2-(5-aminopyrazin-2-yl)ethynyl]-4-(difluoromethoxy)-N-[(1S,2S,4S)-2-hydroxy-4-(trifluoromethoxy)cyclopentyl]benzamide,
(5) 3-[2-(2-amino-4-methoxypyrimidin-5-yl)ethynyl]-4-(difluoromethoxy)-N-[(1S,2S,4S)-2-hydroxy-4-(trifluoromethoxy)cyclopentyl]benzamide,
(6) 4-(difluoromethoxy)-N-[(1S,2S,4S)-2-hydroxy-4-(trifluoromethoxy)cyclopentyl]-3-[2-(1-methyl-1H-pyrazol-4-yl)ethynyl]benzamide,
(7) 3-[2-(5-aminopyrazin-2-yl)ethynyl]-4-(difluoromethoxy)-N-[(2S)-1-hydroxy-4-(trifluoromethoxy)butan-2-yl)benzamide,
(8) 3-[2-(2-aminopyrimidin-5-yl)ethynyl]-4-(difluoromethoxy)-N-[(1S)-1-(3-fluorophenyl)-2-hydroxyethyl]benzamide,
(9) 3-[2-(2-aminopyrimidin-5-yl)ethynyl]-4-(difluoromethoxy)-N-[(2S)-1-hydroxy-3-(4-methoxyphenyl)propan-2-yl]benzamide,
(10) 3-[(1Z)-2-(5-aminopyrazin-2-yl)-2-fluoroethenyl]-4-(difluoromethoxy)-N-[(1S,2S,4S)-2-hydroxy-4-(trifluoromethoxy)cyclopentyl]benzamide,
(11) 5-[(1Z)-2-(2-aminopyrimidin-5-yl)-2-fluoroethenyl]-6-ethyl-N-[(1S,2S,4S)-2-hydroxy-4-(trifluoromethoxy)cyclopentyl]pyridine-3-carboxamide,
(12) 3-[(1Z)-2-(2-aminopyrimidin-5-yl)-1-fluoroethenyl]-4-(difluoromethoxy)-N-[(2S)-1-hydroxy-4-(trifluoromethoxy)butan-2-yl]benzamide,
(13) 4-(difluoromethoxy)-3-fluoro-5-[(1Z)-1-fluoro-2-(5-methoxypyridin-3-yl)ethenyl]-N-[(1S,2S,4S)-2-hydroxy-4-(trifluoromethoxy)cyclopentyl]benzamide,
(14) 7-(5-([(1S,2S)-2-hydroxycyclohexyl]carbamoyl}-2-(trifluoromethyl)phenyl)imidazo[1,5-a]pyridine-3-carboxamide,
(15) 5-(3-carbamoylimidazo[1,5-a]pyridin-7-yl}-N-[(1S,2S,4S)-2-hydroxy-4-(trifluoromethoxy)cyclopentyl]-6-(trifluoromethyl)pyridine-3-carboxamide,
(16) N-(2-ethyl-5-{[(1S,2S,4S)-2-hydroxy-4-(trifluoromethoxy)cyclopentyl]carbamoyl}phenyl)-2-phenyl-1,3-oxazole-5-carboxamide,
(17) 2-(cyclopropylmethyl)-N-(2-ethyl-5-{[(1S,2S,4S)-2-hydroxy-4-(trifluoromethoxy)cyclopentyl]carbamoyl}phenyl)-1,3-thiazole-5-carboxamide,
(18) 4-(difluoromethoxy)-N-[(5S,6S)-6-hydroxyspiro[2.4]heptan-5-yl]-3-[2-(pyrimidin-5-yl)ethynyl]benzamide,
(19) 4-(difluoromethoxy)-3-[2-(5-fluoropyridin-3-yl)ethynyl]-N-[(5S,6S)-6-hydroxyspiro[2.4]heptan-5-yl]benzamide,
(20) 3-[2-(2-aminopyrimidin-5-yl)ethynyl]-N-[(1S,2S)-5,5-difluoro-2-hydroxycyclohexyl]-4-(difluoromethoxy)benzamide,
(21) 3-[2-(2-aminopyrimidin-5-yl)ethynyl]-4-(difluoromethoxy)-N-[(5S,6S)-6-hydroxyspiro[2.4]heptan-5-yl]benzamide,
(22) 3-[2-(2-aminopyrimidin-5-yl)ethynyl]-4-(difluoromethoxy)-N-[(2S)-1-hydroxy-4-(trifluoromethoxy)butan-2-yl]benzamide,
(23) 3-[2-(2-amino-4-methoxypyrimidin-5-yl)ethynyl]-4-(difluoromethoxy)-N-[(5S,6S)-6-hydroxyspiro[2.4]heptan-5-yl]benzamide,
(24) 4-(difluoromethoxy)-N-[(2S)-1-hydroxy-4-(trifluoromethoxy)butan-2-yl]-3-[2-(1-methyl-1H-pyrazol-4-yl)ethynyl]benzamide,
(25) 3-[(1Z)-2-(5-aminopyrazin-2-yl)-2-fluoroethenyl]-N-[(1S,2S)-5,5-difluoro-2-hydroxycyclohexyl]-4-(difluoromethoxy)benzamide,
(26) 3-[(1Z)-2-(5-aminopyrazin-2-yl)-2-fluoroethenyl]-4-(difluoromethoxy)-N-[(5S,6S)-6-hydroxyspiro[2.4]heptan-5-yl]benzamide,
(27) 7-(5-{[(1S,2S)-5,5-difluoro-2-hydroxycyclohexyl]carbamoyl}-2-(trifluoromethyl)phenyl)imidazo[1,5-a]pyridine-3-carboxamide,
(28) 7-(5-{[(5S,6S)-6-hydroxyspiro[2.4]heptan-5-yl]carbamoyl}-2-(trifluoromethyl)phenyl)imidazo[1,5-a]pyridine-3-carboxamide,
(29) 7-(5-{[(1S,2S, 4S)-2-hydroxy-4-(trifluoromethoxy)cyclopentyl]carbamoyl}-2-(trifluoromethyl)phenyl)imidazo[1,5-a]pyridine-3-carboxamide,
(30) 5-{3-carbamoylimidazo[1,5-a]pyridin-7-yl}-N-[(2S)-1-hydroxy-4-(trifluoromethoxy)butan-2-yl]-6-(trifluoromethyl)pyridine-3-carboxamide,
(31) 7-(5-{[(2S)-1-hydroxy-4-(trifluoromethoxy)butan-2-yl]carbamoyl}-2-(trifluoromethyl)phenyl)imidazo[1,5-a]pyridine-3-carboxamide,
(32) N-(2-ethyl-5-{[(2S)-1-hydroxy-4-(trifluoromethoxy)butan-2-yl]carbamoyl}phenyl)-5-(3-fluorophenyl)pyridine-3-carboxamide,
(33) N-(2-ethyl-5-{[(1S,2S,4S)-2-hydroxy-4-(trifluoromethoxy)cyclopentyl]carbamoyl)phenyl)-5-(3-fluorophenyl)pyridine-3-carboxamide,
(34) 3-[(1Z)-2-(2-aminopyrimidin-5-yl)-2-fluoroethenyl)-4-(difluoromethoxy)-N-[(1S)-1-(3-fluorophenyl)-2-hydroxyethyl]benzamide,
(35) 3-[2-(2-aminopyrimidin-5-yl)ethynyl]-N-[(25)-1-(4-chlorophenyl)-3-hydroxypropan-2-yl]-4-(difluoromethoxy)benzamide hydrochloride,
(36) 3-[2-(2-aminopyrimidin-5-yl)ethynyl]-4-(difluoromethoxy)-N-[(1S)-2-hydroxy-1-phenylethyl]benzamide hydrochloride,
(37) 3-[(1Z)-2-(2-aminopyrimidin-5-yl)-2-fluoroethenyl]-N-[(1S,2S)-5,5-difluoro-2-hydroxycyclohexyl]-4-(difluoromethoxy)benzamide,
(38) 3-[(1Z)-2-(2-aminopyrimidin-5-yl)-2-fluoroethenyl]-4-(difluoromethoxy)-N-[(1S,2S)-2-hydroxycyclohexyl]benzamide,
(39) 3-[(1Z)-2-(2-aminopyrimidin-5-yl)-1-fluoroethenyl]-4-(difluoromethoxy)-N-[(1S,2S,4S)-2-hydroxy-4-(trifluoromethoxy)cyclopentyl]benzamide,
(40) 3-[(1Z)-2-(2-aminopyrimidin-5-yl)-1-fluoroethenyl]-4-(difluoromethoxy)-N-[(5S,6S)-6-hydroxyspiro[2.4]heptan-5-yl]benzamide,
(41) 5-{3-carbamoylimidazo[1,5-a]pyridin-7-yl}-N-[(1S,2S)-5,5-difluoro-2-hydroxycyclohexyl]-6-(trifluoromethyl)pyridine-3-carboxamide,
(42) 5-(cyclopropylmethoxy)-N-(2-ethyl-5-{[(1S,25,4S)-2-hydroxy-4-(trifluoromethoxy)cyclopentyl]carbamoyl}phenyl)pyridine-3-carboxamide,
(43) 5-(cyclopropylmethoxy)-N-(2-ethyl-5-{[(2S)-1-hydroxy-4-(trifluoromethoxy)butan-2-yl]carbamoyl}phenyl)pyridine-3-carboxamide,
(44) 2-[(cyclopropylmethyl)amino]-N-(2-ethyl-5-{[(2S)-1-hydroxy-4-(trifluoromethoxy)butan-2-yl]carbamoyl}phenyl)-1,3-thiazole-5-carboxamide,
(45) 2-[(cyclopropylmethyl)amino]-N-(2-ethyl-5-{[(1S,2S,4S)-2-hydroxy-4-(trifluoromethoxy)cyclopentyl]carbamoyl}phenyl)-1,3-thiazole-5-carboxamide,
(46) 2-cyclopropyl-N-(2-ethyl-5-{[(1S,2S,4S)-2-hydroxy-4-(trifluoromethoxy)cyclopentyl]carbamoyl}phenyl)-1,3-thiazole-5-carboxamide, and
(47) 2-(cyclopropylmethyl)-N-(2-ethyl-5-{[(2S)-1-hydroxy-4-(trifluoromethoxy)butan-2-yl]carbamoyl}phenyl)-1,3-thiazole-5-carboxamide,
or a pharmaceutically acceptable salt thereof, or a solvate thereof.

### (Item 7)

A pharmaceutical composition containing the compound or a pharmaceutically acceptable salt thereof, or a solvate thereof, according to Item 1, as an active ingredient.

### (Item 8)

A DDR1 kinase inhibiting agent containing the compound or a pharmaceutically acceptable salt thereof, or a solvate thereof, according to Item 1, as an active ingredient.

### (Item 9)

A method for inhibiting DDR1 kinase, comprising administering the compound or a pharmaceutically acceptable salt thereof, or a solvate thereof, according to Item 1, to a subject in need thereof.

### (Item 10)

A compound or a pharmaceutically acceptable salt thereof according to Item 1 for use in inhibiting DDR1 kinase.

### (Item 11)

Use of the compound or a pharmaceutically acceptable salt thereof according to Item 1 in the manufacture of a DDR1 kinase inhibitor.

### (Item 12)

A prophylactic or therapeutic agent for diseases in which DDR1 kinase is involved, containing the compound or a pharmaceutically acceptable salt thereof, or a solvate thereof, according to Item 1.

### (Item 13)

A method for preventing or treating a disease in which DDR1 kinase is involved, comprising administering the compound or a pharmaceutically acceptable salt thereof, or a solvate thereof, according to Item 1, to a subject in need thereof.

### (Item 14)

The compound disclosed herein or a pharmaceutically acceptable salt thereof, for use in the prevention or treatment of a disease in which DDR1 kinase is involved.

### (Item 15)

Use of the compound disclosed herein or a pharmaceutically acceptable salt thereof, in the manufacture of a prophylactic or therapeutic agent for a disease in which DDR1 kinase is involved.

### (Item 16)

A prophylactic or therapeutic agent for Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic renal failure, microvascular nephrotic syndrome, mesangial proliferative glomerulonephritis, membranous proliferative glomerulonephritis, semilunar glomerulonephritis, membranous nephropathy, pulmonary fibrosis, myelofibrosis, liver fibrosis, acute myeloid leukemia, chronic myeloid leukemia, acute lymphoid leukemia, chronic lymphoid leukemia, Hodgkin lymphoma, non-Hodgkin's lymphoma, glioma, non-small cell lung cancer, small cell lung cancer, breast cancer, ovarian cancer, prostate cancer, colon cancer or osteoarthritis, containing the compound or a pharmaceutically acceptable salt thereof, or a solvate thereof, according to Item 1, as an active ingredient.

### Mode for Carrying Out the Invention

The following is an explanation of the meaning of each term used herein. Unless otherwise specified, each term has the same meaning when used alone or in combination with other terms.

The term "alkyl" includes, for example, straight or branched alkyls having from 1 to 6 carbon atoms, preferably from 1 to 4 carbon atoms, more preferably from 1 to 3 carbon atoms. Specifically, examples include methyl, ethyl, n-propyl, isopropyl, n-butyl, isobutyl, sec-butyl, tert-butyl, n-pentyl, sec-pentyl, 1-ethylpropyl, 1,2-dimethylpropyl, tert-pentyl, 2-methylbutyl, isopentyl, neopentyl, n-hexyl, sec-hexyl, 1-ethylbutyl, isohexyl, neohexyl, 1,1-dimethylbutyl, 2-ethylbutyl, 1,2,2-trimethylpropyl, 2,2-dimethylbutyl and the like.

The term "halogen" refers to fluorine, chlorine, bromine and iodine.

The term "amino" refers to -NH₂.

The term "alkoxy" refers to a group in which the above "alkyl" is bonded to an oxygen atom, for example, a straight or branched alkoxy having 1 to 6 carbon atoms, preferably 1 to 4 carbon atoms. Specifically, examples include methoxy, ethoxy, n-propoxy, isopropoxy, n-butoxy, isobutoxy, sec-butoxy, tert-butoxy, n-pentyloxy, n-hexyloxy and the like.

The alkoxy moiety of "haloalkoxy" can be the same as "alkoxy" described above.

Examples of "aryl" include mono- to tri-cyclic aromatic hydrocarbon groups having 6 to 14 carbons. Specifically, examples include phenyl, 1-naphthyl, 2-naphthyl, 1-anthryl, 2-anthryl, 9-anthryl, 1-phenanthryl 2-phenanthryl, 3-phenanthryl, 4-phenanthryl, 10-phenanthryl, and the like. Phenyl is preferred among others.

The term "haloalkyl" refers to a group in which the hydrogen atom of the above "alkyl" is replaced with the above "halogen". Specifically, examples include fluoromethyl, chloromethyl, fluoroethyl, difluoromethyl, dichloromethyl, difluoroethyl, trifluoromethyl, trichloromethyl, trifluoroethyl, and the like.

The term "cycloalkyl" refers to mono- to tri-cyclic non-aromatic hydrocarbon groups. Specifically, examples include cyclopropyl, cyclobutyl, cyclopentyl, cyclohexyl, cycloheptyl, cyclooctyl, and the like.

The above "non-aromatic hydrocarbon group" may be a bridged hydrocarbon group. Examples of the bridged hydrocarbon group include bicyclo[2.2.1]heptanyl (for example, bicyclo[2.2.1]heptan-1-yl, bicyclo[2.2.1]heptan-2-yl, bicyclo[2.2.1]heptan-7-yl), bicyclo[1.1.1]pentanyl (for example, bicyclo[1.1.1]pentan-1-yl, bicyclo[1.1.1]pentan-2-yl), bicyclo[4.1.0]heptanyl (for example, bicyclo[4.1.0]heptan-1-yl, bicyclo[4.1.0]heptan-2-yl, bicyclo[4.1.0]heptan-3-yl, bicyclo[4.1.0]heptan-7-yl), bicyclo[2.2.2]octanyl (for example, bicyclo[2.2.2]octan-1-yl, bicyclo[2.2.2]octan-2-yl), bicyclo[3.1.1]heptanyl (for example, bicyclo[3.1.1]heptan-1-yl, bicyclo[3.1.1]heptan-2-yl, bicyclo[3.1.1]heptan-3-yl, bicyclo[3.1.1]heptan-6-yl), and cuban-1-yl.

The above "non-aromatic carbocyclic group" may be a spirocyclic group. Examples of the spirocyclic group include spiro[3.3]heptanyl (for example, spiro[3.3]heptan-1-yl, spiro[3.3]heptan-2-yl), spiro[2.4]heptanyl (for example, spiro[2.4]heptan-1-yl, spiro[2.4]heptan-4-yl, spiro[2.4]heptan-5-yl), spiro[4.4]nonanyl (for example, spiro[4.4]nonan-1-yl, spiro[4.4]nonan-2-yl), spiro[5.5]undecanyl (for example, spiro[5.5]undecan-1-yl, spiro[5.5]undecan-2-yl, spiro[5.5]undecan-3-yl), and spiro[2.5]octanyl (for example, spiro[2.5]octan-1-yl, spiro[2.5]octan-4-yl, spiro[2.5]octan-5-yl, spiro[2.5]octan-6-yl).

The term "carbamoyl" refers to -CO-NH₂.

The term "carbonyl" refers to -C(=O)-.

Examples of "heteroaryl" include a mono- to tri-cyclic aromatic ring having 1 to 3 heteroatoms, which are selected from the group consisting of nitrogen atom, oxygen atom and sulfur atom, and having 6 to 14 carbon atoms. Specific examples include:
furyl (e.g., 2-furyl, 3-furyl);
thienyl (e.g., 2-thienyl, 3-thienyl);
pyrrolyl (e.g., 1-pyrrolyl, 2-pyrrolyl, 3-pyrrolyl);
imidazolyl (e.g., 1-imidazolyl, 2-imidazolyl, 4-imidazolyl);
pyrazolyl (e.g., 1-pyrazolyl, 3-pyrazolyl, 4-pyrazolyl);
triazolyl (e.g., 1,2,4-triazol-1-yl, 1,2,4-triazol-3-yl, 1,2,4-triazol-4-yl);
tetrazolyl (e.g., 1-tetrazolyl, 2-tetrazolyl, 5-tetrazolyl);
oxazolyl (e.g., 2-oxazolyl, 4-oxazolyl, 5-oxazolyl);
isoxazolyl (e.g., 3-isoxazolyl, 4-isoxazolyl, 5-isoxazolyl);
oxadiazolyl (e.g., 1,3,4-oxadiazol-2-yl);
thiazolyl (e.g., 2-thiazolyl, 4-thiazolyl, 5-thiazolyl);
thiadiazolyl (e.g., 1,3,4-thiadiazolyl, 1,2,4-thiadiazolyl, 1,2,3-thiadiazolyl);
isothiazolyl (e.g., 3-isothiazolyl, 4-isothiazolyl, 5-isothiazolyl);
pyridyl (e.g., 2-pyridyl, 3-pyridyl, 4-pyridyl);
pyridazinyl (e.g., 3-pyridazinyl, 4-pyridazinyl);
pyrimidinyl (e.g., 2-pyrimidinyl, 4-pyrimidinyl, 5-pyrimidinyl);
pyrazinyl (e.g., 2-pyrazinyl);
imidazo[1,5-a]pyridyl (e.g., imidazo[1,5-a]pyridin-1-yl, imidazo[1,5-a]pyridin-3-yl, imidazo[1,5-a]pyridin-5-yl, imidazo[1,5-a]pyridin-6-yl, imidazo[1,5-a]pyridin-7-yl, imidazo[1,5-a]pyridin-8-yl);
benzothiadiazolyl (e.g., 1,2,3-benzothiadiazol-4-yl, 1,2,3-benzothiadiazol-5-yl, 2,1,3-benzothiadiazol-4-yl, 2,1,3-benzothiadiazol-5-yl);
benzothiazolyl (e.g., benzothiazol-2-yl, benzothiazol-4-yl, benzothiazol-5-yl, benzothiazol-6-yl, benzothiazol-7-yl);
indolyl (e.g., indol-3-yl, indol-4-yl, indol-5-yl, indol-6-yl, indol-7-yl);
benzothiophenyl (e.g., 1-benzothiophen-2-yl, 1-benzothiophen-3-yl, 1-benzothiophen-4-yl, 1-benzothiophen-5-yl, 1-benzothiophen-6-yl, 1-benzothiophen-7-yl);
1,1-dioxo-1-benzothiophenyl (e.g., 1,1-dioxo-1-benzothiophen-2-yl, 1,1-dioxo-1-benzothiophen-3-yl, 1,1-dioxo-1-benzothiophen-4-yl, 1,1-dioxo-1-benzothiophen-5-yl, 1,1-dioxo-1-benzothiophen-6-yl, 1,1-dioxo-1-benzothiophen-7-yl);
quinolyl (e.g., quinolin-2-yl, quinolin-3-yl, quinolin-4-yl, quinolin-5-yl, quinolin-6-yl, quinolin-7-yl, quinolin-8-yl); and
1,3-benzoxazol-2-yl.

Each symbol in formula [1] is explained below.

R¹ in formula [1] may be one or two groups selected from the group consisting of hydrogen, halogen, an optionally-substituted alkyl, an optionally-substituted amino, an optionally-substituted alkoxy, an optionally-substituted aryl, haloalkyl, cycloalkyl, an optionally-substituted sulfonyl, cyano, an optionally-substituted carbamoyl, heteroaryl and dialkylphosphoryl.

Preferably, R¹ may be one or two groups selected from the group consisting of hydrogen, halogen, an optionally-substituted alkyl, an optionally-substituted amino, an optionally-substituted alkoxy, an optionally-substituted aryl, haloalkyl, and an optionally-substituted carbamoyl; more preferably, one or two groups selected from the group consisting of hydrogen, an optionally-substituted alkyl, an optionally-substituted amino and an optionally-substituted carbamoyl; even more preferably, one or two groups selected from the group consisting of hydrogen, alkyl, amino and carbamoyl; and most preferably, one group selected from the group consisting of hydrogen, alkyl, amino and carbamoyl.

"An optionally-substituted alkyl" described above for R¹ is preferably an alkyl substituted with cycloalkyl, more preferably an alkyl substituted with cyclopropyl, and still more preferably cyclopropylmethyl.

"An optionally-substituted aryl" described above for R¹ is preferably an aryl substituted with halogen, more preferably an aryl substituted with fluorine.

"An optionally-substituted alkoxy" described above for R¹ is preferably an alkoxy substituted with cycloalkyl, more preferably an alkoxy substituted with cyclopropyl, even more preferably cyclopropylmethoxy.

Het may be a 5- to 10-membered heteroaryl, preferably pyrimidinyl, pyridyl, pyrazinyl, pyrazolyl, pyridinyl, imidazo[1,5-a]pyridyl, oxazolyl, thiazolyl, or pyrazyl, more preferably pyrimidinyl, pyrazinyl, pyrazolyl, or imidazo[1,5-a]pyridyl.

L¹ may be a bond, -CO-NH-, -CR^{a}=CR^{b}- or -C=C-, wherein R^{a} is hydrogen or halogen and R^{b} is hydrogen or halogen, preferably a bond, -CR^{a}=CR^{b}- or -C=C-, and R^{a} is preferably hydrogen and R^{b} is preferably halogen, more preferably a bond, -CR^{a}=CR^{b}- or -C≡C-, and R^{a} is preferably hydrogen and R^{b} is preferably fluorine.

X may be N or C-R^{c} wherein R^{c} is hydrogen or halogen, preferably N or C-R^{c} and R^{c} is preferably hydrogen or halogen, more preferably N or C-R^{c} and R^{c} is more preferably hydrogen.

R² may be: hydrogen, ethyl, C₁-C₆ haloalkoxy or C₁-C₆ haloalkyl; preferably ethyl, C₁-C₆ haloalkoxy or C₁-C₆ haloalkyl; more preferably C₁-C₆ haloalkoxy or C₁-C₆ haloalkyl; even more preferably C₁-C₆ fluoroalkoxy, C₁-C₆ difluoroalkoxy, C₁-C₆ trifluoroalkoxy, C₁-C₆ fluoroalkyl, C₁-C₆ difluoroalkyl or C₁-C₆ trifluoroalkyl; still more preferably C₁-C₆ difluoroalkoxy, C₁-C₆ fluoroalkyl or C₁-C₆ trifluoroalkyl; and most preferably C₁-C₆ difluoroalkoxy.

R³ may be hydrogen, or R³ and R⁴ may be taken together with the carbon atoms to which they are attached to form an optionally-substituted C₃-C₈ cycloalkyl. Preferably, R³ and R⁴ are taken together with the carbon atoms to which they are attached to form an optionally-substituted C₃-C₈ cycloalkyl; more preferably, R³ and R⁴ are taken together with the carbon atoms to which they are attached to form an optionally-substituted cyclopentyl, cyclohexyl or spiro[2.4]heptanyl; even more preferably cyclopentyl.

"An optionally-substituted C₃-C₈ cycloalkyl" described above for R³ is preferably C₃-C₈ cycloalkyl substituted with one or two groups selected from the group consisting of halogen and haloalkoxy; more preferably C₃-C₈ cycloalkyl substituted with one or two haloalkoxy groups; even more preferably C₃-C₈ cycloalkyl substituted with one or two trifluoroalkoxy groups; still more preferably C₃-C₈ cycloalkyl substituted with one trifluoroalkoxy group; most preferably C₃-C₈ cycloalkyl substituted with one trifluoromethoxy group.

R⁴ may be an optionally-substituted phenyl or an optionally-substituted alkyl, or R⁴ and R³ are taken together with the carbon atoms to which they are attached to form an optionally-substituted C₃-C₈ cycloalkyl.

Preferably, R⁴ is an optionally-substituted phenyl or an optionally-substituted alkyl, or R⁴ and R³ are taken together with the carbon atoms to which they are attached to form an optionally-substituted C₃-C₈ cycloalkyl; more preferably R⁴ is an optionally-substituted alkyl, or R⁴ and R³ are taken together with the carbon atoms to which they are attached to form an optionally-substituted C₃-C₈ cycloalkyl; even more preferably R⁴ and R³ are taken together with the carbon atoms to which they are attached to form an optionally-substituted C₃-C₈ cycloalkyl; still more preferably, R⁴ and R³ are taken together with the carbon atoms to which they are attached to form an optionally-substituted cyclopentyl, cyclohexyl or spiro[2.4]heptanyl; most preferably, R⁴ and R³ are taken together with the carbon atoms to which they are attached to form an optionally-substituted cyclopentyl.

"An optionally-substituted C₃-C₈ cycloalkyl" described above for R⁴ is preferably C₃-C₈ cycloalkyl substituted with one or two groups selected from the group consisting of halogen and haloalkoxy; more preferably C₃-C₈ cycloalkyl substituted with one or two haloalkoxy groups; even more preferably C₃-C₈ cycloalkyl substituted with one or two trifluoroalkoxy groups; still more preferably C₃-C₈ cycloalkyl substituted with one trifluoroalkoxy group; and most preferably C₃-C₈ cycloalkyl substituted with one trifluoromethoxy group.

The compounds of the invention can be produced using known compounds or intermediates that can be easily synthesized from known compounds, for example, according to the methods as described below, the methods according to the working examples described below or known methods. In the production of the compounds of the invention, if the starting material has a substituent group capable of affecting the reaction, it is generally protected in advance with an appropriate protecting group, and then the reaction is carried out. The protecting group can be deprotected by a known method after the reaction.

Although the compounds of the invention can be used as it is for pharmaceuticals, they can also be used in the form of a pharmaceutically acceptable salt or solvate, or solvate of the salt according to a known method. Examples of pharmaceutically acceptable salts include salts of inorganic acids such as hydrochloric acid, hydrobromic acid, sulfuric acid, and phosphoric acid, and organic acids such as acetic acid, malic acid, lactic acid, citric acid, tartaric acid, maleic acid, succinic acid, fumaric acid, p-toluenesulfonic acid, benzenesulfonic acid, and methanesulfonic acid, salts with alkali metal such as lithium, potassium, and sodium, salts with alkaline earth metal such as magnesium and calcium, and salts with organic base such as ammonium salts. These salts can be formed according to a conventional method.

For example, a hydrochloride salt of the compound can be obtained by dissolving the free base of the compound in an aqueous solution of hydrogen chloride (hydrochloric acid), or a solution of hydrogen chloride in alcohol, ethyl acetate, 1,4-dioxane, cyclopentyl methyl ether or diethyl ether.

The compounds of the invention may become a solvate by incorporating solvent molecules when left in air or recrystallized, and the invention also includes such solvates. Examples of such solvates include solvates formed with solvent molecules such as methanol, ethanol, isopropyl alcohol, butanol, dimethyl sulfoxide, and acetonitrile, as well as monohydrate and dihydrate.

Some of the compounds of the invention may have an asymmetric carbon, and the respective stereo isomers and mixtures thereof are all included in the present invention. The stereo isomers can be prepared, for example, by means of optical resolution from the racemate thereof according to a known method using an optically active acid, such as tartaric acid, dibenzoyltartaric acid, mandelic acid, or 10-camphor sulfonic acid, utilizing its basicity, or by using an optically active compound prepared in advance as a starting material. In addition, the stereo isomers may also be prepared by optical resolution using a chiral column or by asymmetric synthesis.

The compound of the invention has DDR1 kinase inhibitory activity, as shown below in the Test Examples.

Accordingly, one embodiment of the invention provides a DDR1 kinase inhibiting agent containing the compound of the invention.

Furthermore, one embodiment of the invention provides a method for inhibiting DDR1 kinase, comprising administering the compound of the invention to a subject in need thereof.

Furthermore, one embodiment of the invention provides the compound of the invention for use in DDR1 kinase inhibition.

Furthermore, one embodiment of the invention provides the use of the compound of the invention in the manufacture of DDR1 kinase inhibitor.

One embodiment of the invention provides a prophylactic or therapeutic agent for diseases in which DDR1 kinase is involved, containing the compound of the invention.

Furthermore, one embodiment of the invention provides a method for preventing or treating a disease in which DDR1 kinase is involved, comprising administering the compound of the invention to a subject in need thereof.

Furthermore, one embodiment of the invention provides the compound of the invention for use in the prevention or treatment of a disease in which DDR1 kinase is involved.

Furthermore, one embodiment of the invention provides the use of the compound of the invention, in the manufacture of a prophylactic or therapeutic agent for a disease in which DDR1 kinase is involved.

Examples of the diseases to which the compounds of the invention are applicable include Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic renal failure, microvascular nephrotic syndrome, mesangial proliferative glomerulonephritis, membranous proliferative glomerulonephritis, crescentic glomerulonephritis, membranous nephropathy, pulmonary fibrosis, myelofibrosis, liver fibrosis, acute myeloid leukemia, chronic myeloid leukemia, acute lymphocytic leukemia, chronic lymphocytic leukemia, Hodgkin lymphoma, non-Hodgkin lymphoma, glioma, non-small cell lung cancer, small cell lung cancer, breast cancer, ovarian cancer, prostate cancer, colorectal cancer, and osteoarthritis.

The term "subject" refers to a human or non-human animal that has or is suspected of having a disease in which DDR1 kinase is involved. In one embodiment of the invention, the subject is a mammal. In one embodiment of the invention, the subject is a human.

The compound of the invention can be used as a therapeutic agent for various disorders as described above, for example, for mammals such as humans, mice, rats, rabbits, dogs, cats, cows, horses, pigs, and monkeys, as it is or by mixing the compound of the invention with a pharmacologically acceptable carrier or the like to prepare a pharmaceutical composition containing, for example, 0.001% to 99.5%, preferably 0.1% to 90%, of the compound of the present invention.

The carrier may be one or more of pharmaceutically acceptable solid, semi-solid or liquid diluents, fillers, and other auxiliaries for formulations. The pharmaceutical composition of the invention is preferably administered in a unit dosage form. The pharmaceutical composition can be administered by tissue administration, oral administration, intravenous administration, topical administration (transdermal administration, eye drops, intraperitoneal cavity, intrathoracic cavity, etc.) or transrectally. Of course, the composition is administered in a dosage form suitable for the mode of administration.

The dose as a pharmaceutical is preferably adjusted taking into consideration the conditions such as age, weight, type and severity of disease of the patient, administration route, type of the compound of the invention, whether or not it is a salt, and the type of the salt. Typically, the active ingredient amount of the compound of the invention or a pharmaceutically acceptable salt thereof for adult, in the case of oral administration, is suitably within a range of 0.01 mg to 5 g/day/adult, preferably 1 mg to 500 mg/day/adult. In some cases, a smaller amount may be sufficient or a larger amount may be required. Usually, the dosage can be administered once a day or can be divided and administered several times a day, or in the case of intravenous administration, the dosage can be administered rapidly or sustainably within 24 hours.

One or more hydrogen, carbon, and/or the other atoms in the compound of the invention may be replaced with an isotope thereof. Examples of such isotopes include ²H, ³H, ¹¹C, ¹³C, ¹⁴C, ¹⁵N, ¹⁸O, ¹⁷O, ³¹P, ³²P, ³⁵S, ¹⁸F, ¹²³I, and ³⁶Cl, i.e., hydrogen, carbon, nitrogen, oxygen, phosphorus, sulfur, fluorine, iodine, and chlorine. The compound substituted with such an isotope is also useful as a pharmaceutical and includes all radiolabeled compounds of the compound of the present invention.

### (Preparation of the compound of the invention)

The compound of the invention can be prepared from a known compound per se or an intermediate that is easily preparable from the known compound, according to the following method, the Examples described below, or a known method.

If the solvents, reagents and starting materials used in each step in the following preparation methods are commercially available, such commercially available products can be used as they are. Also, the compound obtained or the starting material used in each step in the following preparation methods may form a salt and can be converted by a known method into another type of salt or a free form. Alternatively, when the compound obtained or the starting material used in each step in the following preparation methods is a free form, it can be converted into a desired salt by a known method. Examples of such salts include those similar to the salts described above for the compound of the present invention.

In the preparation of the compound of the invention, if the starting material has a substituent group capable of affecting the reaction, a protective group may be introduced in these substituent groups by a known method in advance, and the target compound can be obtained by removing the protective group, if necessary, after the reaction. For such introduction of a protective group and removal of the protective group, for example, the conditions described in Wuts and Greene, "Greene's Protective Groups in Organic Synthesis", 4th edition, John Wiley & Sons Inc., 2006, or P.J. Kocienski, "Protecting Groups", 3rd edition, Thieme, 2005, may be selected and used as appropriate.

The compound obtained in each step of the following preparation methods can be isolated or purified according to a conventional method such as solvent extraction, concentration, distillation, sublimation, recrystallization, reprecipitation, chromatography, and the like. Alternatively, the compound may also be used in the next step as a reaction mixture or a crude product.

Unless otherwise specified, the reaction in each step in the following preparation methods is conducted according to known methods, for example, such as methods as described in "Comprehensive Organic Transformations: A Guide to Functional Group Preparations", 2nd Ed. by R. C. Larock, John Wiley & Sons, Inc., 1999; The Chemical Society of Japan, "Experimental Chemistry", 4th edition, Maruzen, 1992; L. Kuerti and B. Czako, "Strategic Applications of Named Reactions in Organic Synthesis", translated by Kiyoshi Tomioka, Kagaku-Dojin Publishing Company, Inc., 2006; and G. S. Zweifel and M.H. Nantz, "Modern Organic Synthesis: An Introduction", translated by Tamejiro Hiyama, Kagaku-Dojin Publishing Company, Inc., 2009; or methods in similar manners as described in the Examples, such that these methods are modified or combined as appropriate.

The compounds of the invention can be produced, for example, by the general synthetic methods shown below. Extraction, purification and other processes can be carried out as in ordinary organic chemistry experiments.

Preparation method of Compound 1-A 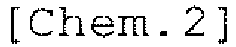 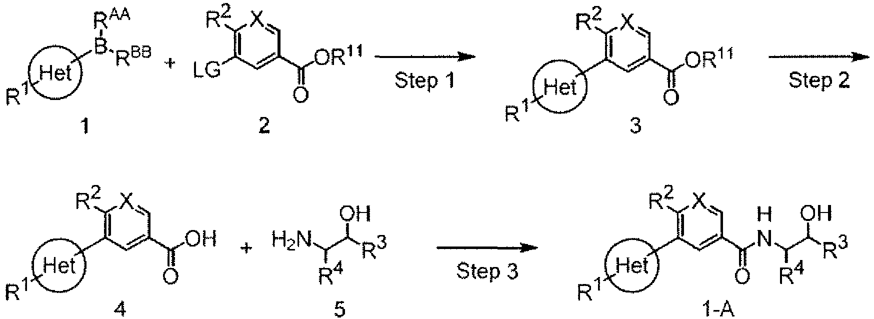 wherein R¹, R², R³, R⁴, Het and X are as defined above. R^{AA} and R^{BB} are both hydroxy groups, or R^{AA} and R^{BB} are taken together to form -O-C(CH₃)₂-C(CH₃)₂-O-, -O-(CH₂)₃-O-, or -O-CH₂-C(CH₃)₂-CH₂-O-. LG is a leaving group, for example, halogen, trifluoromethanesulfonate, or the like. R¹¹ is alkyl, for example, methyl, ethyl, or the like.

### Step 1

This step is to obtain Compound 3 by coupling reaction of Compound 1 with Compound 2 in the presence of a transition metal such as palladium.

The reaction can be carried out under the conditions generally used in coupling reactions using transition metals. The Suzuki-Miyaura coupling reaction and the Heck reaction are examples of such coupling reactions using transition metals.

Examples of the Suzuki-Miyaura coupling reaction include the reactions found in literature such as Suzuki et al., Chem. Rev., 1995, 95, 2457-2483.

Examples of the Heck reaction include the reactions found in literature such as Heck et al., Org. Synth., 2005, 81, 63-76, Heck et al., J. Org. Chem., 1972, 37, 2320-2322, and Beletskaya et al, Chem. Rev., 2000, 100, 3009-3066.

The amount of Compound 1 is appropriately within the range of 0.5 molar equivalent to 3 molar equivalents of Compound 2.

The organometallic catalyst used in this reaction is not particularly limited. Preferred examples of the organometallic catalyst include metal catalysts such as tris(dibenzylideneacetone)bispalladiumochloroform adduct (hereinafter, referred to as "Pd₂(dba)₃•CHCl₃"), tris(dibenzylideneacetone)bispalladium (hereinafter, referred to as "Pdz(dba)₃"), tetrakistriphenylphosphine palladium (hereinafter, referred to as "Pd(PPh₃)₄"), [1,1'-bis(diphenylphosphino)ferrocene]-dichloropalladium(II)•dichloromethane adduct (hereinafter referred to as "Pd(dppf)Cl₂•CH₂Cl₂"), bis(triphenylphosphine)palladium(II) dichloride (hereinafter, referred to as "PdCl₂(PPh₃)₂"), [1,1'-bis(di-tert-butylphosphino)ferrocene]-dichloropalladium(II) (hereinafter, referred to as "Pd(dtbpf)Cl₂), bis(tricyclohexylphosphine)palladium(II) dichloride (hereinafter, referred to as "PdCl₂(PCy₃)₂"), palladium(II) acetate (hereinafter, referred to as "Pd(OAc)₂"), and [1,3-bis(diphenylphosphino)propane]nickel (II), and mixtures of these metal catalysts.

The amount of the transition metal used is appropriately within the range of 0.01 molar equivalent to 0.3 molar equivalent of Compound 1.

In this step, a base or a salt may be used as necessary. Examples of the base or the salt to be used include bases or salts such as potassium carbonate, cesium carbonate, sodium carbonate, sodium bicarbonate, sodium acetate, potassium acetate, trisodium phosphate, tripotassium phosphate, and a solution thereof, triethylamine (hereinafter, referred to as "TEA"), N,N-diisopropylethylamine (hereinafter, referred to as "DIPEA"), lithium chloride, and copper(I) iodide.

The amount of base to be used is appropriately within the range of 1 molar equivalent to 4 molar equivalents of Compound 1.

In this step, a suitable ligand may be used as necessary. Examples of a ligand that can be used include 1,1'-bis(diphenylphosphino)ferrocene (hereinafter, referred to as "dppf"), 4,5-bis(diphenylphosphino)-9,9-dimethylxanthene (hereinafter, referred to as "Xantphos"), 2-dicyclohexylphosphino-2',4',6'-triisopropylbiphenyl (hereinafter, referred to as "XPhos"), 2,2'-bis(diphenylphosphino)-1,1'-binaphthyl (hereinafter, referred to as "BINAP"), 2-dicyclohexylphosphino-2',6'-diisopropylbiphenyl (hereinafter, referred to as "RuPhos"), triphenylphosphine (hereinafter, referred to as "PPh₃"), and tricyclohexylphosphine (hereinafter referred to as "PCy₃").

The amount of ligand to be used is appropriately within the range of 1 to 5 molar equivalents of the transition metal.

The solvent to be used in this step is not particularly limited as long as it is not involved in the reaction, and examples of the solvent include: hydrocarbons such as toluene and xylene; ethers such as 1,4-dioxane, tetrahydrofuran (hereinafter, referred to as "THF"), and dimethoxyethane (hereinafter, referred to as "DME"); amides such as N,N-dimethylformamide (hereinafter, referred to as "DMF"), N,N-dimethylacetamide (hereinafter, referred to as "DMA"), and N-methylpyrrolidone (hereinafter, referred to as "NMP"); alcohols such as ethanol, 2-propanol, and tert-butanol; water; and mixed solvents thereof.

The reaction temperature varies depending on the type of starting materials and reagents used, but usually a temperature within the range of 20°C to 200°C is appropriate. If necessary, a microwave reaction apparatus may be used.

The reaction time varies depending on the type of starting materials used and the reaction temperature, but is usually appropriate within the range of 0.1 to 24 hours.

### Step 2

This step is to obtain Compound 4 by hydrolyzing the ester moiety in the presence of an appropriate acid or base in an appropriate solvent.

Examples of the acid used in this step include: inorganic acids such as hydrochloric acid and sulfuric acid; and organic acids such as trifluoroacetic acid (hereinafter referred to as "TFA"), methanesulfonic acid, and toluene sulfonic acid. Examples of the base include inorganic bases such as sodium hydroxide, potassium hydroxide, and lithium hydroxide.

The amount of the acid or base used in this step is appropriately within the range of 1 molar equivalent to 10 molar equivalents of Compound 3. An acid or base may be used in an excess amount to Compound 3. When an acid is used, hydrolysis can be carried out even at a catalytic amount.

The solvent to be used in this step is not limited as long as it is not involved in the reaction, and examples of the solvent include: alcohols such as methanol, ethanol, and 2-propanol; ethers such as THF, diethyl ether, 1,4-dioxane, and DME; nitriles such as acetonitrile and propionitrile; ketones such as acetone; water; and mixed solvents thereof.

The reaction temperature can vary depending on the types of the starting material and the reagent to be used, and the reaction can be carried out typically at a temperature within the range of 20°C to 200°C, preferably within the range of 20°C to 100°C. Also, a microwave reaction apparatus may be used as necessary.

The reaction time can vary depending on the type of the starting material to be used and the reaction temperature, and is appropriately within the range of 0.5 hour to 4 days.

### Step 3

The step is to obtain Compound 1-A by coupling Compound 4 or its reactive derivative with amine Compound 5 in the presence of a coupling agent.

Examples of the reactive derivatives of Compound 4 include acid halides (e.g., acid chlorides, acid bromides), mixed acid anhydrides, imidazolides, active amides, and the like, which are conventionally used in amide coupling reactions.

Appropriate amounts of coupling agent and amine Compound 5 used in this step are respectively within the range of 1 molar equivalent to 10 molar equivalents of Compound 4.

Examples of the coupling agent used in this step include 1,1'-carbonyldiimidazole (hereinafter, referred to as "CDI"), 1-ethyl-3-(3-dimethylaminopropyl)carbodiimide (hereinafter, referred to as "EDCI"), diisopropylcarbodiimide (hereinafter, referred to as "DIC"), diethyl cyanophosphonate, O-(benzotriazol-1-yl)-N,N,N',N'-tetramethyluronium hexafluorophosphate (hereinafter, referred to as "HBTU"), O-(7-azabenzotriazol-1-yl)-N,N,N',N'-tetramethyluronium hexafluorophosphate (hereinafter referred to as "HATU") and the like.

In this step, a base may be used as necessary. Examples of the base to be used include organic bases such as TEA, DIPEA, N,N-dimethylaniline, and DBU.

The amount of the base to be used is appropriately within the range of 1 molar equivalent to 10 molar equivalents of Compound 4.

In this step, additives, such as 1-hydroxybenzotriazole (hereinafter referred to as "HOBt"), N-hydroxysuccinimide, and 1-hydroxy-7-azabenzotriazole (hereinafter referred to as "HOAt"), may be added.

When said additive is used in this step, an appropriate amount of the additive is within the range of 0.1 to 10 molar equivalents of Compound 4.

The solvents to be used are not limited as long as they are not involved in the reaction. Examples of the solvents include: hydrocarbons such as toluene and xylene; ethers such as 1,4-dioxane, THF and DME; amides such as DMF and DMA; halogenated hydrocarbons such as dichloromethane and chloroform; nitriles such as acetonitrile and propionitrile; and mixed solvents thereof.

The reaction temperature varies depending on the type of starting materials and reagents used, but usually a temperature within the range of -20°C to 150°C is appropriate. If necessary, a microwave reaction apparatus may be used.

The reaction time varies depending on the type of starting materials used and the reaction temperature, but is usually appropriate within the range of 0.1 to 72 hours.

Preparation method of Compound 1-B 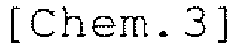 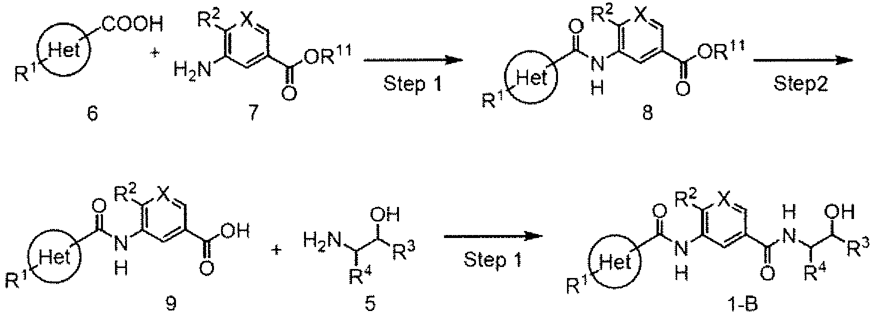 wherein R¹, R², R³, R⁴, Het and X are as defined above. R¹¹ is alkyl, such as methyl or ethyl.

### Step 1

This step is to obtain Compound 8 by coupling Compound 6 or its reactive derivative with amine Compound 7 in the presence of a coupling agent, in a manner similar to that described in Step 3 of the Preparation Method 1.

### Step 2

This step is to obtain Compound 9 by hydrolyzing the ester moiety in the presence of an appropriate acid or base in an appropriate solvent, in a manner similar to that described in Step 2 of the Preparation Method 1.

### Step 3

This step is to obtain Compound 1-B by coupling Compound 9 or its reactive derivative with amine Compound 5 in the presence of a coupling agent, in a manner similar to that described in Step 3 of the Preparation Method 1.

### Preparation of Compound 1-C

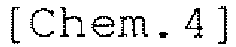

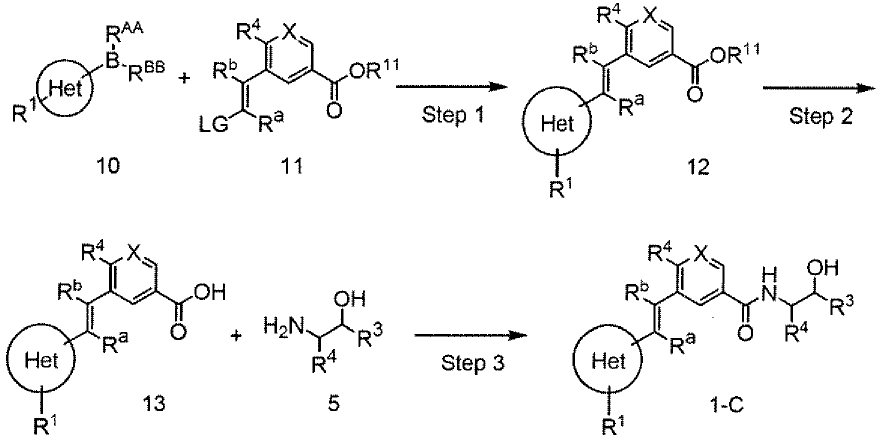

wherein R¹, R², R³, R⁴, R^{a}, R^{b}, Het, R^{AA}, R^{BB}, X and LG are as defined above.

### Step 1

This step is to obtain Compound 12 by coupling reaction of Compound 10 and Compound 11 in the presence of a transition metal such as palladium, in a manner similar to that described in Step 1 of the Preparation Method 1.

### Step 2

This step is to obtain Compound 13 by hydrolyzing the ester moiety of Compound 12 in an appropriate solvent in the presence of an appropriate acid or base, in a manner similar to that described in Step 2 of the Preparation Method 1.

### Step 3

This step is to obtain Compound 1-C by coupling Compound 13 or its reactive derivative with amine Compound 5 in the presence of a coupling agent, in a manner similar to that described in Step 3 of the Preparation Method 1.

Compound 12 can also be prepared by the following method. 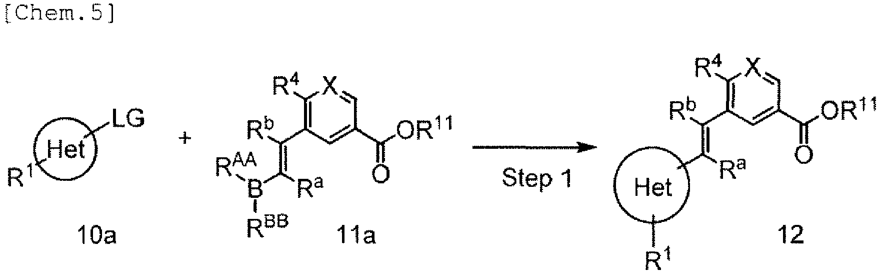 wherein R¹, R², R³, R⁴, R^{a}, R^{b}, Het, R^{AA}, R^{BB}, X and LG are as defined above.

### Step 1

This step is to obtain Compound 12 by coupling reaction of Compound 10a with Compound 11a in the presence of transition metal such as palladium, in a manner similar to that described in Step 1 of the Preparation Method 1.

### Preparation of Compound 1-D

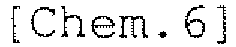

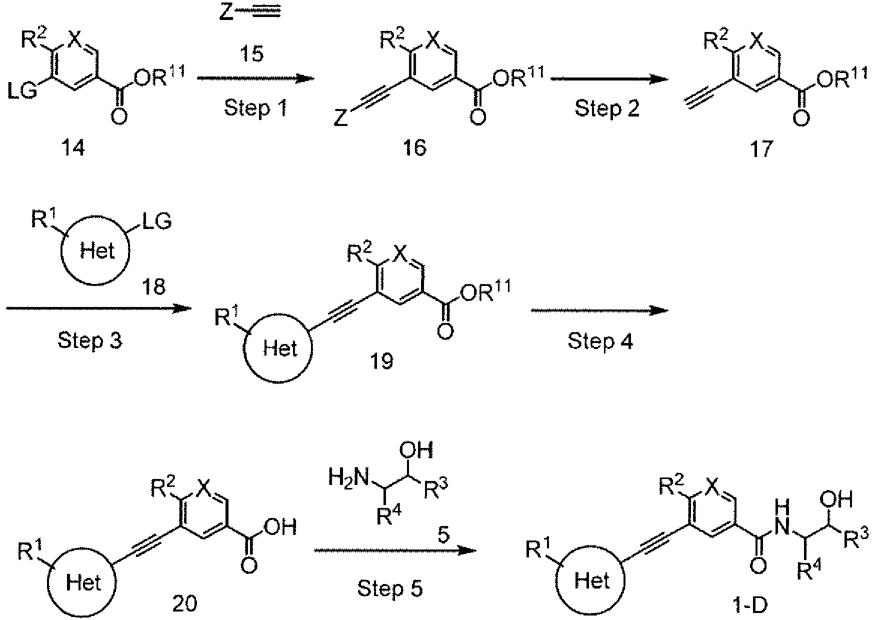

wherein R¹, R², R³, R⁴, R¹¹, Het, X and LG are as defined above. Z is a leaving group, for example, trimethylsilyl or triethylsilyl.

### Step 1

This step is to obtain Compound 16 by coupling Compound 14 with Compound 15 in the presence of a transition metal such as palladium.

This reaction can be carried out under the conditions commonly used in coupling reactions using transition metals, specifically the Sonogashira coupling reaction as described in references, such as: Sonogashira et al., J. Organomet. Chem. 2002, 653, 46-49; Negishi et al., Chem. Rev., 2003, 103, 1979-2017.

The amount of Compound 15 is appropriately within the range of 0.5 molar equivalent to 3 molar equivalents of Compound 14.

The amount of the transition metal is appropriately within the range of 0.01 molar equivalent to 0.3 molar equivalent of Compound 14.

In this step, a base or salt may be used if necessary. Examples of the base or salt include potassium carbonate, cesium carbonate, sodium carbonate, sodium bicarbonate, sodium acetate, potassium acetate, trisodium phosphate, tripotassium phosphate and a solution thereof, triethylamine (hereinafter referred to as "TEA"), N,N-diisopropylethylamine (hereinafter referred to as "DIPEA"), lithium chloride, copper(I) iodide, and other bases or salts.

The amount of the base is appropriately within the range of 1 molar equivalent to 4 molar equivalents of Compound 14.

Suitable ligands may be used in this step. Examples of such ligands include 1,1'-bis(diphenylphosphino)ferrocene (hereinafter referred to as "dppf"), 4,5-bis(diphenylphosphino)-9,9-dimethylxanthene (hereinafter referred to as "Xantphos"), 2-dicyclohexylphosphino-2',4',6'-triisopropylbiphenyl (hereinafter referred to as "XPhos"), 2,2'-bis(diphenylphosphino)-1,1'-binaphthyl ("BINAP"), 2-dicyclohexylphosphino-2',6'-diisopropylbiphenyl (hereinafter referred to as "RuPhos"), triphenylphosphine (hereinafter referred to as "PPh₃"), and tricyclohexylphosphine (hereinafter referred to as "PCy₃").

The amount of ligand is appropriately within the range of 1 molar equivalent to 5 molar equivalents of the transition metal to be used.

The solvent used in this step is not limited so long as it is not involved in the reaction, and examples include: hydrocarbons such as toluene and xylene; ethers such as 1,4-dioxane, tetrahydrofuran (hereinafter referred to as "THF"), and dimethoxyethane (hereinafter referred to as "DME"); amides such as N,N-dimethylformamide (hereinafter referred to as "DMF"), N,N-dimethylacetamide (hereinafter referred to as "DMA"), and N-methylpyrrolidone (hereinafter referred to as "NMP"); alcohols such as ethanol, 2-propanol, and tert-butanol; water; and a mixed solvent thereof.

The reaction temperature varies depending on the type of starting materials and reagents used, but usually a temperature within the range of 20°C to 200°C is appropriate. If necessary, a microwave reaction apparatus may be used.

The reaction time varies depending on the type of starting materials used and the reaction temperature, but is usually appropriate within the range of 0.1 to 24 hours.

### Step 2

This step is to obtain Compound 17 by deprotecting Z, and can be carried out as described in: Wuts and Greene, "Greene's Protective Groups in Organic Synthesis", 4th edition, John Wiley & Sons Inc., 2006; or P. J. Kocienski, "Protecting Groups", 3rd edition, Thieme, 2005.

### Step 3

This step is to obtain Compound 19 by coupling Compound 17 with Compound 18 in the presence of a transition metal such as palladium, in a manner similar to that described in Preparation of Compound 1-D, Step 1.

Compound 19 can be prepared directly from Compound 16 and Compound 18 according to the method of Tolnai et al. (J. Org. Chem., 2018, 83, 8281-8291).

### Step 4

This step is to obtain Compound 20 by hydrolyzing the ester moiety of Compound 19 in an appropriate solvent in the presence of an appropriate acid or base, in a manner similar to that described in Step 2 of the Preparation Method 1.

### Step 5

This step is to obtain Compound 1-D by coupling Compound 20 or its reactive derivative with amine Compound 5 in the presence of a coupling agent, in a manner similar to that described in Step 3 of the Preparation Method 1.

The compound of the invention has DDR1 kinase inhibitory activity, as shown in the Test Examples below. Since the compound of the invention has DDR1 kinase inhibitory activity, it is also effective in the treatment of Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic renal failure, microvascular nephrosis syndrome, mesangial proliferative glomerulonephritis, membranous proliferative glomerulonephritis, semilunar glomerulonephritis, membranous nephropathy, pulmonary fibrosis, myelofibrosis, liver fibrosis, acute myeloid leukemia, chronic myeloid leukemia, acute lymphocytic leukemia, chronic lymphocytic leukemia, Hodgkin lymphoma, non-Hodgkin lymphoma, glioma, non-small cell lung cancer, small cell lung cancer, breast cancer, ovarian cancer, prostate cancer, colorectal cancer or osteoarthritis.

Therefore, the compounds of the invention or their pharmaceutically acceptable salts can be used, for example, as prophylactic or therapeutic agents for diseases in which DDR1 kinase is involved.

The following Reference Examples, Examples, and Test Examples are provided to explain the present invention in more detail, but the present invention is not limited only to them.

Abbreviations as used herein have the following meanings.

In the Examples, the following abbreviations are used.
Pd-C: palladium-carbon
Pd(PPh₃)₄: tetrakistriphenylphosphine palladium
PdCl₂(PPh₃)₂: bis(triphenylphosphine)palladium(II) dichloride
PPh₃: triphenylphosphine
Boc₂O: di-tert-butyl dicarbonate
HATU: O-(7-azabenzotriazol-1-yl)-N,N,N',N'-tetramethyluronium hexafluorophosphate
HBTU: O-(benzotriazol-1-yl)-N,N,N',N'-tetramethyluronium hexafluorophosphate
EDCI: 1-ethyl-3-(3-dimethylaminopropyl)carbodiimide
HOBt: 1-hydroxybenzotriazole
THF: tetrahydrofuran
DMF: dimethylformamide
DMSO: dimethyl sulfoxide
NMP: N-methylpyrrolidone
IPA: 2-propanol
DIPA: diisopropylamine
DIPEA: N,N-diisopropylethylamine
TEA: triethylamine
DBU: 1,8-diazabicyclo[5.4.0]-7-undecene
MS: mass spectrometry
LCMS: High performance liquid chromatography-mass spectrometry
ESI: Electron Spray Ionization
M: Molar concentration (mol/L)

MS was measured by LCMS. The ESI method was used as the ionization method. The observed mass spectrometry values are expressed as m/z.

The LCMS measurement conditions are as follows.
Instrument: ACQUITY UPLC MS/PDA system (Waters Corp.)
Mass spectrometer: ACQUITY QDa detector or Waters 3100 MS detector
Photodiode array detector: ACQUITY PDA detector (UV
detection wavelength: 210-400 nm)
Column: Acquity BEH C18, 1.7 µm, 2.1 × 50 mm
Flow rate: 0.5 mL/min
Column temperature: 40°C
Solvents;
Liquid A: 0.1% formic acid/H₂O (v/v; same for below)
Liquid B: 0.1% formic acid/acetonitrile

The measurement conditions for chiral column chromatography are as follows.

### Method A:

Instrument: i-Series UHPLC model LC-2060C 3D (PDA model) (Shimadzu Corporation)
Detection wavelength: 220 nm
Column: CHIRALPAK IB, 0.46 cm I.D. x 25 cm L
Flow rate: 1.0 mL/min
Column temperature: 40°C
Mobile phase: n-Hexane/IPA = 92/8 (v/v)

### Method B:

Pump: LC-20AD (Shimadzu Corporation)
Detector: SPD-20A (Shimadzu Corporation)
Detection wavelength: 210 nm
Column: CHIRALPAK AD, 0.46 cm I.D. x 25 cm L
Flow rate: 1.0 mL/min
Column temperature: 40°C
Mobile phase: n-Hexane/IPA = 90/10 (v/v)

¹H NMR spectra were obtained by JNM-ECS400 nuclear magnetic resonance system (JEOL RESONANCE Corporation). The observed peaks are expressed as chemical shift values δ (ppm) (s = singlet, d = doublet, t = triplet, q = quartet, brs = broad singlet, m = multiplet, dd = double doublet, dt = double triplet).

Microwave experiments were performed using an Initiator 60 (Biotage). Temperatures of 40-250°C can be achieved and pressures up to 20 bar can be reached.

The conditions for the measurement of the specific rotatory index [α]₅₈₉ are as follows.
Instrument: Automatic Polarimeter SEPA-500 (HORIBA, Ltd.)
Solvent: methanol
Optical path length: 50 mm

The compound names as used herein are named using the naming software ACD/NAME (registered trademark, Advanced Chemistry Development Inc.), which is naming software that complies with IUPAC rules, ChemBioDraw (version 19.1, Cambridge Soft, Inc.), or in accordance with IUPAC nomenclature.

The "r" and "s" (lower case) in the compound names indicate the stereochemistry of the pseudo-chiral carbon atom according to the IUPAC rule.

### Reference Example 1

### Methyl 3-bromo-4-(difluoromethoxy)benzoate

To a mixture of methyl 3-bromo-4-hydroxybenzoate (10.0 g) and DMF (87 mL), sodium chlorodifluoroacetate (13.2 g) and potassium carbonate (8.97 g) were added sequentially at room temperature. The mixture was stirred at 100°C for 4 hours. The reaction solution was diluted with ethyl acetate, washed with water and saturated brine, and dried over anhydrous sodium sulfate. The solvent was removed under reduced pressure, and the residue was purified by silica gel column chromatography to afford the title compound (9.5 g).

### Reference Example 2

### Methyl 4-(difluoromethoxy)-3-[(trimethylsilyl)ethynyl] benzoate

To a mixture of methyl 3-bromo-4-(difluoromethoxy)benzoate (4.0 g), DMF (14 mL) and TEA (14 mL), ethynyl(trimethyl)silane (3 mL), copper(I) iodide (27 mg) and Pd(dppf)Cl₂•CH₂Cl₂ (116 mg) were added sequentially at room temperature and stirred at 80°C for 15 hours. Water was added to the reaction solution and extracted with ethyl acetate. The organic layer was washed with saturated brine and dried over anhydrous sodium sulfate. The solvent was removed under reduced pressure, and the residue was purified by silica gel column chromatography to afford the title compound (3.74 g). MS (m/z): 299.0 [M+H]⁺.

### Reference Example 3

### 4-(Difluoromethoxy)-3-[(pyrimidin-5-yl)ethynyl]benzoic acid [Step 1]

### Preparation of methyl 4-(difluoromethoxy)-3-[(pyrimidin-5-yl)ethynyl]benzoate

To a mixture of methyl 4-(difluoromethoxy)-3-[(trimethylsilyl)ethynyl]benzoate (400 mg) obtained in Reference Example 2, DMF (1.34 mL), DIPEA (1.34 mL), water (0.24 mL) and 5-bromopyridine (320 mg), hexafluorosilicic acid (0.020 mL), copper(I) iodide (13 mg) and PdCl₂(PPh₃)₂ (47 mg) were added in this order, and the mixture was degassed and stirred overnight at 80°C under argon atmosphere. After filtering off insoluble materials with Celite (registered trademark), the filtrate was washed with saturated brine, and the organic layer was dried over anhydrous magnesium sulfate. The solvent was removed under reduced pressure, and the residue was purified by silica gel column chromatography to afford the title compound (283 mg). MS (m/z): 305.0 [M+H]⁺

### [Step 2]

### Preparation of 4-(difluoromethoxy)-3-[(pyrimidin-5-yl)ethynyl]benzoic acid

To a mixture of methyl 4-(difluoromethoxy)-3-[(pyrimidin-5-yl)ethynyl]benzoate (283 mg), methanol (1.9 mL) and THF (1.9 mL), 2 M aqueous sodium hydroxide solution (1.4 mL) was added at room temperature, and the mixture was stirred overnight. The reaction solution was concentrated under reduced pressure, and the residue was neutralized with acetic acid. The precipitate was collected by filtration to afford the title compound (244 mg). MS (m/z): 291.1 [M+H]⁺

### Reference Example 4

### 4-(Difluoromethoxy)-3-[(5-fluoropyridin-3-yl)ethynyl]benzoic acid

### [Step 1]

### Preparation of methyl 4-(difluoromethoxy)-3-[(5-fluoropyridin-3-yl)ethynyl]benzoate

To a mixture of methyl 4-(difluoromethoxy)-3-[(trimethylsilyl)ethynyl]benzoate (250 mg) obtained in Reference Example 2, DMF (0.84 mL), DIPEA (0.84 mL), water (0.15 mL) and 3-bromo-5-fluoropyridine (140 mg), hexafluorosilicic acid (0.012 mL), copper(I) iodide (8.0 mg), and Pd(PPh₃)₄ (48 mg) were added in this order, and the mixture was degassed and stirred overnight at 90°C under argon atmosphere. The reaction solution was purified by silica gel column chromatography to afford the title compound (239 mg). MS (m/z): 322.1 [M+H]⁺

### [Step 2]

### Preparation of 4-(difluoromethoxy)-3-[(5-fluoropyridin-3-yl)ethynyl]benzoic acid

To a mixture of methyl 4-(difluoromethoxy)-3-[(5-fluoropyridin-3-yl)ethynyl]benzoate (239 mg), THF (2.5 mL) and methanol (0.074 mL), 2 M aqueous sodium hydroxide solution (1.1 mL) was added at room temperature, and the mixture was stirred for 5 hours. The reaction solution was concentrated under reduced pressure, diluted with water, and then 1 M hydrochloric acid was added to adjust the pH to 4. The precipitate was filtered to afford the title compound (190 mg). MS (m/z): 308.0 [M+H]⁺

### Reference Example 5

### 3-[(2-Aminopyrimidin-5-yl)ethynyl]-4-(difluoromethoxy)benzoic acid

### [Step 1]

### Preparation of methyl 3-[(2-aminopyrimidin-5-yl)ethynyl]-4-(difluoromethoxy)benzoate

To a mixture of methyl 4-(difluoromethoxy)-3-[(trimethylsilyl)ethynyl]benzoate (6.0 g) obtained in Reference Example 2, DMF (20 mL), DIPA (20 mL), water (3.6 mL) and 5-bromopyrimidin-2-amine (4.55 g), hexafluorosilicic acid (0.119 mL), copper(I) iodide (115 mg) and PdCl₂(PPh₃)₂ (423 mg) were added in this order, and the mixture was degassed and stirred at 80°C for 1 hour under argon atmosphere. Water was added to the reaction solution, and the precipitate was filtered and purified by silica gel column chromatography. The solvent was removed under reduced pressure, and ethyl acetate was added to the residue to suspend the precipitate, which was filtered to afford the title compound (4.4 g). MS (m/z): 320.0 [M+H]⁺

### [Step 2]

### Preparation of 3-[(2-aminopyrimidin-5-yl)ethynyl]-4-(difluoromethoxy)benzoic acid

To a mixture of methyl 3-[(2-aminopyrimidin-5-yl)ethynyl]-4-(difluoromethoxy)benzoate (4.4 g) and methanol (28 mL), 2 M aqueous sodium hydroxide solution (14 mL) was added at room temperature, and the mixture was stirred at 80°C for 3 hours. Insoluble materials were filtered off using Celite (registered trademark), and the filtrate was concentrated under reduced pressure. After dilution with water, concentrated hydrochloric acid was added to neutralize. The precipitate was filtered to afford the title compound (3.93 g). MS (m/z): 306.0 [M+H]⁺

### Reference Example 6

### 3-[(5-Aminopyrazin-2-yl)ethynyl]-4-(difluoromethoxy)benzoic acid

### [Step 1]

### Preparation of methyl 3-[(5-aminopyrazin-2-yl)ethynyl]-4-(difluoromethoxy)benzoate

To a mixture of methyl 4-(difluoromethoxy)-3-[(trimethylsilyl)ethynyl]benzoate (1.12 g) obtained in Reference Example 2, DMF (4.1 mL), DIPA (4.1 mL), water (0.52 mL) and 5-bromopyrazin-2-amine (500 mg), hexafluorosilicic acid (0.025 mL), copper(I) iodide (16 mg) and Pd(PPh₃)₄ (100 mg) were added in this order, and the mixture was degassed and stirred at 80°C for 3 hours under argon atmosphere. Water and ethyl acetate were added to the reaction solution, and the precipitate was collected by filtration to afford the title compound (600 mg). MS (m/z): 320.1 [M+H]⁺

### [Step 2]

### Preparation of 3-[(5-aminopyrazin-2-yl)ethynyl]-4-(difluoromethoxy)benzoic acid

To a mixture of methyl 3-[(5-aminopyrazin-2-yl)ethynyl]-4-(difluoromethoxy)benzoate (600 mg) and methanol (3.8 mL), 2 M aqueous sodium hydroxide solution (2.8 mL) was added at room temperature, and the mixture was stirred at 50°C for 2 hours. The reaction solution was concentrated under reduced pressure, diluted with water, and neutralized with concentrated hydrochloric acid under ice cooling. The precipitate was collected by filtration to afford the title compound (500 mg). MS (m/z): 306.0 [M+H]⁺

### Reference Example 7

### 3-[(2-Amino-4-methoxypyrimidin-5-yl)ethynyl]-4-(difluoromethoxy)benzoic acid

### [Step 1]

### Preparation of methyl 3-[(2-amino-4-methoxypyrimidin-5-yl)ethynyl]-4-(difluoromethoxy)benzoate

To a mixture of methyl 4-(difluoromethoxy)-3-[(trimethylsilyl)ethynyl]benzoate (3.22 g) obtained in Reference Example 2, DMF (20 mL), DIPEA (20 mL), water (1.8 mL) and 5-bromo-4-methoxypyrimidin-2-amine (2.00 g), hexafluorosilicic acid (0.14 mL), copper(I) iodide (93 mg) and Pd(PPh₃)₄ (566 mg) were added in this order, and the mixture was degassed and stirred at 95°C for 15 hours under argon atmosphere. Water was added to the reaction solution, and the precipitate was collected by filtration. The solid as obtained was suspended in methanol, and the suspension was stirred for 1 hour, and then filtered to afford the title compound (1.92 g). MS (m/z): 350.1 [M+H]⁺

### [Step 2]

### Preparation of 3-[(2-amino-4-methoxypyrimidin-5-yl)ethynyl]-4-(difluoromethoxy)benzoic acid

To a mixture of methyl 3-[(2-amino-4-methoxypyrimidin-5-yl)ethynyl]-4-(difluoromethoxy)benzoate (1.92 g), THF (28 mL) and methanol (5.5 mL), 2 M aqueous sodium hydroxide solution (8.3 mL) was added at room temperature, and the mixture was stirred at 40°C for 2 hours. The reaction solution was concentrated under reduced pressure, and 2 M hydrochloric acid was added to adjust the pH to 3. The precipitate was collected by filtration to afford the title compound (1.78 g). MS (m/z): 336.2 [M+H]⁺

### Reference Example 8

### 4-(Difluoromethoxy)-3-[(1-methyl-1H-pyrazol-4-yl)ethynyl]benzoic acid

### [Step 1]

### Preparation of methyl 4-(difluoromethoxy)-3-[(1-methyl-1H-pyrazol-4-yl)ethynyl]benzoate

A mixture of methyl 4-(difluoromethoxy)-3-[(trimethylsilyl)ethynyl]benzoate (344 mg) obtained in Reference Example 2, 4-iodo-1-methylpyrazole (200 mg), DMF (3.2 mL) and DIPEA (0.96 mL) was degassed, and then Pd(PPh₃)₄ (56 mg) and copper(I) iodide (9.2 mg) were added under argon atmosphere, and the mixture was degassed again and placed under argon atmosphere. Then, hexafluorosilicic acid (0.014 mL) and water (0.17 mL) were added in sequence, and the mixture was stirred at 80°C for 11 hours. Ethyl acetate was added to the reaction solution, and the mixture was filtered through Celite (registered trademark). The filtrate was washed with saturated brine, and the organic layer was dried over anhydrous magnesium sulfate. The solvent was removed under reduced pressure, and the residue was purified by silica gel column chromatography to afford the title compound (234 mg). MS (m/z): 307.1 [M+H]⁺

### [Step 2]

### Preparation of 4-(difluoromethoxy)-3-[(1-methyl-1H-pyrazol-4-yl)ethynyl]benzoic acid

To a mixture of methyl 4-(difluoromethoxy)-3-[(1-methyl-1H-pyrazol-4-yl)ethynyl]benzoate (234 mg), methanol (2.0 mL), and THF (2.0 mL), 2 M aqueous sodium hydroxide solution (1.9 mL) was added at room temperature, and the mixture was stirred for 4 hours. The reaction mixture was concentrated under reduced pressure, diluted with water, and neutralized with acetic acid. The precipitate was collected by filtration to give the title compound (204 mg). MS (m/z): 293.1 [M+H]⁺

### Reference Example 9

### (1S,2S,4S)-2-Amino-4-(trifluoromethoxy)cyclopentan-1-ol hydrochloride

### [Step 1]

### Preparation of (1S,3S,4S)-3-azido-4-(benzyloxy)cyclopentan-1-ol

To a mixture of cis-1-acetoxycyclopentane 3,4-epoxide (200 g) (synthesized, for example, according to the method described in J. Org. Chem. 1973, 38(24), 4122-4126) and trimethylsilyl azide (228 mL), (R, R) - (-) -N, N'-bis (3, 5-di-tert-butylsalicylidene)-1,2-cyclohexanediaminorhodium(III) chloride (9.9 g) was added under ice-bath cooling, and the mixture was stirred at room temperature for 2 days. The reaction solution was concentrated under reduced pressure, hexane (703 mL) and triethylsilane (337 mL) were added, and trimethylsilyl trifluoromethanesulfonate (12.7 mL) was then added under ice-bath cooling, followed by dropwise addition of benzaldehyde (172 mL) and stirring for 1 hour. Then, potassium carbonate (292 g) and methanol (352 mL) were added and stirred at 80°C for 2 hours. The reaction solution was cooled to room temperature, diluted with ethyl acetate, filtered through silica gel, and the silica gel was washed with ethyl acetate, followed by distilling off the solvent under reduced pressure. The residue was purified by silica gel column chromatography to afford the title compound (238 g). ¹H-NMR (400 MHz, CDCl₃) δ 7.40-7.28 (m, 5H), 4.63-4.54 (m, 2H), 4.40-4.31 (m, 1H), 4.19-4.12 (m, 1H), 3.92-3.86 (m, 1H), 2.24-2.07 (m, 3H), 1. 99-1.83 (m, 2H).

### [Step 2]

### Preparation of ({[(1S,2S,4S)-2-azido-4-(trifluoromethoxy)cyclopentyl]oxy}methyl)benzene

Silver trifluoromethanesulfonate (220 g), potassium fluoride (54.8 g) and toluene (429 mL) were stirred at room temperature until homogenous. 2-Fluoropyridine (111 mL) was added at room temperature. (1S,3S,4S)-3-Azido-4-(benzyloxy)cyclopentan-1-ol (100 g), Selectfluor (registered trademark) (227 g), 2,6-di-tert-butyl-4-methylphenol (9.4 g), and dehydrated ethyl acetate (1.43 L) were added at 20°C, and the mixture was degassed. Under argon atmosphere, (trifluoromethyl)trimethylsilane (127 mL) was added dropwise over 3 hours at 20-23°C, and the mixture was stirred for 2 hours. The reaction solution was diluted with hexane, filtered through silica gel, the silica gel was washed with hexane/ethyl acetate (1/1), and the solvent was removed under reduced pressure. The residue was purified by silica gel column chromatography to afford the title compound (95 g). ¹H-NMR (400 MHz, CDCl₃) δ 7.41-7.28 (m, 5H), 4.76-4.67 (m, 1H), 4.62-4.52 (m, 2H), 4.12-4.03 (m, 1H), 3.85-3.79 (m, 1H), 2.52-2.40 (m, 1H), 2.35-2.25 (m, 1H), 2.07-1.94 (m, 2H).

### [Step 3]

### Preparation of tert-butyl [(1S,2S,4S)-2-hydroxy-4-(trifluoromethoxy)cyclopentyl]carbamate

A mixture of ({[(1S,2S,4S)-2-azido-4-(trifluoromethoxy)cyclopentyl]oxy}methyl)benzene (15.7 g), 5% Pd-C (4.71 g), methanol (52 mL) and concentrated hydrochloric acid (6.51 mL) was stirred at 50°C under hydrogen atmosphere (0.4 MPa) for 6 hours. The reaction solution was cooled to 0°C, and TEA (14.5 mL) and Boc₂O (18.0 mL) were added and stirred at room temperature for 1 hour. Insoluble materials were filtered off through Celite (registered trademark), washed with methanol, and the filtrate was concentrated under reduced pressure. Water was added to the residue, which was extracted with ethyl acetate and dried over anhydrous sodium sulfate. The solvent was removed under reduced pressure, and the residue was purified by silica gel column chromatography to afford the title compound (9.0 g). With reference to, for example, Tetrahedron, 2013, 69, 5407-5412, the optical purity was increased to 100% ee by optical resolution using lipase (a mixture of the title compound, Candida antarctica-derived lipase B (supported on acrylic resin) (0.05 g/g), vinyl acetic acid, TEA and 1,4-dioxane was stirred at 40°C for 19 hours). A mixture of the title compound, 3,5-dinitrobenzene chloride, pyridine and THF was stirred at 60°C for 1 hour to afford a 3,5-dinitrobenzene ester derivative of the title compound, and the optical purity was determined by chiral HPLC. ¹H-NMR (400 MHz, CD₃OD) δ 4.83-4.73 (m, 1H), 3.97-3.88 (m, 1H), 3.88-3.79 (m, 1H), 2.53-2.40 (m, 1H), 2.30-2.17 (m, 1H), 1.95-1.83 (m, 1H), 1.83-1.72 (m, 1H), 1.44 (s, 9H). HPLC analysis: Method A, t (1S, 2S, 4S) isomer = 15.7 min.

### [Step 4]

### Preparation of (1S,2S,4S)-2-amino-4-(trifluoromethoxy)cyclopentan-1-ol hydrochloride

tert-Butyl [(1S,2S,4S)-2-hydroxy-4-(trifluoromethoxy)cyclopentyl]carbamate (3.25 g), methanol (11 mL) and hydrogen chloride (4 M in 1,4-dioxane, 1.7 mL) were stirred at 60°C for 30 minutes. The reaction solution was concentrated under reduced pressure, diethyl ether was added to the residue, and the precipitate was collected by filtration to afford the title compound (2.48 g). MS (m/z): 186.1 [M+H]⁺, ¹H-NMR (400 MHz, D₂O) δ 4.85-4.77 (m, 1H), 4.18-4.09 (m, 1H), 3.60-3.51 (m, 1H), 2.53-2.41 (m, 1H), 2.41-2.31 (m, 1H), 2.02-1.91 (m, 1H), 1.89-1.79 (m, 1H). [α] ₅₈₉²⁰ = +24.3° (c=1.0, MeOH)

### Reference Example 10

### Methyl 4-(difluoromethoxy)-3-[(Z)-2-fluoro-2-(4,4,5,5-tetramethyl-1,3,2-dioxaborolan-2-yl)ethenyl]benzoate

### [Step 1]

### Preparation of methyl 4-(difluoromethoxy)-3-ethenylbenzoate

A mixture of methyl 3-bromo-4-(difluoromethoxy)benzoate (10.0 g) obtained in Reference Example 1, 2-ethenyl-4,4,5,5-tetramethyl-1,3,2-dioxaborolane (9.0 mL), tripotassium phosphate (15.1 g), Pd(dppf)Cl₂·CH₂Cl₂ (1.45 g), 1,4-dioxane (71 mL), and water (12 mL) was stirred overnight at 80°C under argon atmosphere. Water was added to the reaction solution, which was then extracted with ethyl acetate. The organic layer was dried over anhydrous sodium sulfate. The solvent was removed under reduced pressure, and the residue was purified by silica gel column chromatography to give the title compound (6.84 g). MS (m/z): 299.1 [M+H]⁺

### [Step 2]

### Preparation of methyl 4-(difluoromethoxy)-3-formylbenzoate

A mixture of methyl 4-(difluoromethoxy)-3-ethenylbenzoate (6.84 g), methylene chloride (60 mL) and methanol (60 mL) was cooled to -78°C and stirred for 4 hours while bubbling O₃. After then bubbling oxygen gas for 15 minutes, PPh₃ (9.43 g) was added at -78°C and stirred at room temperature for 1 hour. The reaction solution was concentrated under reduced pressure, and the residue was purified by silica gel column chromatography to afford the title compound (5.6 g).

### [Step 3]

### Preparation of methyl 3-(2,2-difluoroethenyl)-4-(difluoromethoxy)benzoate

To a mixture of methyl 4-(difluoromethoxy)-3-formylbenzoate (5.6 g), PPh₃ (7.7 g) and DMF (49 mL), a solution of sodium chlorodifluoroacetate (5.6 g) in DMF (24 mL) was added dropwise using a dropping funnel at 100°C, and the mixture was stirred at 100°C for 30 minutes. Water was added to the reaction solution, which was then extracted with ethyl acetate and dried over anhydrous sodium sulfate. The solvent was removed under reduced pressure, and the residue was purified by silica gel column chromatography to afford the title compound (5.1 g).

### [Step 4]

### Preparation of methyl 4-(difluoromethoxy)-3-[(Z)-2-fluoro-2-(4,4,5,5-tetramethyl-1,3,2-dioxaborolan-2-yl)ethenyl]benzoate

To a mixture of methyl 3-(2,2-difluoroethenyl)-4-(difluoromethoxy)benzoate (1.0 g), bis(pinacolato)diboron (1.44 g), potassium acetate (557 mg), tricyclohexylphosphine (106 mg) and copper(I) chloride (19 mg), THF (12 mL) was added and stirred at 40°C for 4 hours. A saturated aqueous solution of ammonium chloride was added to the reaction solution, and the mixture was extracted with ethyl acetate. After drying with anhydrous sodium sulfate, the solvent was removed under reduced pressure, followed by addition of hexane/ethyl acetate (1/1) and filtration, and the filtrate was concentrated under reduced pressure. Hexane was added to the residue, and the resulting precipitate was collected by filtration to afford the title compound (1.05 g). ¹H-NMR (400 MHz, CDCl₃) δ 8.66 (s, 1H), 7.98 (dd, 1H), 7.17 (d, 1H), 6.69 (d, 1H), 6.60 (t, 1H), 3.91 (s, 3H), 1.35 (s, 12H).

### Reference Example 11

### 3-[(Z)-2-(5-Aminopyrazin-2-yl)-2-fluoroethenyl]-4-(difluoromethoxy)benzoic acid

### [Step 1]

### Preparation of methyl 3-[(Z)-2-(5-aminopyrazin-2-yl)-2-fluoroethenyl]-4-(difluoromethoxy)benzoate

A mixture of methyl 4-(difluoromethoxy)-3-[(Z)-2-fluoro-2-(4,4,5,5-tetramethyl-1,3,2-dioxaborolan-2-yl)ethenyl]benzoate (600 mg), 5-bromopyrazin-2-amine (286 mg), sodium carbonate (513 mg), Pd (dppf) Cl₂ ·CH₂Cl₂ (66 mg), 1,4-dioxane (2.0 mL) and water (0.54 mL) was degassed and then stirred at 90°C under argon atmosphere for 4 hours. Water was added to the reaction solution, and the mixture was extracted with ethyl acetate. The organic layer was washed with saturated brine and dried over anhydrous sodium sulfate. The solvent was distilled off under reduced pressure, and the residue was purified by silica gel column chromatography to afford the title compound (430 mg). MS (m/z): 340.0 [M+H]⁺

### [Step 2]

### Preparation of 3-[(Z)-2-(5-aminopyrazin-2-yl)-2-fluoroethenyl]-4-(difluoromethoxy)benzoic acid

To a mixture of methyl 3-[(Z)-2-(5-aminopyrazin-2-yl)-2-fluoroethenyl]-4-(difluoromethoxy)benzoate (430 mg), methanol (1.3 mL) and THF (1.3 mL), 2 M aqueous sodium hydroxide solution (3.2 mL) was added at room temperature, and the mixture was stirred overnight. The reaction solution was concentrated under reduced pressure, and 1 M hydrochloric acid was added to adjust the pH to 3. The precipitate was collected by filtration to afford the title compound (350 mg). MS (m/z): 326.1 [M+H]⁺

### Reference Example 12

### (2S)-2-Amino-4-(trifluoromethoxy)butan-1-ol hydrochloride

### [Step 1]

### Preparation of methyl N-[(benzyloxy)carbonyl]-O-(trifluoromethyl)-L-homoserinate

Potassium fluoride (6.7 g) in an eggplant flask was heated with a heat gun under reduced pressure for 1 minute. The temperature was returned to room temperature, and silver trifluoromethanesulfonate (26.9 g), 2-fluoropyridine (13.5 mL) and dehydrated ethyl acetate (262 mL) were added and stirred at room temperature for 15 minutes. To this reaction solution, 2,6-di-tert-butyl-4-methylphenol (1.15 g), Selectfluor (registered trademark) (27.8 g) and methyl N-[(benzyloxy)carbonyl]-L-homoserinate (14 g) (synthesized, for example, according to the method described in WO 2009/127546) in ethyl acetate (52 mL) were added. (Trifluoromethyl)trimethylsilane (13.9 mL) was added using a dropping funnel, and the mixture was stirred at room temperature for 72 hours under light-shielded conditions. The reaction solution was filtered through silica gel, and the silica gel was washed with ethyl acetate. The filtrate was evaporated under reduced pressure, and the residue was purified by silica gel column chromatography to afford the title compound (10.5 g). MS (m/z): 336.1 [M+H]⁺

### [Step 2]

### Preparation of benzyl [(2S)-1-hydroxy-4-(trifluoromethoxy)butan-2-yl]carbamate

To a mixture of methyl N-[(benzyloxy)carbonyl]-O-(trifluoromethyl)-L-homoserinate (10.5 g) and methanol (31 mL), sodium borohydride (3.55 g) was added under ice-bath cooling and stirred for 15 minutes, then stirred at room temperature for 5 hours. The reaction solution was concentrated under reduced pressure, and water was added, followed by extraction with ethyl acetate. After drying over anhydrous sodium sulfate, the solvent was removed under reduced pressure, and the residue was purified by silica gel column chromatography to afford the title compound (9.2 g). MS (m/z): 308.1 [M+H]⁺

### [Step 3]

### Preparation of (2S)-2-amino-4-(trifluoromethoxy)butan-1-ol hydrochloride

Benzyl [(2S)-1-hydroxy-4-(trifluoromethoxy)butan-2-yl]carbamate (300 mg), methanol (3.9 mL), hydrogen chloride (2 M methanol solution, 0.49 mL) and 10% Pd-C (100 mg) were added and stirred at room temperature under hydrogen atmosphere (0.3 MPa) for 4 hours. After filtering off insoluble materials using Celite (registered trademark), the filtrate was concentrated under reduced pressure to afford the title compound (200 mg). ¹H-NMR (400 MHz, CD₃OD) δ: 4.25-4.16 (m, 2H), 3.80 (dd, 1H), 3.61 (dd, 1H), 3.41-3.34 (m, 1H), 2.14-2.00 (m, 2H).

### Reference Example 13

### 5-[(Z)-2-(2-Aminopyrimidin-5-yl)-2-fluoroethenyl]-6-ethylpyridine-3-carboxylic acid

### [Step 1]

### Preparation of ethyl 5-ethenyl-6-ethylpyridine-3-carboxylate

A mixture of ethyl 5-bromo-6-ethylpyridine-3-carboxylate (1.0 g) (synthesized, for example, according to the method described in WO 2013/164769), 2-ethenyl-4,4,5,5-tetramethyl-1,3,2-dioxaborolane (0.9 mL), tripotassium phosphate (1.64 g), Pd(dppf)Cl₂ ·CH₂Cl₂ (158 mg), 1,4-dioxane (13 mL) and water (1.3 mL) was stirred at 80°C for 1 hour. Water was added to the reaction solution, which was then extracted with ethyl acetate and dried over anhydrous sodium sulfate. The solvent was removed under reduced pressure, and the residue was purified by silica gel column chromatography to give the title compound (180 mg). MS (m/z): 206.1 [M+H]⁺

### [Step 2]

### Preparation of ethyl 6-ethyl-5-formylpyridine-3-carboxylate

A mixture of ethyl 5-ethenyl-6-ethylpyridine-3-carboxylate (350 mg), methylene chloride (5.7 mL) and methanol (5.7 mL) was cooled to -78°C and stirred for 1 hour while bubbling ozone. PPh₃ (537 mg) was added at -78°C and stirred at room temperature for 1 hour. The reaction solution was concentrated under reduced pressure, and the residue was purified by silica gel column chromatography to afford the title compound (165 mg). MS (m/z): 208.1 [M+H]⁺

### [Step 3]

### Preparation of ethyl 5-(2,2-difluoroethenyl)-6-ethylpyridine-3-carboxylate

To a mixture of ethyl 6-ethyl-5-formylpyridine-3-carboxylate (165 mg), PPh₃ (251 mg) and DMF (1.6 mL), sodium chlorodifluoroacetate (158 mg) in DMF (0.4 mL) was added dropwise over 30 minutes using a syringe pump at 100°C, and the mixture was stirred at 100°C for 30 minutes. Water was added to the reaction solution, which was then extracted with ethyl acetate and dried over anhydrous sodium sulfate. The solvent was removed under reduced pressure, and the residue was purified by silica gel column chromatography to afford the title compound (130 mg). MS (m/z): 242.1 [M+H]⁺

### [Step 4]

### Preparation of ethyl 5-[(Z)-2-(2-aminopyrimidin-5-yl)-2-fluoroethenyl]-6-ethylpyridine-3-carboxylate

Ethyl 5-(2,2-difluoroethenyl)-6-ethylpyridine-3-carboxylate (160 mg), bis(pinacolato)diboron (337 mg), potassium acetate (130 mg), tricyclohexylphosphine (74 mg) and copper(I) chloride (13 mg) were added with THF (4.4 mL) and stirred at 50°C for 3 hours. A saturated aqueous solution of ammonium chloride was added to the reaction solution, which was then extracted with ethyl acetate. After drying over anhydrous sodium sulfate, the solvent was distilled off under reduced pressure to afford a residue, to which 5-bromopyrimidin-2-amine (115 mg), Pd(dppf)Cl₂ ·CH₂Cl₂ (54 mg), potassium carbonate (183 mg), 1,4-dioxane (2.2 mL) and water (0.22 mL) were added and stirred at 80°C for 3 hours. Water was added to the reaction solution, which was then extracted with ethyl acetate and dried over anhydrous sodium sulfate. The solvent was distilled off under reduced pressure, and the residue was purified by silica gel column chromatography to afford the title compound (22 mg). MS (m/z): 317.2 [M+H]⁺

### [Step 5]

### Preparation of 5-[(Z)-2-(2-aminopyrimidin-5-yl)-2-fluoroethenyl]-6-ethylpyridine-3-carboxylic acid

To a mixture of ethyl 5-[(Z)-2-(2-aminopyrimidin-5-yl)-2-fluoroethenyl]-6-ethylpyridine-3-carboxylate (22 mg), methanol (0.070 mL), THF (0.070 mL) and water (0.070 mL), lithium hydroxide monohydrate (15 mg) was added at room temperature, and the mixture was stirred for 3 hours. The reaction solution was concentrated under reduced pressure, diluted with water, and then 1 M hydrochloric acid was added. The precipitate was collected by filtration to afford the title compound (10 mg). MS (m/z): 289.2 [M+H]⁺

### Reference Example 14

### Methyl 3-[(Z)-2-bromo-1-fluoroethenyl]-4-(difluoromethoxy)benzoate

A solution of silver nitrate (I) (140 mg) in water (1.2 mL) was added to a mixture of methyl 4-(difluoromethoxy)-3-[(trimethylsilyl)ethynyl]benzoate (4.9 g) obtained in Reference Example 2, N-bromosuccinimide (3.7 g) and acetone (55 mL), and the mixture was stirred overnight at room temperature. The reaction solution was filtered through amino silica gel, and the silica gel was washed with ethyl acetate. The filtrate was concentrated under reduced pressure, and silver fluoride (I) (6.3 g), acetonitrile (55 mL) and water (3 mL) were added to the residue, and the mixture was stirred overnight at 80°C. Insoluble materials were filtered off through Celite (registered trademark), and the filtrate was concentrated under reduced pressure. The residue was purified by silica gel column chromatography to afford the title compound (2.6 g). ¹H-NMR (400 MHz, CDCl₃) δ 8.28 (d, 1H), 8.09 (dd, 1H), 7.25-7.21 (m, 1H), 6.64 (t, 1H), 6.52 (d, 1H), 3.94 (s, 3H).

### Reference Example 15

### 3-[(Z)-2-(2-Aminopyrimidin-5-yl)-1-fluoroethenyl]-4-(difluoromethoxy)benzoic acid

### [Step 1]

### Preparation of methyl 3-[(Z)-2-(2-aminopyrimidin-5-yl)-1-fluoroethenyl]-4-(difluoromethoxy)benzoate

A mixture of methyl 3-[(Z)-2-bromo-1-fluoroethenyl]-4-(difluoromethoxy)benzoate (300 mg) obtained in Reference Example 14, (2-aminopyrimidin-5-yl)boronic acid (192 mg), potassium carbonate (220 mg), Pd(dppf)Cl₂·CH₂Cl₂ (38 mg), 1,4-dioxane (4.6 mL) and water (0.46 mL) was degassed and then stirred at 100°C for 3 hours under argon atmosphere. The reaction mixture was concentrated under reduced pressure, and the residue was purified by silica gel column chromatography to give the title compound (270 mg). MS (m/z): 340.0 [M+H]⁺

### [Step 2]

### Preparation of 3-[(Z)-2-(2-aminopyrimidin-5-yl)-1-fluoroethenyl)-4-(difluoromethoxy)benzoic acid

To a mixture of methyl 3-[(Z)-2-(2-aminopyrimidin-5-yl)-1-fluoroethenyl)-4-(difluoromethoxy)benzoate (270 mg) and methanol (4 mL), 2 M aqueous sodium hydroxide solution (1.2 mL) was added at room temperature, and the mixture was stirred at 50°C for 2 hours. The reaction solution was concentrated under reduced pressure, diluted with water, and neutralized with acetic acid. The precipitate was collected by filtration to afford the title compound (238 mg). MS (m/z): 326.0 [M+H]⁺

### Reference Example 16

### Methyl 3-[(Z)-Z-bromo-1-fluoroethenyl]-4-(difluoromethoxy)-5-fluorobenzoate

### [Step 1]

### Preparation of methyl 4-(difluoromethoxy)-3-fluoro-5-[(trimethylsilyl)ethynyl]benzoate

To a mixture of methyl 3-bromo-4-(difluoromethoxy)-5-fluorobenzoate (2.48 g) (synthesized, for example, according to the method described in WO 2020/100959), DMF (8.3 mL) and TEA (8.3 mL), ethynyl(trimethyl)silane (1.7 mL), copper(I) iodide (15.8 mg) and Pd(dppf)Cl₂·CH₂Cl₂ (67.7 mg) were added sequentially at room temperature, and the mixture was stirred overnight at 80°C under argon atmosphere. Water was added to the reaction solution, and the mixture was extracted with ethyl acetate. The organic layer was washed with water and saturated brine, and dried over anhydrous sodium sulfate. The solvent was removed under reduced pressure, and the residue was purified by silica gel column chromatography to afford the title compound (1.97 g).

### [Step 2]

### Preparation of methyl 3-[(Z)-2-bromo-1-fluoroethenyl]-4-(difluoromethoxy)-5-fluorobenzoate

A solution of silver nitrate (I) (26.8 mg) in water (1.05 mL) was added to a mixture of methyl 4-(difluoromethoxy)-3-fluoro-5-[(trimethylsilyl)ethynyl]benzoate (1.00 g), N-bromosuccinimide (703 mg) and acetone (10.5 mL), and the mixture was stirred overnight at room temperature. The reaction solution was filtered through amino silica gel, and the silica gel was washed with ethyl acetate. The filtrate was concentrated under reduced pressure, and silver fluoride (I) (1.00 g), acetonitrile (10.5 mL) and water (0.57 mL) were added to the residue, and the mixture was stirred at 80°C for 4 hours. The reaction solution was filtered through silica gel, and the silica gel was washed with ethyl acetate. The filtrate was concentrated under reduced pressure, and the residue was purified by silica gel column chromatography to afford the title compound (692 mg). ¹H-NMR (400 MHz, CDCl₃) δ 8.07 (t, 1H), 7.89 (dd, 1H), 6.71 (td, 1H), 6.60 (d, 1H), 3.95 (s, 3H).

### Reference Example 17

### 4-(Difluoromethoxy)-3-fluoro-5-[(Z)-1-fluoro-2-(5-methoxypyridin-3-yl)ethenyl]benzoic acid

A mixture of methyl 3-[(Z)-2-bromo-1-fluoroethenyl]-4-(difluoromethoxy)-5-fluorobenzoate (150 mg) obtained in Step 2 of Reference Example 16, (2-aminopyrimidin-5-yl)boronic acid (100 mg), sodium carbonate (139 mg), Pd(dppf)Cl₂·CH₂Cl₂ (35.7 mg), 1,4-dioxane (2.0 mL) and water (0.4 mL) was degassed and then stirred at 100°C for 3 hours under argon atmosphere. The reaction solution was concentrated under reduced pressure, and the residue was purified by silica gel column chromatography to afford methyl 4-(difluoromethoxy)-3-fluoro-5-[(Z)-1-fluoro-2-(5-methoxypyridin-3-yl)ethenyl]benzoate. To a mixture of this compound and methanol (4 mL), 2 M aqueous sodium hydroxide solution (0.66 mL) was added at room temperature, and the mixture was stirred at 50°C for 2 hours. The reaction solution was concentrated under reduced pressure, and neutralized with 2 M hydrochloric acid. The precipitate was collected by filtration to afford the title compound (125 mg). MS (m/z): 357.9 [M+H]⁺

### Reference Example 18

### 7-Bromoimidazo[1,5-a]pyridine-3-carboxylic acid

### [Step 1]

### Preparation of 7-bromoimidazo[1,5-a]pyridine-3-carbaldehyde

To 2,2-dimethoxyacetaldehyde (45% in 2-methoxy-2-methylpropane, 11.9 mL), acetic acid (90 mL) and ammonium acetate (5.80 g) were added water (7.4 mL) and 4-bromopyridine-2-carbaldehyde (5.00 g) and stirred at room temperature for 4 hours. Saturated sodium bicarbonate solution and water were added to the reaction solution and stirred at room temperature for 30 minutes. The precipitate was collected by filtration to afford the title compound (4.86 g). ¹H-NMR (400 MHz, DMSO-D₆) δ 9.88 (s, 1H), 9.28 (d, 1H), 8.37-8.32 (m, 1H), 7.82 (s, 1H), 7.38 (dd, 1H).

### [Step 2]

### Preparation of 7-bromoimidazo[1,5-a]pyridine-3-carboxylic acid

A separately prepared solution of sodium dihydrogen phosphate (5.04 g) in water (10 mL) was added to a suspension of 7-bromoimidazo[1,5-a]pyridine-3-carbaldehyde (3.15 g), tert-butanol (32 mL), THF (64 mL) and 2-methyl-2-butene (15.6 mL). Sodium chlorite (3.85 g) was added and stirred for 1 hour under ice-bath cooling, and then the mixture was allowed to room temperature and stirred for 1 hour. Sodium chlorite (385 mg) was added, and the mixture was further stirred at room temperature for 1 hour. 2 M Hydrochloric acid was added to the reaction solution under ice-bath cooling to adjust the pH to 5. The precipitate was collected by filtration to afford the title compound (3.23 g). MS (m/z): 240.9 [M+H]⁺

### Reference Example 19

### 3-(3-Carbamoylimidazo[1,5-a]pyridin-7-yl)-4-(trifluoromethyl)benzoic acid

### [Step 1]

### Preparation of 7-bromoimidazo[1,5-a]pyridine-3-carboxamide

To a mixture of 7-bromoimidazo[1,5-a]pyridine-3-carboxylic acid (6.34 g) obtained in Step 2 of Reference Example 18, DIPEA (13.7 mL) and DMF (88 mL), HBTU (13.0 g) was added under ice-bath cooling and stirred at room temperature for 5 minutes. Ammonium chloride (2.81 g) was added and stirred at room temperature for 2 hours. Saturated sodium bicarbonate solution and water were added to the reaction solution under ice-bath cooling. The resulting precipitate was collected by filtration to afford the title compound (5.02 g). MS (m/z): 240.0 [M+H]⁺

### [Step 2]

### Preparation of methyl 3-(3-carbamoylimidazo[1,5-a]pyridin-7-yl)-4-(trifluoromethyl)benzoate

A mixture of 7-bromoimidazo[1,5-a]pyridine-3-carboxamide (3.48 g), methyl 3-(4,4,5,5-tetramethyl-1,3,2-dioxaborolan-2-yl)-4-(trifluoromethyl)benzoate (5.54 g) (synthesized, for example, according to the method described in WO 2016/161572), sodium carbonate (4.61 g), Pd(dppf)Cl₂ ·CH₂Cl₂ (592 mg), 1,4-dioxane (73 mL) and water (7.3 mL) was degassed and stirred at 95°C under argon atmosphere for 15 hours. The reaction solution was concentrated under reduced pressure, water was added, and the resulting precipitate was collected by filtration. The resulting solid was suspended in hexane/ethyl acetate (1/1) and stirred at room temperature for 1 hour. The precipitate was collected by filtration to give the title compound (4.86 g). MS (m/z): 364.1 [M+H]⁺

### [Step 3]

### Preparation of 3-(3-carbamoylimidazo[1,5-a]pyridin-7-yl)-4-(trifluoromethyl)benzoic acid

Methyl 3-(3-carbamoylimidazo[1,5-a]pyridin-7-yl)-4-(trifluoromethyl)benzoate (4.86 g), THF (67 mL), methanol (13 mL) and 2 M aqueous sodium hydroxide solution (21 mL) were added at room temperature, and the mixture was stirred for 2 hours. The solvent was removed under reduced pressure, and 2 M hydrochloric acid was added to adjust the pH to 5. The precipitate was filtered off, suspended in ethyl acetate, and the precipitate was filtered off to afford the title compound (4.35 g). MS (m/z): 350.0 [M+H]⁺

### Reference Example 20

### 5-(3-Carbamoylimidazo[1,5-a]pyridin-7-yl)-6-(trifluoromethyl)pyridine-3-carboxylic acid

### [Step 1]

### Preparation of methyl 5-(3-carbamoylimidazo[1,5-a]pyridin-7-yl)-6-(trifluoromethyl)pyridine-3-carboxylate

A mixture of 7-bromoimidazo[1,5-a]pyridine-3-carboxamide (500 mg) obtained in Step 1 of Reference Example 19, bis(pinacolato)diboron (635 mg), potassium acetate (409 mg), Pd(dppf) Cl₂ ·CH₂Cl₂ (85 mg) and 1,4-dioxane (6.5 mL) was degassed and then stirred at 100°C under argon atmosphere for 16 hours. The mixture was allowed to room temperature, and methyl 5-bromo-6-(trifluoromethyl)pyridine-3-carboxylate (550 mg) (synthesized, for example, according to the method described in WO 2009/141398), sodium carbonate (616 mg) and water (0.97 mL) were added and stirred at 100°C for 41 hours. The reaction solution was concentrated under reduced pressure, water was added to the residue, and the resulting precipitate was collected by filtration. The resulting precipitate was purified by silica gel column chromatography to afford the title compound (211 mg). MS (m/z): 365.3 [M+H]⁺

### [Step 2]

### Preparation of 5-(3-carbamoylimidazo[1,5-a]pyridin-7-yl)-6-(trifluoromethyl)pyridine-3-carboxylic acid

To a mixture of methyl 5-(3-carbamoylimidazo[1,5-a]pyridin-7-yl)-6-(trifluoromethyl)pyridine-3-carboxylate (211 mg), THF (5.8 mL) and methanol (0.58 mL), 2 M aqueous sodium hydroxide solution (1.16 mL) was added at room temperature, and the mixture was stirred for 1 hour. The reaction solution was concentrated under reduced pressure, and 2 M hydrochloric acid was added to adjust the pH to 4. The precipitate was collected by filtration to afford the title compound (144 mg). MS (m/z): 351.2 [M+H]⁺

### Reference Example 21

### 4-Ethyl-3-[(2-phenyl-1, 3-oxazole-5-carbonyl)amino]benzoic acid

### [Step 1]

### Preparation of methyl 4-ethyl-3-[(2-phenyl-1,3-oxazole-5-carbonyl)amino]benzoate

To a mixture of 2-phenyl-1,3-oxazole-5-carboxylic acid (70 mg), methyl 3-amino-4-ethylbenzoate (70 mg), HATU (169 mg) and DMF (0.74 mL), DIPEA (0.13 mL) was added and stirred at room temperature for 2 hours. The reaction solution was purified by silica gel column chromatography to afford the title compound (84 mg). MS (m/z): 351.3 [M+H]⁺

### [Step 2]

### Preparation of 4-ethyl-3-[(2-phenyl-1,3-oxazole-5-carbonyl)amino]benzoic acid

To a mixture of methyl 4-ethyl-3-[(2-phenyl-1,3-oxazole-5-carbonyl)amino]benzoate (84 mg), methanol (0.24 mL) and THF (0.24 mL), 2 M aqueous sodium hydroxide solution (0.60 mL) was added at room temperature, and the mixture was stirred overnight. The reaction solution was concentrated under reduced pressure and neutralized with 1 M hydrochloric acid. The precipitate was collected by filtration to afford the title compound (75 mg). MS (m/z): 337.2 [M+H]⁺

### Reference Example 22

### 3-{[2-(Cyclopropylmethyl)-1,3-thiazole-5-carbonyl]amino]-4-ethylbenzoic acid

### [Step 1]

### Preparation of methyl 3-{[2-(cyclopropylmethyl)-1,3-thiazole-5-carbonyl]amino]-4-ethylbenzoate

To a mixture of 2-(cyclopropylmethyl)-1,3-thiazole-5-carboxylic acid (300 mg), HATU (747 mg) and DMF (3.3 mL), DIPEA (0.85 mL) was added and the mixture was stirred at room temperature for 10 minutes. Methyl 3-amino-4-ethylbenzoate (323 mg) was added, and the mixture was stirred at room temperature for 3 hours. Saturated aqueous sodium bicarbonate was added to the reaction solution, which was then extracted with ethyl acetate and dried over anhydrous sodium sulfate. The solvent was removed, and the residue was purified by silica gel column chromatography to afford the title compound (600 mg). MS (m/z) 345.1 [M+H]⁺

### [Step 2]

### Preparation of 3-{[2-(cyclopropylmethyl)-1,3-thiazole-5-carbonyl]amino]-4-ethylbenzoic acid

To a mixture of methyl 4-ethyl-3-[(2-phenyl-1,3-oxazole-5-carbonyl) amino] benzoate (600 mg), methanol (5.8 mL), THF (5.8 mL) and water (5.8 mL), lithium hydroxide monohydrate (200 mg) was added at room temperature, and the mixture was stirred overnight. The reaction solution was concentrated under reduced pressure and neutralized with 1 M hydrochloric acid. The precipitate was collected by filtration to afford the title compound (400 mg). MS (m/z) 331.1 [M+H]⁺

### Reference Example 23

### (1S,2S)-2-Amino-4,4-difluorocyclohexan-1-ol hydrochloride

### [Step 1]

### Preparation of (4,4-difluorocyclohexylidene)amino 4-methylbenzene-1-sulfonate

To a mixture of 4,4-difluorocyclohexanone (20 g) in acetonitrile (75 mL), 50% aqueous hydroxylamine solution (13.2 mL) was added under ice-bath cooling and stirred for 1 hour under ice-bath cooling. The mixture was concentrated under reduced pressure on a water bath at 35°C. To the residue was added water, filtered and dried to afford N-(4,4-difluorocyclohexylidene)hydroxylamine (23.6 g).

Then, 2 M aqueous sodium hydroxide solution (48 mL) and 4-methylbenzenesulfonyl chloride (17.0 g) were added to a mixture of N-(4,4-difluorocyclohexylidene)hydroxylamine (13 g) obtained above and acetonitrile (87 mL), and the mixture was stirred under ice-bath cooling for 1 hour. The mixture was concentrated under reduced pressure on a water bath at 30°C, and water was added to the residue, and the resulting precipitate was collected by filtration. The obtained solid was stirred as a slurry in hexane/ethyl acetate (10/1), collected by filtration, and dried to afford the title compound (22.8 g). MS (m/z): 304.0 [M+H]⁺

### [Step 2]

### Preparation of benzyl N-(5,5-difluoro-2-oxocyclohexyl) carbamate

To a mixture of (4,4-difluorocyclohexylidene)amino 4-methylbenzene-1-sulfonate (29 g) and toluene (640 mL), potassium ethoxide (48 mL, 24% w/w in ethanol) was added under ice-bath cooling and stirred for 1 hour. Insoluble materials were filtered and washed with diethyl ether. Sodium hydrogen carbonate (8.0 g), water (150 mL) and benzyl chloroformate (17.7 mL) were added to the filtrate, and the mixture was stirred for 30 minutes under ice-bath cooling and then stirred overnight at room temperature. The reaction solution was concentrated under reduced pressure, and THF (48 mL) and concentrated hydrochloric acid (16 mL) were added under ice-bath cooling and stirred for 15 hours at room temperature. Saturated aqueous sodium bicarbonate was added to the reaction solution, which was then extracted with ethyl acetate and dried over anhydrous sodium sulfate. The solvent was removed under reduced pressure, and the residue was purified by silica gel column chromatography to give the title compound (21.4 g). ¹H-NMR (400 MHz, CDCl₃) δ: 7.43-7.30 (m, 5H), 5.70-5.51 (m, 1H), 5.11 (s, 2H), 4.64-4.47 (m, 1H), 3.20-3.03 (m, 1H), 2.80-2.65 (m, 1H), 2.62-2.42 (m, 2H), 2.34-2.08 (m, 1H), 2.08-1.89 (m, 1H).

### [Step 3]

### Preparation of benzyl N-[(1S,2R)-5,5-difluoro-2-hydroxycyclohexyl]carbamate

Benzyl N-(5,5-difluoro-2-oxocyclohexyl)carbamate (9.84 g), [(R,R)-N-(2-amino-1,2-diphenylethyl)-p-toluenesulfonamido]chloro(p-cymene)ruthenium (II) (111 mg) and 5:2 formic acid-TEA complex (35 mL) were stirred at 50°C for 6 hours under argon atmosphere. Saturated aqueous sodium bicarbonate was added to the reaction solution, which was then extracted with ethyl acetate, washed with saturated brine, and dried over anhydrous sodium sulfate. The solvent was removed under reduced pressure, and the residue was purified by silica gel column chromatography to afford the title compound (5.78 g, 94% ee). The stereochemistry was obtained with reference to, for example, the Abstracts of the 2017 Summer Symposium of the Japanese Society for Process Chemistry, 2P-06, 2017, 178-179; Org. Lett., 2006, 8, 1, 127-130; and Tetrahedron, 2007, 63, 6755-6763.
MS (m/z): 286.0 [M+H]⁺
¹H-NMR (400 MHz, CDCl₃) δ 7.42-7.30 (m, 5H), 5.25-5.02 (m, 3H), 4.13-3.86 (m, 2H), 2.25-1.73 (m, 7H)
HPLC analysis: Method B, t(1S,2R) isomer = 8.3 min. t(1R,2S) isomer = 10.2 min.

### [Step 4]

### Preparation of benzyl N-[(1S,2R)-5,5-difluoro-2-(methanesulfonyloxy)cyclohexyl]carbamate

To a mixture of benzyl N-[(1S,2R)-5,5-difluoro-2-hydroxycyclohexyl]carbamate (5.78 g), toluene (58 mL), trimethylamine hydrochloride (194 mg) and TEA (8.47 mL), methanesulfonyl chloride (2.6 mL) was added dropwise under ice-bath cooling and stirred at room temperature for 3 hours. Water was added to the reaction solution, which was then extracted with ethyl acetate, which was washed with saturated brine and dried over anhydrous sodium sulfate. The solvent was removed under reduced pressure, and the residue was stirred as a slurry in hexane, filtered and dried to afford the title compound (7.3 g). MS (m/z): 364.1 [M+H]⁺

### [Step 5]

### Preparation of benzyl N-[(1S,2S)-5,5-difluoro-2-hydroxycyclohexyl]carbamate

DBU (6 mL) was added to a mixture of acetic acid (4.6 mL) and toluene (20 mL) and stirred at room temperature for 5 minutes. Benzyl N-[(1S,2R)-5,5-difluoro-2-(methanesulfonyloxy)cyclohexyl]carbamate (7.3 g) was added and stirred at 100°C for 6 hours, then at room temperature for 2 days, and then at 120°C for 6 hours. Water was added to the reaction solution, which was then extracted with ethyl acetate and washed with saturated brine. It was dried over anhydrous sodium sulfate. The solvent was removed under reduced pressure to afford (1S,2S)-2-{[(benzyloxy)carbonyl]amino}-4,4-difluorocyclohexyl acetate.

Then, (1S,25)-2-{[(benzyloxy)carbonyl]amino}-4,4-difluorocyclohexyl acetate obtained above, methanol (20 mL) and potassium carbonate (5.55 g) were stirred at room temperature for 15 hours. The reaction solution was filtered, and a saturated aqueous solution of ammonium chloride was added to the filtrate, which was then extracted with ethyl acetate and dried over anhydrous sodium sulfate. The solvent was removed under reduced pressure, and recrystallization in EtOAc/hexane was carried out to afford the title compound (2.53 g, 100% ee).

¹H-NMR (400 MHz, CDCl₃) & 7.41-7.30 (m, 5H), 5.17-4.93 (m, 3H), 3.87-3.74 (m, 1H), 3.74-3.61 (m, 1H), 2.70-2.54 (m, 1H), 2.51-2.37 (m, 1H), 2.22-2.06 (m, 1H), 2.03-1.93 (m, 1H), 1.92-1.66 (m, 3H)

HPLC analysis: Method B, t(1S,2S) isomer = 10.1 min.

### [Step 6]

### (1S,2S)-2-Amino-4,4-difluorocyclohexan-1-ol hydrochloride

Benzyl N-[(1S,2S)-5,5-difluoro-2-hydroxycyclohexyl]carbamate (4.2 g), methanol (30 mL), hydrogen chloride (2 M in methanol, 15 mL), and 10% Pd/C (2.1 g) were stirred overnight at room temperature under hydrogen atmosphere. After filtering off insoluble materials, the filtrate was concentrated to afford the title compound (2.7 g).
MS (m/z): 152.1 [M+H]⁺
¹H-NMR (400 MHz, D₂O) δ 3.69-3.57 (m, 1H), 3.23-3.10 (m, 1H), 2.50-2.35 (m, 1H), 2.17-1.73 (m, 4H), 1.59-1.43 (m, 1H).

### Reference Example 24

### (5S,6S)-6-Aminospiro[2.4]heptan-5-ol hydrochloride

### [Step 1]

### Preparation of (5S,6S)-6-bromospiro[2.4]heptan-5-ol

Iodine (37 g) was added to a mixture of cyclopent-3-ene-1,1-diyldimethanol (7.4 g) (synthesized, for example, according to the method described in Tetrahedron, 2009, 65, 1162-1170), PPh₃ (38 g), imidazole (12 g) and THF (290 mL), and the mixture was stirred at 80°C for 2 hours. The reaction solution was diluted with hexane, filtered using silica gel, and the silica gel was washed with hexane/ethyl acetate (10/1). The filtrate was concentrated under reduced pressure to afford 4,4-bis(iodomethyl)cyclopent-1-ene.

Then, 4,4-bis(iodomethyl)cyclopent-1-ene obtained above, indium (16 g), 1,4-dioxane (118.8 mL) and water (1.2 mL) were stirred overnight at 100°C. After filtering off the insoluble materials using Celite (registered trademark), the filtrate was concentrated under reduced pressure to afford spiro[2.4]hept-5-ene.

Then, N-bromosuccinimide (12 g) was added to spiro[2.4]hept-5-ene obtained above and water (120 mL), and the mixture was stirred at room temperature for 30 minutes. Sodium sulfite was added to the reaction mixture, and the mixture was stirred for 30 minutes. The mixture was extracted with ethyl acetate and dried over anhydrous sodium sulfate. The solvent was removed under reduced pressure, and the residue was purified by silica gel column chromatography to afford the title compound (7.1 g).

### [Step 2]

### Preparation of (SS,6S)-6-aminospiro[2.4]heptan-5-ol hydrochloride

Sodium hydroxide (4.5 g) was added to a mixture of (5S,6S)-6-bromospiro[2.4]heptan-5-ol (7.1 g) and diethyl ether (74 mL) and stirred at room temperature for 1 hour. The reaction solution was filtered using silica gel, and the silica gel was washed with diethyl ether. The filtrate was concentrated under reduced pressure to afford 6-oxaspiro[bicyclo[3.1.0]hexane-3,1'-cyclopropane].

Then, (R,R)-(-)-N,N'-bis(3,5-di-tert-butylsalicylidene)-1,2-cyclohexanediaminorhodium(III) chloride (520 mg) was added to a mixture of 6-oxaspiro[bicyclo[3.1.0]hexane-3,1'-cyclopropane] obtained above and trimethylsilyl azide (9.8 mL), and the mixture was stirred at room temperature overnight. The reaction solution was filtered using amino silica gel, and the amino silica gel was washed with hexane/ethyl acetate (10/1). The filtrate was concentrated under reduced pressure to afford {[(5S,6S)-6-azidospiro[2.4]heptan-5-yl]oxy}trimethylsilane.

Then, {[(5S,6S)-6-azidospiro[2.4]heptan-5-yl]oxy}trimethylsilane obtained above, methanol (37 mL), 5% Pd/C (2.1 g) and hydrogen chloride (4 M in 1,4-dioxane, 9.3 mL) were stirred overnight at room temperature under hydrogen atmosphere. Insoluble materials were removed by filtration using Celite (registered trademark), and the filtrate was concentrated under reduced pressure to afford the title compound (3.3 g).
¹H-NMR (400 MHz, D₂O) δ 4.29-4.11 (m, 1H), 3.54-3.36 (m, 1H), 1.96 (dd, 1H), 1.87 (dd, 1H), 1.62-1.52 (m, 2H), 0.45-0.32 (m, 4H).

### Reference Example 25

### 4-Ethyl-3-[5-(3-fluorophenyl)pyridine-3-amido]benzoic acid

### [Step 1]

### Preparation of methyl 4-ethyl-3-[S-(3-fluorophenyl)pyridine-3-amido]benzoate

To a mixture of 5-(3-fluorophenyl)pyridine-3-carboxylic acid (150 mg), HATU (315 mg) and DMF (1.4 mL), DIPEA (0.36 mL) was added and stirred at room temperature for 10 minutes. Methyl 3-amino-4-ethylbenzoate (136 mg) was added and stirred at room temperature for 3 hours. Saturated aqueous sodium bicarbonate was added to the reaction solution, which was then extracted with ethyl acetate and dried over anhydrous sodium sulfate. The solvent was removed, and the residue was purified by silica gel column chromatography to afford the title compound (200 mg). MS (m/z): 379.2 [M+H]⁺

### [Step 2]

### Preparation of 4-ethyl-3-[5-(3-fluorophenyl)pyridin-3-amido]benzoic acid

To a mixture of methyl 4-ethyl-3-[5-(3-fluorophenyl)pyridin-3-amido]benzoate (200 mg), methanol (1.1 mL) and THF (1.1 mL), 2 M aqueous sodium hydroxide solution (0.80 mL) was added at room temperature, and the mixture was stirred overnight. The reaction solution was concentrated under reduced pressure and neutralized with 1 M hydrochloric acid. The precipitate was collected by filtration to afford the title compound (160 mg).
MS (m/z): 365.1 [M+H]⁺

### Reference Example 26

### 3-[(1Z)-2-(2-Aminopyrimidin-5-yl)-2-fluoroethenyl]-4-(difluoromethoxy)benzoic acid

### [Step 1]

### Preparation of methyl 3-[(1Z)-2-(2-aminopyrimidin-5-yl)-2-fluoroethenyl]-4-(difluoromethoxy)benzoate

A mixture of methyl 4-(difluoromethoxy)-3-[(Z)-2-fluoro-2-(4,4,5,5-tetramethyl-1,3,2-dioxaborolan-2-yl)ethenyl]benzoate (1.50 g) obtained in Step 4 of Reference Example 10, 5-bromopyrimidin-2-amine (700 mg), potassium carbonate (1.10 g), Pd(dppf)Cl₂·CH₂Cl₂ (160 mg), DMF (13 mL) and water (1.3 mL) was degassed and then stirred for 4 hours at 85°C under argon atmosphere. The reaction solution was purified by silica gel column chromatography to afford the title compound (820 mg). MS (m/z): 340.0 [M+H]⁺

### [Step 2]

### Preparation of 3-[(1Z)-2-(2-aminopyrimidin-5-yl)-2-fluoroethenyl]-4-(difluoromethoxy)benzoic acid

To a mixture of methyl 3-[(1Z)-2-(2-aminopyrimidin-5-yl)-2-fluoroethenyl]-4-(difluoromethoxy)benzoate (820 mg), methanol (2.4 mL), THF (2.4 mL) and water (2.4 mL), lithium hydroxide monohydrate (530 mg) was added at room temperature, and the mixture was stirred overnight. The reaction solution was concentrated under reduced pressure and neutralized with 1 M hydrochloric acid. The precipitate was collected by filtration to afford the title compound (720 mg). MS (m/z): 326.0 [M+H]⁺

### Reference Example 27

### 3-[5-(Cyclopropylmethoxy)pyridine-3-amido]-4-ethylbenzoic acid

### [Step 1]

### Preparation of methyl 3-[5-(cyclopropylmethoxy)pyridine-3-amido]-4-ethylbenzoate

To a mixture of 5-(cyclopropylmethoxy)nicotinic acid (300 mg), HATU (709 mg) and DMF (3.1 mL), DIPEA (0.81 mL) was added and stirred at room temperature for 10 minutes. Methyl 3-amino-4-ethylbenzoate (306 mg) was added and stirred at room temperature for 3 hours. Saturated aqueous sodium bicarbonate was added to the reaction solution, which was then extracted with ethyl acetate and dried over anhydrous sodium sulfate. The solvent was removed, and the residue was purified by silica gel column chromatography to afford the title compound (500 mg). MS (m/z): 355.1 [M+H]⁺

### [Step 2]

### Preparation of 3-[5-(cyclopropylmethoxy)pyridine-3-amido]-4-ethylbenzoic acid

To a mixture of methyl 3-[5-(cyclopropylmethoxy)pyridine-3-amido]-4-ethylbenzoate (500 mg), methanol (4.7 mL), THF (4.7 mL) and water (4.7 mL), lithium hydroxide monohydrate (200 mg) was added at room temperature, and the mixture was stirred overnight. The reaction solution was concentrated under reduced pressure and neutralized with 1 M hydrochloric acid. The precipitate was collected by filtration to afford the title compound (430 mg). MS (m/z): 341.1 [M+H]⁺

### Reference Example 28

### 3-{2-[(Cyclopropylmethyl)amino]-1,3-thiazole-5-amido}-4-ethylbenzoic acid

### [Step 1]

### Preparation of methyl 3-(2-bromo-1,3-thiazole-5-amido)-4-ethylbenzoate

To a mixture of methyl 3-amino-4-ethylbenzoate (431 mg), 2-bromothiazole-5-carboxylic acid (500 mg), HATU (1.10 g) and DMF (20 mL), DIPEA (0.83 mL) was added and stirred overnight at room temperature. Saturated aqueous sodium bicarbonate was added to the reaction solution, which was then extracted with ethyl acetate and dried over anhydrous sodium sulfate. The solvent was removed, and the residue was purified by silica gel column chromatography to afford the title compound (100 mg).

### [Step 2]

### Preparation of methyl 3-{2-[(cyclopropylmethyl)amino]-1,3-thiazole-5-amido}-4-ethylbenzoate

A mixture of methyl 3-(2-bromo-1,3-thiazole-5-amido)-4-ethylbenzoate (100 mg), cyclopropylmethanamine (96 mg) and NMP (0.9 mL) was stirred overnight at 80°C. The reaction solution was purified by silica gel column chromatography to afford the title compound (65 mg). MS (m/z): 360.3 [M+H]⁺

### [Step 3]

### Preparation of 3-{2-[(cyclopropylmethyl)amino]-1,3-thiazole-5-amido}-4-ethylbenzoic acid

To a mixture of methyl 3-{2-[(cyclopropylmethyl)amino]-1,3-thiazole-5-amido}-4-ethylbenzoate (65 mg) and methanol (0.72 mL), 2 M aqueous sodium hydroxide solution (0.27 mL) was added at room temperature, and the mixture was stirred overnight. The reaction solution was concentrated under reduced pressure and neutralized with 1 M hydrochloric acid. The precipitate was collected by filtration to afford the title compound (60 mg). MS (m/z): 346.2 [M+H]⁺

### Reference Example 29

### 3-(2-Cyclopropyl-1,3-thiazole-5-amido)-4-ethylbenzoic acid [Step 1]

### Preparation of methyl 3-(2-cyclopropyl-1,3-thiazole-5-amido)-4-ethylbenzoate

To a mixture of 2-cyclopropyl-1,3-thiazole-5-carboxylic acid (70 mg), methyl 3-amino-4-ethylbenzoate (78 mg), HATU (189 mg) and DMF (0.83 mL), DIPEA (0.143 mL) was added and stirred at room temperature for 2 hours. The reaction solution was purified by silica gel column chromatography to afford the title compound (120 mg). MS (m/z): 331.1 [M+H]⁺

### [Step 2]

### Preparation of 3-(2-cyclopropyl-1,3-thiazol-5-amido)-4-ethylbenzoic acid

To a mixture of methyl 3-(2-cyclopropyl-1,3-thiazol-5-amido)-4-ethylbenzoate (120 mg), methanol (0.36 mL) and THF (0.36 mL), 2 M aqueous sodium hydroxide solution (0.90 mL) was added at room temperature, and the mixture was stirred overnight. The reaction solution was concentrated under reduced pressure and neutralized with 1 M hydrochloric acid. The precipitate was collected by filtration to afford the title compound (80 mg). MS (m/z): 317.2 [M+H]⁺

### Example 1

### 4-(Difluoromethoxy)-N-[(1S,2S,4S)-2-hydroxy-4-(trifluoromethoxy)cyclopentyl]-3-[(pyrimidin-5-yl)ethynyl]benzamide

To a solution of 4-(difluoromethoxy)-3-[(pyrimidin-5-yl)ethynyl]benzoic acid (30 mg), as obtained in Step 2 of Reference Example 3, in DMF (0.34 mL), HATU (47 mg) and DIPEA (0.054 mL) were added sequentially, and the mixture was stirred at room temperature for 15 minutes. Then, (1S,2S,4S)-2-amino-4-(trifluoromethoxy)cyclopentan-1-ol hydrochloride (27 mg) was added, and the mixture was stirred at room temperature overnight. The reaction solution was purified by silica gel column chromatography to afford the title compound (31 mg). MS (m/z): 458.1 [M+H]⁺.

### Example 2

### 4-(Difluoromethoxy)-3-[2-(5-fluoropyridin-3-yl)ethynyl]-N-[(1S,2S,4S)-2-hydroxy-4-(trifluoromethoxy)cyclopentyl]benzamide

The title compound (35 mg) was obtained following a method similar to that described in Example 1, using 4-(difluoromethoxy)-3-[(5-fluoropyridin-3-yl)ethynyl]benzoic acid (30 mg) as obtained in Step 2 of Reference Example 4 instead of 4-(difluoromethoxy)-3-[(pyrimidin-5-yl)ethynyl]benzoic acid. MS (m/z): 475.1 [M+H]⁺

### Example 3

### 3-[2-(2-Aminopyrimidin-5-yl)ethynyl]-4-(difluoromethoxy)-N-[(1S,2S,4S)-2-hydroxy-4-(trifluoromethoxy)cyclopentyl]benzamide

The title compound (35 mg) was obtained following a method similar to that described in Example 1, using 3-[(2-aminopyrimidin-5-yl)ethynyl]-4-(difluoromethoxy)benzoic acid (30 mg) as obtained in Step 2 of Reference Example 5 instead of 4-(difluoromethoxy)-3-[(pyrimidin-5-yl)ethynyl]benzoic acid. MS (m/z): 473.1 [M+H]⁺

### Example 4

### 3-[2-(5-Aminopyrazin-2-yl)ethynyl]-4-(difluoromethoxy)-N-[(1S,2S,4S)-2-hydroxy-4-(trifluoromethoxy)cyclopentyl]benzamide

The title compound (45 mg) was obtained following a method similar to that described in Example 1, using 3-[(5-aminopyrazin-2-yl)ethynyl]-4-(difluoromethoxy)benzoic acid (35 mg) obtained in Step 2 of Reference Example 6, instead of 4-(difluoromethoxy)-3-[(pyrimidin-5-yl)ethynyl]benzoic acid. MS (m/z): 473.2 [M+H]⁺

### Example 5

### 3-[2-(2-Amino-4-methoxypyrimidin-5-yl)ethynyl]-4-(difluoromethoxy)-N-[(1S,2S,4S)-2-hydroxy-4-(trifluoromethoxy)cyclopentyl]benzamide

The title compound (25 mg) was obtained following a method similar to that described in Example 1, using 3-[(2-amino-4-methoxypyrimidin-5-yl)ethynyl]-4-(difluoromethoxy)benzoic acid (30 mg) obtained in Step 2 of Reference Example 7, instead of 4-(difluoromethoxy)-3-[(pyrimidin-5-yl)ethynyl]benzoic acid. MS (m/z): 503.2 [M+H]⁺

### Example 6

### 4-(Difluoromethoxy)-N-[(1S,2S,4S)-2-hydroxy-4-(trifluoromethoxy)cyclopentyl]-3-[2-(1-methyl-1H-pyrazol-4-yl)ethynyl]benzamide

The title compound (27 mg) was obtained following a method similar to that described in Example 1, using 4-(difluoromethoxy)-3-[(1-methyl-1H-pyrazol-4-yl)ethynyl]benzoic acid (20 mg) obtained in Step 2 of Reference Example 8, instead of 4-(difluoromethoxy)-3-[(pyrimidin-5-yl)ethynyl]benzoic acid. MS (m/z): 460.2 [M+H]⁺

### Example 7

### 3-[2-(5-Aminopyrazin-2-yl)ethynyl]-4-(difluoromethoxy)-N-[(2S)-1-hydroxy-4-(trifluoromethoxy)butan-2-yl]benzamide

The title compound (35 mg) was obtained following a method similar to that described in Example 1, using 3-[(5-aminopyrazin-2-yl)ethynyl]-4-(difluoromethoxy)benzoic acid (30 mg) obtained in Step 2 of Reference Example 6 and (2S)-2-amino-4-(trifluoromethoxy)butan-1-ol hydrochloride (23 mg) obtained in Step 3 of Reference Example 12, instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 461.1 [M+H]⁺ +

### Example 8

### 3-[2-(2-Aminopyrimidin-5-yl)ethynyl]-4-(difluoromethoxy)-N-[(1S)-1-(3-fluorophenyl)-2-hydroxyethyl]benzamide hydrochloride

3-[2-(2-Aminopyrimidin-5-yl)ethynyl]-4-(difluoromethoxy)-N-[(1S)-1-(3-fluorophenyl)-2-hydroxyethyl]benzamide (34 mg) was obtained following a method similar to that described in Example 1, using 3-[(2-aminopyrimidin-5-yl)ethynyl]-4-(difluoromethoxy)benzoic acid (30 mg) obtained in Step 2 of Reference Example 5 and (2S)-2-amino-2-(3-fluorophenyl)ethanol (18 mg) instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. The obtained 3-[2-(2-aminopyrimidin-5-yl)ethynyl]-4-(difluoromethoxy)-N-[(1S)-1-(3-fluorophenyl)-2-hydroxyethyl]benzamide (34 mg) was dissolved in 1,4-dioxane (2 mL), hydrogen chloride (4 M in 1,4-dioxane, 0.020 mL) was added, and the mixture was stirred at room temperature for 20 minutes. The solvent was removed under reduced pressure to give the title compound (25 mg). MS (m/z): 443.1 [M+H]⁺

### Example 9

### 3-[2-(2-Aminopyrimidin-5-yl)ethynyl]-4-(difluoromethoxy)-N-[(2S)-1-hydroxy-3-(4-methoxyphenyl)propan-2-yl]benzamide hydrochloride

3-[2-(2-Aminopyrimidin-5-yl)ethynyl]-4-(difluoromethoxy)-N-[(2S)-1-hydroxy-3-(4-methoxyphenyl)propan-2-yl]benzamide (41 mg) was obtained following a method similar to that described in Example 1, using 3-[(2-aminopyrimidin-5-yl)ethynyl]-4-(difluoromethoxy)benzoic acid (30 mg) obtained in Step 2 of Reference Example 5 and (2S)-2-amino-3-(4-methoxyphenyl)propan-1-ol (21 mg) instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. The obtained 3-[2-(2-aminopyrimidin-5-yl)ethynyl]-4-(difluoromethoxy)-N-[(25)-1-hydroxy-3-(4-methoxyphenyl)propan-2-yl]benzamide (40 mg) was dissolved in 1,4-dioxane (2 mL), hydrogen chloride (4 M in 1,4-dioxane, 0.022 mL) was added, and the mixture was stirred at room temperature for 20 minutes. The solvent was distilled off under reduced pressure to afford the title compound (25 mg). MS (m/z): 469.1 [M+H]⁺

### Example 10

### 3-[(1Z)-2-(5-Aminopyrazin-2-yl)-2-fluoroethenyl]-4-(difluoromethoxy)-N-[(1S,2S,4S)-2-hydroxy-4-(trifluoromethoxy)cyclopentyl]benzamide

The title compound (30 mg) was obtained following a method similar to that described in Example 1, using 3-[(Z)-2-(5-aminopyrazin-2-yl)-2-fluoroethenyl]-4-(difluoromethoxy)benzoic acid (30 mg) obtained in Step 2 of Reference Example 11, instead of 4-(difluoromethoxy)-3-[(pyrimidin-5-yl)ethynyl]benzoic acid. MS (m/z): 493.2 [M+H]⁺

### Example 11

### 5-[(1Z)-2-(2-Aminopyrimidin-5-yl)-2-fluoroethenyl]-6-ethyl-N-[(1S,2S,4S)-2-hydroxy-4-(trifluoromethoxy)cyclopentyl]pyridine-3-carboxamide

The title compound (3 mg) was obtained following a method similar to that described in Example 1, using 5-[(Z)-2-(2-aminopyrimidin-5-yl)-2-fluoroethenyl]-6-ethylpyridine-3-carboxylic acid (10 mg) obtained in Step 5 of Reference Example 13, instead of 4-(difluoromethoxy)-3-[(pyrimidin-5-yl)ethynyl]benzoic acid. MS (m/z): 456.2 [M+H]⁺

### Example 12

### 3-[(1Z)-2-(2-Aminopyrimidin-5-yl)-1-fluoroethenyl]-4-(difluoromethoxy)-N-[(2S)-1-hydroxy-4-(trifluoromethoxy)butan-2-yl]benzamide

The title compound (29 mg) was obtained following a method similar to that described in Example 1, using 3-[(Z)-2-(2-aminopyrimidin-5-yl)-1-fluoroethenyl]-4-(difluoromethoxy)benzoic acid (30 mg) obtained in Step 2 of Reference Example 15 and (2S)-2-amino-4-(trifluoromethoxy)butan-1-ol hydrochloride (21 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 481.2 [M+H]⁺

### Example 13

### 9-(Difluoromethoxy)-3-fluoro-5-[(1Z)-1-fluoro-2-(5-methoxypyridin-3-yl)ethenyl]-N-[(1S,2S,4S)-2-hydroxy-4-(trifluoromethoxy)cyclopentyl]benzamide

The title compound (22 mg) was obtained following a method similar to that described in Example 1, using 4-(difluoromethoxy)-3-fluoro-5-[(Z)-1-fluoro-2-(5-methoxypyridin-3-yl)ethenyl]benzoic acid (30 mg) obtained in Reference Example 17, instead of 4-(difluoromethoxy)-3-[(pyrimidin-5-yl)ethenyl]benzoic acid. MS (m/z): 525.2

### [M+H]⁺

### Example 14

### 7-(5-{[(1S,2S)-2-Hydroxycyclohexyl]carbamoyl}-2-(trifluoromethyl)phenyl)imidazo[1,5-a]pyridine-3-carboxamide

To a mixture of 3-(3-carbamoylimidazo[1,5-a]pyridin-7-yl)-4-(trifluoromethyl)benzoic acid (22 mg) obtained in Step 3 of Reference Example 19, (1S,2S)-2-aminocyclohexan-1-ol hydrochloride (14 mg), HATU (35 mg) and DMF (0.5 mL), DIPEA (0.037 mL) was added and stirred at room temperature for 1 hour. The reaction solution was purified by silica gel column chromatography to afford the title compound (23 mg). MS (m/z): 447.3 [M+H]⁺

### Example 15

### 5-{3-Carbamoylimidazo[1,5-a]pyridin-7-yl)-N-[(1S,2S,4S)-2-hydroxy-4-(trifluoromethoxy)cyclopentyl]-6-(trifluoromethyl)pyridine-3-carboxamide

To a mixture of 5-(3-carbamoylimidazo[1,5-a]pyridin-7-yl)-6-(trifluoromethyl)pyridine-3-carboxylic acid (30 mg) obtained in Step 2 of Reference Example 20 and methylene chloride (0.29 mL), TEA (0.048 mL), EDCI-HCl (164 mg) and HOBt (12 mg) were added in this order and stirred at room temperature for 5 minutes. Then, (1S,2S,4S)-2-amino-4-(trifluoromethoxy)cyclopentan-1-ol hydrochloride (25 mg) was added and stirred at room temperature overnight. The reaction solution was purified by silica gel column chromatography to afford the title compound (35 mg). MS (m/z): 518.2 [M+H]⁺

### Example 16

### N-(2-Ethyl-5-{[(1S,2S,4S)-2-hydroxy-4-(trifluoromethoxy)cyclopentyl]carbamoyl}phenyl)-2-phenyl-1,3-oxazole-5-carboxamide

The title compound (50 mg) was obtained following a method similar to that described in Example 1, using 4-ethyl-3-[(2-phenyl-1,3-oxazole-5-carbonyl)amino]benzoic acid (40 mg) obtained in Step 2 of Reference Example 23, instead of 4-(difluoromethoxy)-3-[(pyrimidin-5-yl)ethynyl]benzoic acid. MS (m/z): 504.3 [M+H]⁺

### Example 17

### 2-(Cyclopropylmethyl)-N-(2-ethyl-5-{[(1S,2S,4S)-2-hydroxy-4-(trifluoromethoxy)cyclopentyl]carbamoyl}phenyl)-1,3-thiazole-5-carboxamide

The title compound (33 mg) was obtained following a method similar to that described in Example 1, using 3-{[2-(cyclopropylmethyl)-1,3-thiazole-5-carbonyl]amino]-4-ethylbenzoic acid (30 mg) obtained in Step 2 of Reference Example 22, instead of 4-(difluoromethoxy)-3-[(pyrimidin-5-yl)ethynyl]benzoic acid. MS (m/z): 498.2 [M+H]⁺

### Example 18

### 4-(Difluoromethoxy)-N-[(5S,6S)-6-hydroxyspiro[2.4]heptan-5-yl]-3-[2-(pyrimidin-5-yl)ethynyl]benzamide

The title compound (29 mg) was obtained following a method similar to that described in Example 1, using (5S,6S)-6-aminospiro[2.4]heptan-5-ol hydrochloride (20 mg) obtained in Step 2 of Reference Example 24, instead of (1S,2S,4S)-2-amino-4-(trifluoromethoxy)cyclopentan-1-ol hydrochloride. MS (m/z): 400.1 [M+H]⁺

### Example 19

### 4-(Difluoromethoxy)-3-[2-(5-fluoropyridin-3-yl)ethynyl]-N-((5S,6S)-6-hydroxyspiro[2.4]heptan-5-yl]benzamide

The title compound (34 mg) was obtained following a method similar to that described in Example 1, using 4-(difluoromethoxy)-3-[(5-fluoropyridin-3-yl)ethynyl]benzoic acid (30 mg) obtained in Step 2 of Reference Example 4 and (5S,6S)-6-aminospiro[2.4]heptan-5-ol hydrochloride (19 mg) obtained in Step 2 of Reference Example 24 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 417.1 [M+H]⁺

### Example 20

### 3-[2-(2-Aminopyrimidin-5-yl)ethynyl]-N-[(1S,2S)-5,5-difluoro-2-hydroxycyclohexyl]-4-(difluoromethoxy)benzamide p-toluenesulfonate

Following a method similar to that described in Example 1, 3-[(2-aminopyrimidin-5-yl)ethynyl]-4-(difluoromethoxy)benzoic acid (30 mg) obtained in Step 2 of Reference Example 5 and (1S,2S)-2-amino-4,4-difluorocyclohexan-1-ol hydrochloride (22 mg) obtained in Step 6 of Reference Example 23 were used instead of 4-(difluoromethoxy)-3-[(pyrimidin-5-yl)ethynyl]benzoic acid and (15,2S,4S)-2-amino-4-(trifluoromethoxy)cyclopentan-1-ol hydrochloride to afford 3-[2-(2-aminopyrimidin-5-yl)ethynyl]-N-[(1S,2S)-5,5-difluoro-2-hydroxycyclohexyl]-4-(difluoromethoxy) benzamide. This was dissolved in a small amount of methanol, p-toluenesulfonic acid monohydrate (22 mg) was added, and the solvent was distilled off under reduced pressure. The product was triturated in 2-propanol/ethyl acetate to afford the title compound (22 mg). MS (m/z): 439.2 [M+H]⁺

### Example 21

### 3-[2-(2-Aminopyrimidin-5-yl)ethynyl]-4-(difluoromethoxy)-N-[(5S,6S)-6-hydroxyspiro[2.4]heptan-5-yl]benzamide

The title compound (194 mg) was obtained following a method similar to that described in Example 1, using 3-[(2-aminopyrimidin-5-yl)ethynyl]-4-(difluoromethoxy)benzoic acid (200 mg) obtained in Step 2 of Reference Example 5 and (55,6S)-6-aminospiro[2.4]heptan-5-ol hydrochloride (161 mg) obtained in Step 2 of Reference Example 24 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 415.2 [M+H]⁺

### Example 22

### 3-[2-(2-Aminopyrimidin-5-yl)ethynyl]-4-(difluoromethoxy)-N-[(2S)-1-hydroxy-4-(trifluoromethoxy)butan-2-yl]benzamide

The title compound (25 mg) was obtained following a method similar to that described in Example 1, using 3-[(2-aminopyrimidin-5-yl)ethynyl]-4-(difluoromethoxy)benzoic acid (30 mg) obtained in Step 2 of Reference Example 5 and (2S)-2-amino-4-(trifluoromethoxy)butan-1-ol hydrochloride (25 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 461.2 [M+H]⁺

### Example 23

### 3-[2-(2-Amino-4-methoxypyrimidin-5-yl)ethynyl]-4-(difluoramethoxy)-N-[(5S,6S)-6-hydroxyspiro[2.4]heptan-5-yl]benzamide

The title compound (20 mg) was obtained following a method similar to that described in Example 1, using 3-[(2-amino-4-methoxypyrimidin-5-yl)ethynyl]-4-(difluoromethoxy)benzoic acid (30 mg) obtained in Step 2 of Reference Example 7 and (5S,6S)-6-aminospiro[2.4]heptan-5-ol hydrochloride (18 mg) obtained in Step 2 of Reference Example 24 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 445.2 [M+H]⁺

### Example 24

### 4-(Difluoromethoxy)-N-[(2S)-1-hydroxy-4-(trifluoromethoxy)butan-2-yl]-3-[2-(1-methyl-1H-pyrazol-4-yl)ethynyl]benzamide

The title compound (27 mg) was obtained following a method similar to that described in Example 1, using 4-(difluoromethoxy)-3-[(1-methyl-1H-pyrazol-4-yl)ethynyl]benzoic acid (30 mg) obtained in Step 2 of Reference Example 8 (30 mg) and (2S)-2-amino-4-(trifluoromethoxy)butan-1-ol hydrochloride (24 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 448.2 [M+H]⁺

### Example 25

### 3-[(1Z)-2-(5-Aminopyrazin-2-yl)-2-fluoroethenyl]-N-[(1S,2S)-5,5-difluoro-2-hydroxycyclohexyl]-4-(difluoromethoxy)benzamide

The title compound (19 mg) was obtained following a method similar to that described in Example 1, using 3-[(Z)-2-(5-aminopyrazin-2-yl)-2-fluoroethenyl]-4-(difluoromethoxy)benzoic acid (30 mg) obtained in Step 2 of Reference Example 11 and (1S,2S)-2-amino-4,4-difluorocyclohexan-1-ol hydrochloride (21 mg) obtained in Step 6 of Reference Example 23 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 459.1 [M+H]⁺

### Example 26

### 3-[(1Z)-2-(5-Aminopyrazin-2-yl)-2-fluoroethenyl]-4-(difluoromethoxy)-N-[(5S,6S)-6-hydroxyspiro[2.4]heptan-5-yl]benzamide

The title compound (32 mg) was obtained following a method similar to that described in Example 1, using 3-[(Z)-2-(5-aminopyrazin-2-yl)-2-fluoroethenyl]-4-(difluoromethoxy)benzoic acid (30 mg) obtained in Step 2 of Reference Example 11 and (5S,6S)-6-aminospiro[2.4]heptan-5-ol hydrochloride (17 mg) obtained in Step 2 of Reference Example 24 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 435.2 [M+H]⁺

### Example 27

### 7-(S-{[(1S,2S)-5,5-Difluoro-2-hydroxycyclohexyl]carbamoyl}-2-(trifluoromethyl)phenyl)imidazo[1,5-a]pyridine-3-carboxamide

To a solution of 3-(3-carbamoylimidazo[1,5-a]pyridin-7-yl)-4-(trifluoromethyl)benzoic acid (30 mg) obtained in Step 3 of Reference Example 19 in DMF (0.29 mL), DIPEA (0.045 mL) and HBTU (42 mg) were added in this order and stirred at room temperature for 15 minutes. Then, (1S,2S)-2-amino-4,4-difluorocyclohexan-1-ol hydrochloride (18 mg) obtained in Step 6 of Reference Example 23 was added and stirred at room temperature for 2 hours. The reaction mixture was purified by silica gel column chromatography to give the title compound (28 mg). MS (m/z): 483.2 [M+H]⁺

### Example 28

### 7-(5-{[(5S,6S)-6-Hydroxyspirol[2.4]heptan-5-yl]carbamoyl}-2-(trifluoromethyl)phenyl)imidazo[1,5-a]pyridine-3-carboxamide

The title compound (26 mg) was obtained following a method similar to that described in Example 27, using (5S,6S)-6-aminospiro[2.4]heptan-5-ol hydrochloride (15 mg) obtained in Step 2 of Reference Example 24, instead of (1S,2S)-2-amino-4,4-difluorocyclohexan-1-ol hydrochloride. MS (m/z): 459.2

### [M+H]⁺

### Example 29

### 7-(5-{[(1S,2S,4S)-2-Hydroxy-4-(trifluoromethoxy)cyclopentyl]carbamoyl}-2-(trifluoromethyl)phenyl)imidazo[1,5-a]pyridine-3-carboxamide

The title compound (23 mg) was obtained following a method similar to that described in Example 27, using (1S,2S,4S)-2-amino-4-(trifluoromethoxy)cyclopentan-1-ol hydrochloride (25 mg) obtained in Step 4 of Reference Example 9, instead of (1S,2S)-2-amino-4,4-difluorocyclohexan-1-ol hydrochloride. MS (m/z): 517.1 [M+H]⁺

### Example 30

### 5-{3-Carbamoylimidazo[1,5-a]pyridin-7-yl}-N-[(2S)-1-hydroxy-4-(trifluoromethoxy)butan-2-yl]-6-(trifluoromethyl) pyridine-3-carboxamide

The title compound (31 mg) was obtained following a method similar to that described in Example 15, using (2S)-2-amino-4-(trifluoromethoxy)butan-1-ol hydrochloride (23 mg) obtained in Step 3 of Reference Example 12, instead of (1S,2S,4S)-2-amino-4-(trifluoromethoxy)cyclopentan-1-ol

### hydrochloride. MS (m/z): 506.3 [M+H]⁺

### Example 31

### 7-(5-{[(2S)-1-Hydroxy-4-(trifluoromethoxy)butan-2-yl]carbamoyl}-2-(trifluoromethyl)phenyl)imidazo[1,5-a]pyridine-3-carboxamide

The title compound (22 mg) was obtained following a method similar to that described in Example 27, using (2S)-2-amino-4-(trifluoromethoxy)butan-1-ol hydrochloride (23 mg) obtained in Step 3 of Reference Example 12, instead of (1S,2S)-2-amino-4,4-difluorocyclohexan-1-ol hydrochloride. MS (m/z): 505.1 [M+H]⁺

### Example 32

### N-(2-Ethyl-5-{[(2S)-1-hydroxy-4-(trifluoromethoxy)butan-2-yl]carbamoyl}phenyl)-5-(3-fluorophenyl)pyridine-3-carboxamide

The title compound (29 mg) was obtained following a method similar to that described in Example 1, using 4-ethyl-3-[5-(3-fluorophenyl)pyridin-3-amido]benzoic acid (30 mg) obtained in Step 2 of Reference Example 25 and (2S)-2-amino-4-(trifluoromethoxy)butan-1-ol hydrochloride (19 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 520.3 [M+H]⁺

### Example 33

### N-(2-Ethyl-5-{[(1S,2S,4S)-2-hydroxy-4-(trifluoromethoxy)cyclopentyl]carbamoyl}phenyl)-5-(3-fluorophenyl)pyridine-3-carboxamide

The title compound (26 mg) was obtained following a method similar to that described in Example 1, using 4-ethyl-3-[5-(3-fluorophenyl)pyridine-3-amido]benzoic acid (30 mg) obtained in Step 2 of Reference Example 25, instead of 4-(difluoromethoxy)-3-[(pyrimidin-5-yl)ethynyl]benzoic acid. MS (m/z): 532.3 [M+H]⁺

### Example 34

### 3-[(1Z)-2-(2-Aminopyrimidin-5-yl)-2-fluoroethenyl]-4-(difluoromethoxy)-N-[(1S)-1-(3-fluorophenyl)-2-hydroxyethyl]benzamide

The title compound (24 mg) was obtained following a method similar to that described in Example 1, using 3-[(1Z)-2-(2-aminopyrimidin-5-yl)-2-fluoroethenyl]-4-(difluoromethoxy)benzoic acid (30 mg) obtained in Step 2 of Reference Example 26 and (2S)-2-amino-(3-fluorophenyl)ethanol (17 mg) instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 463.1 [M+H]⁺

### Example 35

### 3-[2-(2-Aminopyrimidin-5-yl)ethynyl]-N-[(2S)-1-(4-chlorophenyl)-3-hydroxypropan-2-yl]-4-(difluoromethoxy)benzamide hydrochloride

Following a method similar to that described in Example 1, 3-[2-(2-aminopyrimidin-5-yl)ethynyl]-N-[(2S)-1-(4-chlorophenyl)-3-hydroxypropan-2-yl]-4-(difluoromethoxy)benzamide (43 mg) was obtained using 3-[(2-aminopyrimidin-5-yl)ethynyl]-4-(difluoromethoxy)benzoic acid (30 mg) obtained in Step 2 of Reference Example 5 and (3S)-3-amino-3-(4-chlorophenyl)-1-propanol (24 mg) instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. The obtained 3-[2-(2-aminopyrimidin-5-yl)ethynyl]-N-[(2S)-1-(4-chlorophenyl)-3-hydroxypropan-2-yl]-4-(difluoromethoxy)benzamide (43 mg) was dissolved in methanol (2 mL), hydrogen chloride (4 M in 1,4-dioxane, 0.023 mL) was added, and the mixture was stirred at room temperature for 20 minutes. The solvent was removed under reduced pressure to afford the title compound (34 mg). MS (m/z): 473.4 [M+H]⁺

### Example 36

### 3-[2-(2-Aminopyrimidin-5-yl)ethynyl]-4-(difluoromethoxy)-N-[(1S)-2-hydroxy-1-phenylethyl]benzamide hydrochloride

3-[2-(2-Aminopyrimidin-5-yl)ethynyl]-4-(difluoromethoxy)-N-[(1S)-2-hydroxy-1-phenylethyl]benzamide (33 mg) was obtained following a method similar to that described in Example 1, using 3-[(2-aminopyrimidin-5-yl)ethynyl]-4-(difluoromethoxy)benzoic acid (30 mg) obtained in Step 2 of Reference Example 5 and (2S)-2-amino-2-phenylethanol (16 mg) instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. The obtained 3-[2-(2-aminopyrimidin-5-yl)ethynyl]-N-[(2S)-1-(4-chlorophenyl)-3-hydroxypropan-2-yl]-4-(difluoromethoxy)benzamide (33 mg) was dissolved in 1,4-dioxane (2 mL), hydrogen chloride (4 M in 1,4-dioxane, 0.020 mL) was added, and the mixture was stirred at room temperature for 20 minutes. The solvent was removed under reduced pressure to give the title compound (20 mg). MS (m/z): 425.5 [M+H]⁺

### Example 37

### 3-[(1Z)-2- (2-Aminopyrimidin-5-yl)-2-fluoroethenyl]-N-[(1S,2S)-5,5-difluoro-2-hydroxycyclohexyl]-4-(difluoromethoxy)benzamide

The title compound (61 mg) was obtained following a method similar to that described in Example 1, using 3-[(1Z)-2-(2-aminopyrimidin-5-yl)-2-fluoroethenyl)-4-(difluoromethoxy)benzoic acid (60 mg) obtained in Step 2 of Reference Example 26 and (1S,2S)-2-amino-4,4-difluorocyclohexan-1-ol hydrochloride (42 mg) obtained in Step 6 of Reference Example 23 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 459.1 [M+H]⁺

### Example 38

### 3-[(1Z)-2-(2-Aminopyrimidin-5-yl)-2-fluoroethenyl]-4-(difluoromethoxy)-N-[(1S,2S)-2-hydroxycyclohexyl]benzamide

To a solution of 3-[(1Z)-2-(2-aminopyrimidin-5-yl)-2-fluoroethenyl]-4-(difluoromethoxy)benzoic acid (20 mg) obtained in Step 2 of Reference Example 26 in DMF (0.12 mL), HATU (28 mg) and DIPEA (0.032 mL) were added sequentially, and the mixture was stirred at room temperature for 5 minutes. Then, (1S,2S)-2-aminocyclohexanol hydrochloride (11 mg) was added, and the mixture was stirred at room temperature for 1.5 hours. Saturated aqueous sodium bicarbonate was added to the reaction solution, and the resulting precipitate was collected by filtration to afford the title compound (22 mg). MS (m/z): 423.3 [M+H]⁺

### Example 39

### 3-[(1Z)-2-(2-Aminopyrimidin-5-yl)-1-fluoroethenyl]-4-(difluoromethoxy)-N-[(1S,2S,4S)-2-hydroxy-4-(trifluoromethoxy)cyclopentyl]benzamide

The title compound (35 mg) was obtained following a method similar to that described in Example 27, using 3-[(Z)-2-(2-aminopyrimidin-5-yl)-1-fluoroethenyl]-4-(difluoromethoxy)benzoic acid (30 mg) obtained in Step 2 of Reference Example 15 and (1S,2S,4S)-2-amino-4-(trifluoromethoxy)cyclopentan-1-ol hydrochloride (25 mg) obtained in Step 4 of Reference Example 9 instead of 3-(3-carbamoylimidazo[1,5-a]pyridin-7-yl)-4-(trifluoromethyl)benzoic acid and (1S,2S)-2-amino-4,4-difluorocyclohexan-1-ol hydrochloride. MS (m/z): 493.1 [M+H]⁺

### Example 40

### 3-[(1Z)-2-(2-Aminopyrimidin-5-yl)-1-fluoroethenyl]-4-(difluoromethoxy)-N-[(5S,6S)-6-hydroxyspiro[2.4]heptan-5-yl]benzamide

The title compound (25 mg) was obtained following a method similar to that described in Example 1, using 3-[(Z)-2-(2-aminopyrimidin-5-yl)-1-fluoroethenyl]-4-(difluoromethoxy)benzoic acid (30 mg) obtained in Step 2 of Reference Example 15 and (5S,6S)-6-aminospiro[2.4]heptan-5-ol hydrochloride (18 mg) obtained in Step 2 of Reference Example 24 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 435.2 [M+H]⁺

### Example 41

### 5-{3-Carbamoylimidazo[1,5-a]pyridin-7-yl}-N-[(1S,2S)-5,5-difluoro-2-hydroxycyclohexyl]-6-(trifluoromethyl)pyridine-3-carboxamide

To a mixture of 5-(3-carbamoylimidazo[1,5-a]pyridin-7-yl)-6-(trifluoromethyl)pyridine-3-carboxylic acid (10 mg) obtained in Step 2 of Reference Example 20 and methylene chloride (0.095 mL), TEA (0.016 mL), EDCI-HCl (55 mg) and HOBt (4 mg) were added in this order and stirred at room temperature for 5 minutes. Then, (1S,2S)-2-amino-4,4-difluorocyclohexan-1-ol hydrochloride (7 mg) obtained in Step 6 of Reference Example 23 was added and stirred at room temperature overnight. The reaction solution was purified by silica gel column chromatography to afford the title compound (12 mg). MS (m/z): 484.3 [M+H]⁺

### Example 42

### 5-(Cyclopropylmethoxy)-N-(2-ethyl-5-{[(1S,2S,4S)-2-hydroxy-4-(trifluoromethoxy)cyclopentyl]carbamoyl}phenyl)pyridine-3-carboxamide

The title compound (35 mg) was obtained following a method similar to that described in Example 1, using 3-[5-(cyclopropylmethoxy)pyridine-3-amido]-4-ethylbenzoic acid (30 mg) obtained in Step 2 of Reference Example 27, instead of 4-(difluoromethoxy)-3-[(pyrimidin-5-yl)ethynyl]benzoic acid. MS (m/z): 508.3 [M+H]⁺

### Example 43

### 5-(Cyclopropylmethoxy)-N-(2-ethyl-5-{[(2S)-1-hydroxy-4-(trifluoromethoxy)butan-2-yl] carbamoyl}phenyl) pyridine -3-carboxamide

The title compound (29 mg) was obtained following a method similar to that described in Example 1, using 3-[5-(cyclopropylmethoxy)pyridin-3-amido]-4-ethylbenzoic acid (30 mg) obtained in Step 2 of Reference Example 27 and (2S)-2-amino-4-(trifluoromethoxy)butan-1-ol hydrochloride (22 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 496.2 [M+H]⁺

### Example 44

### 2-[(Cyclopropylmethyl)amino]-N-(2-ethyl-5-{[(2S)-1-hydroxy-4-(trifluoromethoxy)butan-2-yl]carbamoyl}phenyl)-1,3-thiazole-5-carboxamide

The title compound (17 mg) was obtained following a method similar to that described in Example 1, using 3-{2-[(cyclopropylmethyl)amino]-1,3-thiazol-5-amido}-4-ethylbenzoic acid (20 mg) obtained in Step 3 of Reference Example 28 and (2S)-2-amino-4-(trifluoromethoxy)butan-1-ol hydrochloride (13 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 501.3 [M+H]⁺

### Example 45

### 2-[(Cyclopropylmethyl)amino]-N-{2-ethyl-5-{[(1S,2S,4S)-2-hydroxy-4-(trifluoromethoxy)cyclopentyl]carbamoyl}phenyl)-1,3-thiazole-5-carboxamide

The title compound (23 mg) was obtained following a method similar to that described in Example 1, using 3-{2-[(cyclopropylmethyl)amino]-1,3-thiazole-5-amido}-4-ethylbenzoic acid (20 mg) obtained in Step 3 of Reference Example 28, instead of 4-(difluoromethoxy)-3-[(pyrimidin-5-yl)ethynyl]benzoic acid. MS (m/z): 513.3 [M+H]⁺

### Example 46

### 2-Cyclopropyl-N-(2-ethyl-5-{[(1S,2S,4S)-2-hydroxy-4-(trifluoromethoxy)cyclopentyl]carbamoyl}phenyl)-1,3-thiazole-5-carboxamide

The title compound (29 mg) was obtained following a method similar to that described in Example 1, using 3-(2-cyclopropyl-1,3-thiazole-5-amido)-4-ethylbenzoic acid (30 mg) obtained in Step 2 of Reference Example 29, instead of 4-(difluoromethoxy)-3-[(pyrimidin-5-yl)ethynyl]benzoic acid. MS (m/z): 484.3 [M+H]⁺

### Example 47

### 2-(Cyclopropylmethyl)-N-(2-ethyl-5-{[(2S)-1-hydroxy-4-(trifluoromethoxy)butan-2-yl]carbamoyl}phenyl)-1,3-thiazole-5-carboxamide

The title compound (27 mg) was obtained following a method similar to that described in Example 1, using 3-{[2-(cyclopropylmethyl)-1,3-thiazole-5-carbonyl]amino]-4-ethylbenzoic acid (30 mg) obtained in Step 2 of Reference Example 22 and (2S)-2-amino-4-(trifluoromethoxy)butan-1-ol hydrochloride (23 mg) obtained in Step 3 of Reference Example 12 instead of 4-(difluoromethoxy)-3-[(pyrimidin-5-yl)ethynyl]benzoic acid and (1S,2S,4S)-2-amino-4-(trifluoromethoxy)cyclopentan-1-ol hydrochloride. MS (m/z): 486.1 [M+H]⁺

The structural formulas of Examples 1 to 47 are shown in the following tables.

**[Table 1]**

| Example | structural formula |
|---|---|
| 1 | 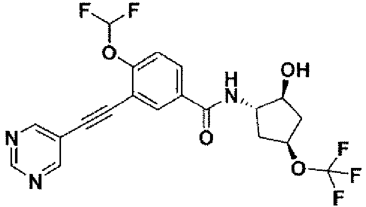 |
| 2 | 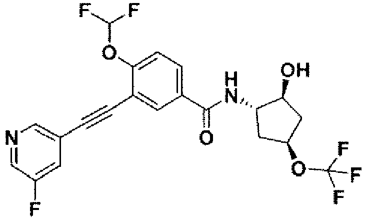 |
| 3 | 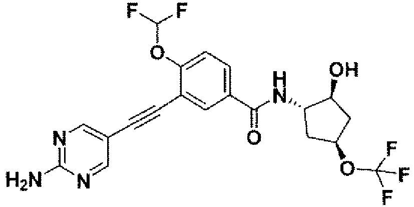 |
| 4 | 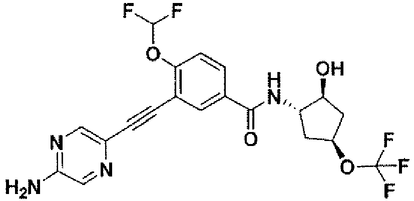 |
| 5 | 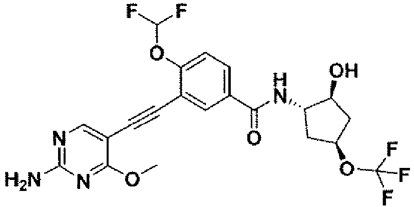 |

**[Table 2]**

| Example | structural formula |
|---|---|
| 6 | 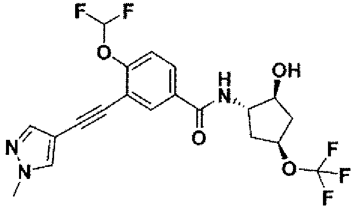 |
| 7 | 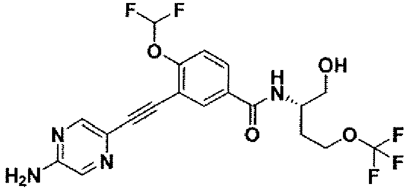 |
| 8 | 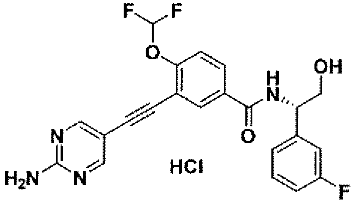 |
| 9 | 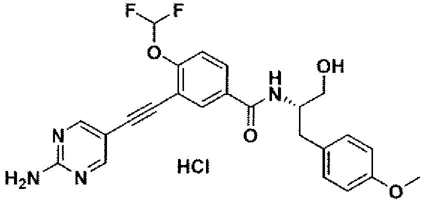 |
| 10 | 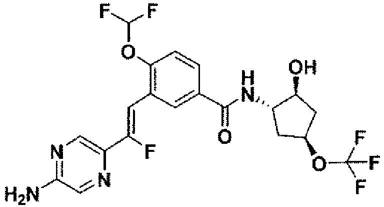 |

**[Table 3]**

| Example | structural formula |
|---|---|
| 11 | 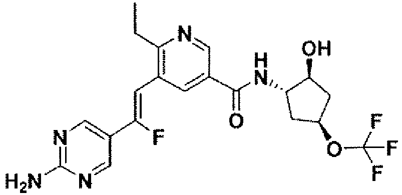 |
| 12 | 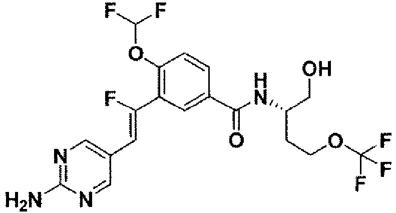 |
| 13 | 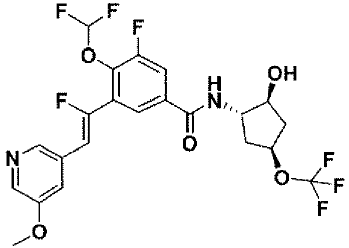 |
| 14 | 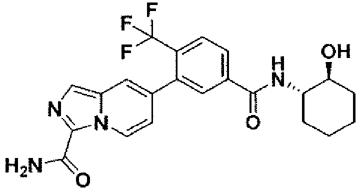 |
| 15 | 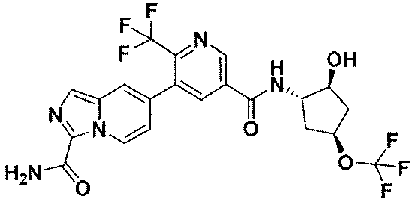 |

**[Table 4]**

| Example | structural formula |
|---|---|
| 16 | 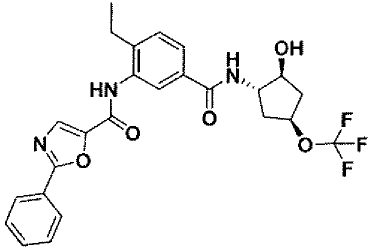 |
| 17 | 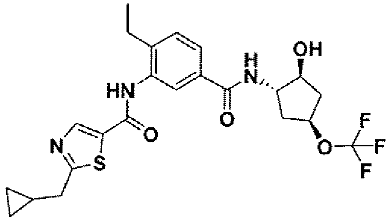 |
| 18 | 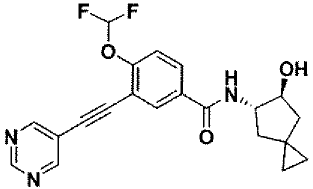 |
| 19 | 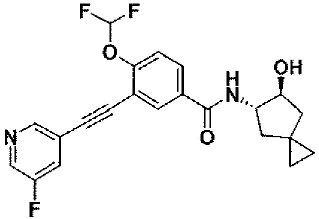 |
| 20 | 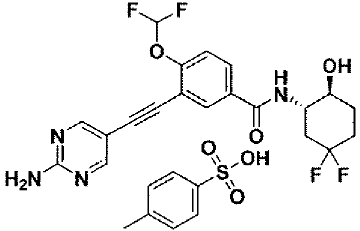 |

**[Table 5]**

| Example | structural formula |
|---|---|
| 21 | 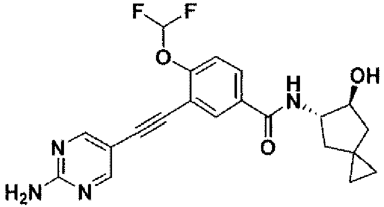 |
| 22 | 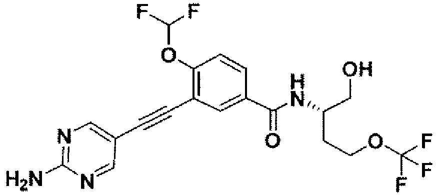 |
| 23 | 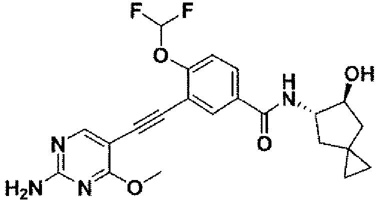 |
| 24 | 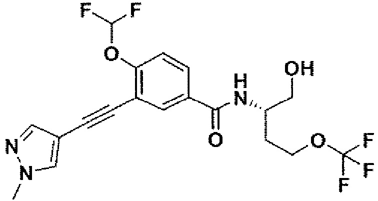 |
| 25 | 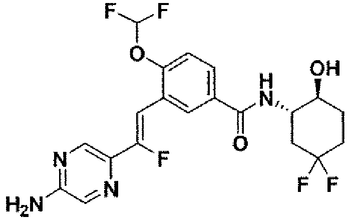 |

**[Table 6]**

| Example | structural formula |
|---|---|
| 26 | 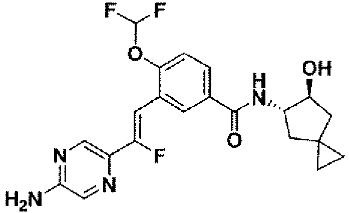 |
| 27 | 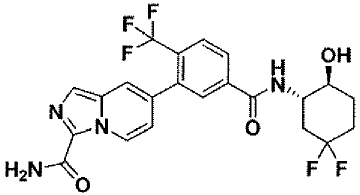 |
| 28 | 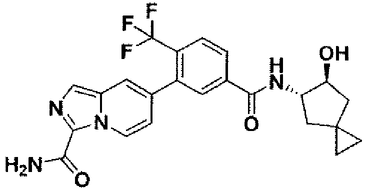 |
| 29 | 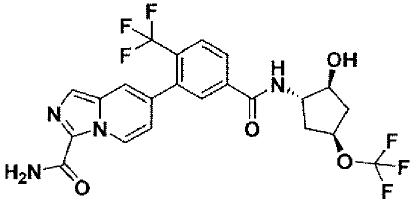 |
| 30 | 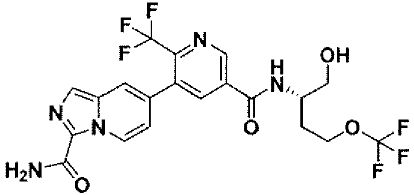 |

**[Table 7]**

| Example | structural formula |
|---|---|
| 31 | 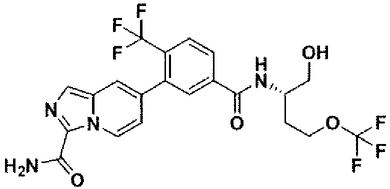 |
| 32 | 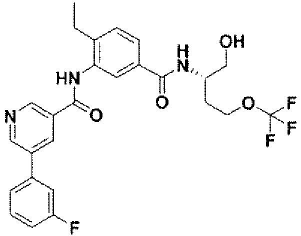 |
| 33 | 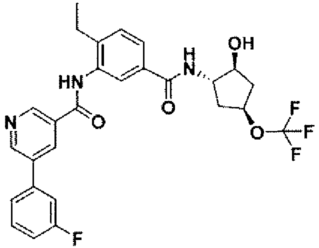 |
| 34 | 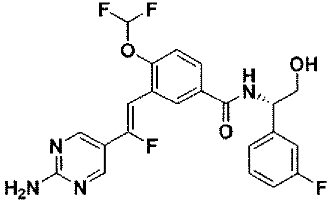 |
| 35 | 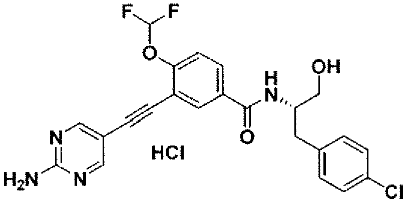 |

**[Table 8]**

| Example | structural formula |
|---|---|
| 36 | 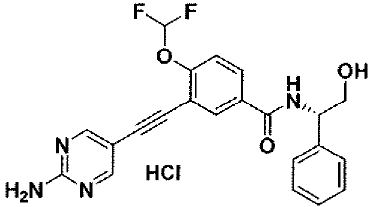 |
| 37 | 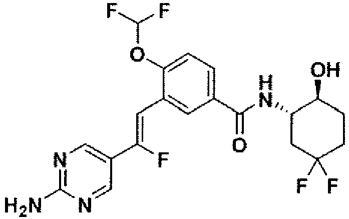 |
| 38 | 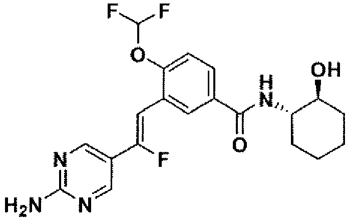 |
| 39 | 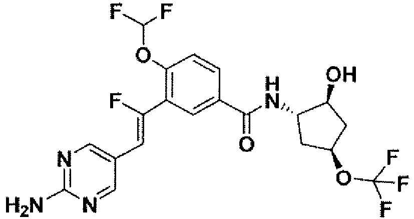 |
| 40 | 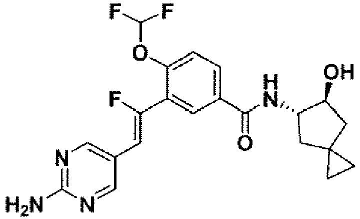 |

**[Table 9]**

| Example | structural formula |
|---|---|
| 41 | 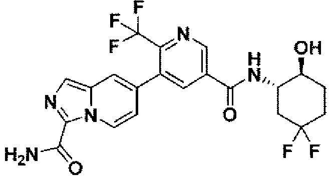 |
| 42 | 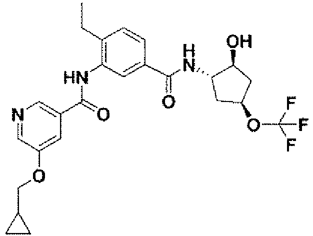 |
| 43 | 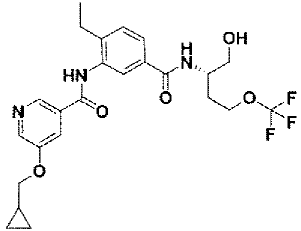 |
| 44 | 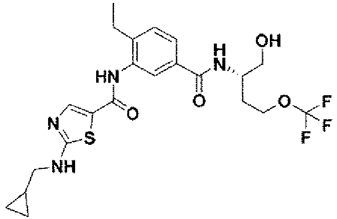 |
| 45 | 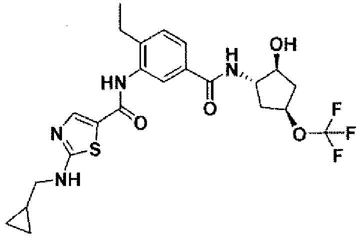 |

**[Table 10]**

| Example | structural formula |
|---|---|
| 46 | 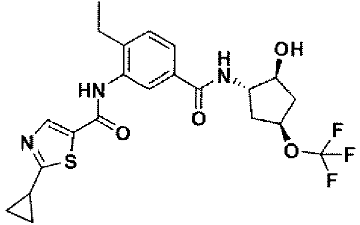 |
| 47 | 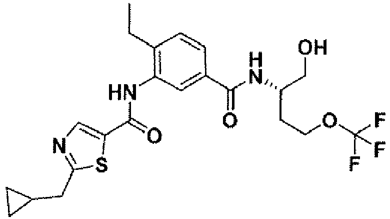 |

Examples of biological tests of the compounds of the invention are provided below.

### <Test Example 1: Evaluation of DDR1 kinase inhibitory activity>

### 1. Preparation of test samples

Test compounds in dimethyl sulfoxide (DMSO, Nacalai Tesque, 13445-74) were prepared at 10 mM, and six concentrations were selected for each compound, which were then diluted with DMSO to final concentrations of 1000, 100, 10, 1, 0.1, 0.01, and 0.001 µM. These solutions were diluted with a buffer containing 50 mM HEPES (Fujifilm Wako Pure Chemical Industries, 340-01371), 1 mM EGTA (DOJINDO, G002), 10 mM magnesium chloride hexahydrate (Nacalai Tesque, 20908-65), 2 mM dithiothreitol (Nacalai Tesque, 14112-52), and 0.01% Tween 20 (Tokyo Chemical Industry, T0543) to 330,000, 33,000, 330, 33, 3, 0.3, and 0.03 nM for each compound with six concentrations selected to obtain test sample solutions.

### 2. Measurement of inhibitory effect on kinase activity of DDR1 kinase

The test sample solutions as prepared at six concentrations for each compound were added to a 384-well plate (Corning, 4512) at 3 µL/well to make the final concentrations at 10,000, 1,000, 100, 10, 1, 0.1, and 0.01 nM. DDR1 protein (Carna Biosciences, 08-113) was added at 2 µL/well to make the final concentration at 0.5 nM. As substrates, LANCE Ultra ULight^{™}-poly GAT (PerkinElmer, TRF0101) and ATP (Sigma-Aldrich, A6559) were mixed to give the final concentrations at 50 nM and 100 µM, respectively, and added at 5 µL/well. After 2 hours of incubation at room temperature, 10 µL/well of LANCE Detection Buffer (PerkinElmer, CR97-100) containing EDTA (DOJINDO, K001) at 10 mM of the final concentration and Europium (PerkinElmer, AD0068) at 1 nM of the final concentration was added. After 1 hour of incubation at room temperature, the fluorescence intensity at 615 nm and 665 nm was measured using Envision (PerkinElmer) or SpectraMax iD5 (Molecular Devices). The test was performed in duplicate.

### 3. Analysis of the measurement results

The fluorescence intensity at 665 nM was divided by the fluorescence intensity at 615 nM to calculate the inhibition rate of each test compound, with the maximum inhibition rate set to 100% and the minimum inhibition rate set to 0%. The IC₅₀ value (50% inhibition concentration) of the test compound was calculated by logistic regression analysis. The results for each Example compound are shown in the following table.

**[Table 11]**

| Example | DDR-1 IC₅₀ (nM) | Example | DDR-1 IC₅₀ (nM) |
|---|---|---|---|
| 1 | 7 | 25 | 1.7 |
| 2 | 3.6 | 26 | 5.1 |
| 3 | 0.86 | 27 | 4 |
| 4 | 0.56 | 28 | 0.86 |
| 5 | 0.25 | 29 | 0.81 |
| 6 | 0.39 | 30 | 18 |
| 7 | 4.4 | 31 | 2.2 |
| 8 | 6.4 | 32 | 26 |
| 9 | 2.9 | 33 | 1.8 |
| 10 | 1.2 | 34 | 8.2 |
| 11 | 22 | 35 | 3.3 |
| 12 | 1.1 | 36 | 8.6 |
| 13 | 0.87 | 37 | 15 |
| 14 | 2.5 | 38 | 12 |
| 15 | 2.2 | 39 | 0.39 |
| 16 | 7.5 | 40 | 0.48 |
| 17 | 1.6 | 41 | 60 |
| 18 | 22 | 42 | 1.6 |
| 19 | 3.2 | 43 | 68 |
| 20 | 3.1 | 44 | 9.4 |
| 21 | 0.27 | 45 | 1.3 |
| 22 | 0.82 | 46 | 9.1 |
| 23 | 0.25 | 47 | 51 |
| 24 | 36 | | |

### <Test Example 2: Evaluation of DDR1 kinase inhibitory effect in DDR1-expressing U2OS cells>

### 1. Preparation of test samples

Test compounds-in DMSO were prepared at 10 mM and diluted with DMSO to final concentrations of 1000, 100, 10, 1, and 0.1 µM. These solutions were diluted further with E-MEM medium (Fujifilm Wako Pure Chemical Industries, 051-07615) containing 10% fetal bovine serum (FBS, Gibco, 10270106) to 60,000, 6,000, 600, 60, 6, and 0.6 nM to obtain test sample solutions.

### 2. Measurement of DDR1 kinase inhibitory effect in DDR1-expressing U2OS cells

DDR1-expressing U2OS cells (DiscoverX, 93-0894C3) suspended at 2.5 x 10⁵ cells/mL in 10% FBS + E-MEM medium were seeded in 384-well plates (DiscoverX, 92-0013) in 20 µL portions and cultured overnight at 37°C and 5% CO₂.

The next day, the test sample solutions as prepared were added at 5 µL/well to final concentrations of 10,000, 1,000, 100, 10, 1, and 0.1 nM. After incubation at 37°C and 5% CO₂ for 1 hour, type I collagen (Nitta Gelatin, 631-00771) was added at 5 µL/well to a final concentration of 50 µg/mL, and cultured overnight at 37°C and 5% CO₂.

The next day, the plate was left at room temperature for 1 hour, after which 5 µL/well of detection solution was added according to the instruction manual for the PathHunter (registered trademark) Detection Kit (DiscoverX, 93-0001). After incubation at room temperature for 2 hours, the luminescence intensity was measured using Envision (PerkinElmer). The test was performed in duplicate.

### 3. Analysis of the measurement results

The inhibition rate of the test compound was calculated by setting the blank (with only type I collagen stimulation) as 100% and the negative control group (without type I collagen stimulation) as 0%. The IC₅₀ value (50% inhibitory concentration) of the test compound was calculated by logistic regression analysis. The results of each Example compound are shown in the table below.

**[Table 12]**

| Example | DDR-1 IC₅₀ (nM) | Example | DDR- 1 IC₅₀ (nM) |
|---|---|---|---|
| 1 | 29 | 25 | 19 |
| 2 | 38 | 26 | 20 |
| 3 | 3.6 | 27 | 10 |
| 4 | 18 | 28 | 7.8 |
| 5 | 1.8 | 29 | 3.1 |
| 6 | 58 | 30 | 78 |
| 7 | 50 | 31 | 5.3 |
| 8 | 16 | 32 | 54 |
| 9 | 25 | 33 | 7.4 |
| 10 | 72 | 34 | 44 |
| 11 | 120 | 35 | 12 |
| 12 | 14 | 36 | 230 |
| 13 | 1.9 | 37 | 40 |
| 14 | 83 | 38 | 99 |
| 15 | 14 | 39 | 3.9 |
| 16 | 13 | 40 | 5.6 |
| 17 | 17 | 41 | 350 |
| 18 | 150 | 42 | 54 |
| 19 | 27 | 43 | 240 |
| 20 | 16 | 44 | 15 |
| 21 | 11 | 45 | 6.8 |
| 22 | 17 | 46 | 44 |
| 23 | 4.1 | 47 | 59 |
| 24 | 97 | | |

### <Test Example 3: In vivo efficacy study using anti-glomerular basement membrane antibody-induced nephritis model>

### 1. Preparation of test sample

The test compound was weighed and suspended in 0.5% methylcellulose (Shin-Etsu Chemical Co., Ltd.) + 99.5% Otsuka distilled water (Otsuka Pharmaceutical Factory) to prepare a test sample solution for administration.

### 2. Preparation of animal model

300 µL of phosphate buffered saline (Wako, 045-29795) containing 33.3 µg/mL anti-glomerular basement membrane antibody (Chondrex, 70221) was intravenously administered into the tail vein of WKY/NCrlCrlj rats (7-week-old males). 7 days after, the test sample was administered twice a day for 7 days, and the rats were moved to a metabolism/urine collection cage from the 6th to 7th days of the starting of the administration, and the 24-hour urine was collected. The weight of the collected urine was measured.

### 3. Measurement of urinary albumin amount

150 µL of urine was transferred to a sample cup (650-M, BMBio ST). The albumin concentration in urine was measured using a JCA-BM6050 automatic analyzer (JEOL). The amount of albumin in urine was determined with correction for the measured urine weight.

### 4. Analysis of measurement results

The amounts of albumin in urine in the group without administration of anti-glomerular basement membrane antibody and the group with administration of vehicle were set at 0% and 100%, respectively, and the ratio of the amount of albumin in urine in the group administered with the test compound was calculated. The experimental results are shown in the table below.

**[Table 13]**

| Example | Dose (mg/kg) | Urinary albumin (% of vehicle-administered group) |
|---|---|---|
| 1 | 50 | 70 |
| 3 | 50 | 78 |
| 4 | 25 | 69 |
| 5 | 10 | 57 |
| 6 | 5 | 72 |
| 7 | 30 | 69 |
| 10 | 10 | 69 |
| 14 | 50 | 76 |

## Claims

1. A compound of the formula [1]: 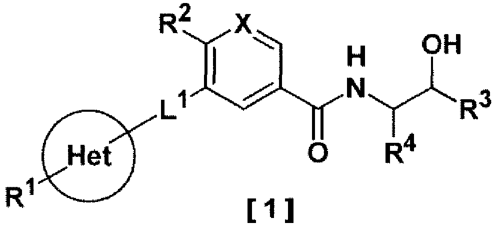 or a pharmaceutically acceptable salt thereof, or a solvate thereof,
wherein
R¹ is one or two groups selected from the group consisting of hydrogen, halogen, an optionally-substituted alkyl, an optionally-substituted amino, an optionally-substituted alkoxy, an optionally-substituted aryl, haloalkyl, cycloalkyl, an optionally-substituted sulfonyl, cyano, an optionally-substituted carbamoyl, heteroaryl and dialkylphosphoryl;
Het is a 5- to 10-membered heteroaryl;
L¹ is a bond, -CO-NH-, -CR^{a}=CR^{b}- or -C=C-, and the carbonyl group (C=O) in -CO-NH- is bonded to Het and -CR^{a} in -CR^{a}=CR^{b}-is bonded to Het;
R^{a} is hydrogen or halogen;
R^{b} is hydrogen or halogen;
X is N or C-R^{c} wherein R^{c} is hydrogen or halogen;
R² is hydrogen, ethyl, C₁-C₆ haloalkoxy or C₁-C₆ haloalkyl; and
R³ is hydrogen, and R⁴ is hydrogen, an optionally-substituted phenyl or an optionally-substituted alkyl, or
R³ and R⁴ are taken together with the carbon atoms to which they are attached to form an optionally-substituted C₃-C₈ cycloalkyl.

2. The compound or a pharmaceutically acceptable salt thereof, or a solvate thereof, according to Claim 1 wherein
R¹ is one or two groups selected from the group consisting of hydrogen, halogen, an optionally-substituted alkyl, an optionally-substituted amino, an optionally-substituted alkoxy, an optionally-substituted aryl, haloalkyl and an optionally-substituted carbamoyl;
Het is a 5- to 10-membered heteroaryl;
L¹ is a bond, -CR^{a}=CR^{b}-, -CO-NH- or -C=C-, and the carbonyl group (C=O) in -CO-NH- is bonded to Het and -CR^{a} in -CR^{a}=CR^{b}-is bonded to Het;
R^{a} is hydrogen or halogen;
R^{b} is hydrogen or halogen;
X is N or C-R^{c} wherein R^{c} is hydrogen or halogen;
R² is ethyl, C₁-C₆ haloalkoxy or C₁-C₆ haloalkyl; and
R³ is hydrogen, and R⁴ is hydrogen, an optionally-substituted phenyl or an optionally-substituted alkyl, or
R³ and R⁴ are taken together with the carbon atoms to which they are attached to form an optionally-substituted C₃-C₈ cycloalkyl.

3. The compound or a pharmaceutically acceptable salt thereof, or a solvate thereof, according to Claim 1 wherein
R¹ is one or two groups selected from the group consisting of hydrogen, an optionally-substituted alkyl, an optionally-substituted amino and an optionally-substituted carbamoyl;
Het is a 5- to 10-membered heteroaryl;
L¹ is a bond, -CR^{a}=CR^{b}- or -C=C-, and -CR^{a} in -CR^{a}=CR^{b}- is bonded to Het;
R^{a} is hydrogen;
R^{b} is halogen;
X is N or C-R^{c} wherein R^{c} is hydrogen;
R² is C₁-C₆ haloalkoxy or C₁-C₆ haloalkyl; and
R³ and R⁴ are taken together with the carbon atoms to which they are attached to form an optionally-substituted C₃-C₈ cycloalkyl.

4. The compound or a pharmaceutically acceptable salt thereof, or a solvate thereof, according to Claim 1 wherein
R¹ is one or two groups selected from the group consisting of hydrogen, alkyl, amino and carbamoyl;
Het is pyrimidinyl, pyridyl, pyrazinyl, pyrazolyl, pyridinyl, imidazo[1,5-a]pyridyl, oxazolyl, thiazolyl or pyrazyl;
L¹ is a bond, -CR^{a}=CR^{b}- or -C≡C-, and -CR^{a} in -CR^{a}=CR^{b}- is bonded to Het;
R^{a} is hydrogen;
R^{b} is halogen;
X is N or C-R^{c} wherein R^{c} is hydrogen;
R² is C₁-C₆ fluoroalkoxy, C₁-C₆ difluoroalkoxy, C₁-C₆ trifluoroalkoxy, C₁-C₆ fluoroalkyl, C₁-C₆ difluoroalkyl or C₁-C₆ trifluoroalkyl; and
R³ and R⁴ are taken together with the carbon atoms to which they are attached to form an optionally-substituted cyclopentyl, an optionally-substituted cyclohexyl, or an optionally-substituted spiro[2.4]heptanyl.

5. The compound or a pharmaceutically acceptable salt thereof, or a solvate thereof, according to Claim 1 wherein
R¹ is a group selected from the group consisting of hydrogen, alkyl, amino and carbamoyl;
Het is pyrimidinyl, pyridyl, pyrazinyl, pyrazolyl, pyridinyl, imidazo[1,5-a]pyridyl, oxazolyl, thiazolyl or pyrazyl;
L¹ is a bond, -CR^{a}=CR^{b}- or -C≡C-, and -CR^{a} in -CR^{a}=CR^{b}- is bonded to Het;
R² is hydrogen;
R^{b} is halogen;
X is N or C-R^{c} wherein R^{c} is hydrogen;
R² is C₁-C₆ fluoroalkoxy, C₁-C₆ difluoroalkoxy, C₁-C₆ trifluoroalkoxy, C₁-C₆ fluoroalkyl, C₁-C₆ difluoroalkyl or C₁-C₆ trifluoroalkyl; and
R³ and R⁴ are taken together with the carbon atoms to which they are attached to form cyclopentyl substituted with one or two groups selected from the group consisting of halogen and haloalkoxy, cyclohexyl substituted with one or two groups selected from the group consisting of halogen and haloalkoxy, or spiro[2.4]heptanyl substituted with one or two groups selected from the group consisting of halogen and haloalkoxy.

6. The compound according to Claim 1 selected from the group consisting of the following compounds (1) to (47):
(1) 4-(difluoromethoxy)-N-[(1S,2S,4S)-2-hydroxy-4-(trifluoromethoxy)cyclopentyl]-3-[(pyrimidin-5-yl)ethynyl]benzamide,
(2) 4-(difluoromethoxy)-3-[2-(5-fluoropyridin-3-yl)ethynyl]-N-[(1S,2S, 4S)-2-hydroxy-4-(trifluoromethoxy)cyclopentyl]benzamide,
(3) 3-[2-(2-aminopyrimidin-5-yl)ethynyl]-4-(difluoromethoxy)-N-[(1S,2S,4S)-2-hydroxy-4-(trifluoromethoxy)cyclopentyl]benzamide,
(4) 3-[2-(5-aminopyrazin-2-yl)ethynyl]-4-(difluoromethoxy)-N-[(1S,2S,4S)-2-hydroxy-4-(trifluoromethoxy)cyclopentyl]benzamide,
(5) 3-[2-(2-amino-4-methoxypyrimidin-5-yl)ethynyl]-4-(difluoromethoxy)-N-[(1S,2S,4S)-2-hydroxy-4-(trifluoromethoxy)cyclopentyl]benzamide,
(6) 4-(difluoromethoxy)-N-[(1S,2S,4S)-2-hydroxy-4-(trifluoromethoxy)cyclopentyl]-3-[2-(1-methyl-1H-pyrazol-4-yl)ethynyl]benzamide,
(7) 3-[2-(5-aminopyrazin-2-yl)ethynyl]-4-(difluoromethoxy)-N-[(2S)-1-hydroxy-4-(trifluoromethoxy)butan-2-yl]benzamide,
(8) 3-[2-(2-aminopyrimidin-5-yl)ethynyl]-4-(difluoromethoxy)-N-[(1S)-1-(3-fluorophenyl)-2-hydroxyethyl]benzamide,
(9) 3-[2-(2-aminopyrimidin-5-yl)ethynyl]-4-(difluoromethoxy)-N-[(2S)-1-hydroxy-3-(4-methoxyphenyl)propan-2-yl]benzamide,
(10) 3-[(1Z)-2-(5-aminopyrazin-2-yl)-2-fluoroethenyl]-4-(difluoromethoxy)-N-[(15,2S,4S)-2-hydroxy-4-(trifluoromethoxy)cyclopentyl]benzamide,
(11) 5-[(1Z)-2-(2-aminopyrimidin-5-yl)-2-fluoroethenyl]-6-ethyl-N-[(1S,2S,4S)-2-hydroxy-4-(trifluoromethoxy)cyclopentyl]pyridine-3-carboxamide,
(12) 3-[(1Z)-2-(2-aminopyrimidin-5-yl)-1-fluoroethenyl]-4-(difluoromethoxy)-N-[(2S)-1-hydroxy-4-(trifluoromethoxy)butan-2-yl]benzamide,
(13) 4-(difluoromethoxy)-3-fluoro-5-[(1Z)-1-fluoro-2-(5-methoxypyridin-3-yl)ethenyl}-N-[(1S,2S,4S)-2-hydroxy-4-(trifluoromethoxy)cyclopentyl]benzamide,
(14) 7-(5-{[(1S,2S)-2-hydroxycyclohexyl]carbamoyl}-2-(trifluoromethyl)phenyl)imidazo[1,5-a]pyridine-3-carboxamide,
(15) 5-{3-carbamoylimidazo[1,5-a]pyridin-7-yl}-N-[(1S,2S, 4S)-2-hydroxy-4-(trifluoromethoxy)cyclopentyl]-6-(trifluoromethyl)pyridine-3-carboxamide,
(16) N-(2-ethyl-5-{[(1S,2S, 4S)-2-hydroxy-4-(trifluoromethoxy)cyclopentyl]carbamoyl}phenyl)-2-phenyl-1,3-oxazole-5-carboxamide,
(17) 2-(cyclopropylmethyl)-N-(2-ethyl-5-{[(1S,2S,4S)-2-hydroxy-4-(trifluoromethoxy)cyclopentyl]carbamoyl}phenyl)-1,3-thiazole-5-carboxamide,
(18) 4-(difluoromethoxy)-N-[(5S,6S)-6-hydroxyspiro[2.4]heptan-5-yl]-3-[2-(pyrimidin-5-yl)ethynyl]benzamide,
(19) 4-(difluoromethoxy)-3-[2-(5-fluoropyridin-3-yl)ethynyl]-N-[(5S,6S)-6-hydroxyspiro[2.4]heptan-5-yl]benzamide,
(20) 3-[2-(2-aminopyrimidin-5-yl)ethynyl]-N-[(1S,2S)-5,5-difluoro-2-hydroxycyclohexyl]-4-(difluoromethoxy)benzamide,
(21) 3-[2-(2-aminopyrimidin-5-yl)ethynyl]-4-(difluoromethoxy)-N-[(5S,6S)-6-hydroxyspiro[2.4]heptan-5-yl]benzamide,
(22) 3-[2-(2-aminopyrimidin-5-yl)ethynyl]-4-(difluoromethoxy)-N-[(2S)-1-hydroxy-4-(trifluoromethoxy)butan-2-yl]benzamide,
(23) 3-[2-(2-amino-4-methoxypyrimidin-5-yl)ethynyl]-4-(difluoromethoxy)-N-[(5S,6S)-6-hydroxyspiro[2.4]heptan-5-yl]benzamide,
(24) 4-(difluoromethoxy)-N-[(2S)-1-hydroxy-4-(trifluoromethoxy)butan-2-yl]-3-[2-(1-methyl-1H-pyrazol-4-yl)ethynyl]benzamide,
(25) 3-[(1Z)-2-(5-aminopyrazin-2-yl)-2-fluoroethenyl]-N-[(1S,2S)-5,5-difluoro-2-hydroxycyclohexyl]-4-(difluoromethoxy)benzamide,
(26) 3-[(1Z)-2-(5-aminopyrazin-2-yl)-2-fluoroethenyl]-4-(difluoromethoxy)-N-[(5S,6S)-6-hydroxyspiro[2.4]heptan-5-yl]benzamide,
(27) 7-(5-{[(1S,2S)-5,5-difluoro-2-hydroxycyclohexyl]carbamoyl}-2-(trifluoromethyl)phenyl)imidazo[1,5-a]pyridine-3-carboxamide,
(28) 7-(5-([(5S,6S)-6-hydroxyspiro[2.4]heptan-5-yl]carbamoyl}-2-(trifluoromethyl)phenyl)imidazo[1,5-a]pyridine-3-carboxamide,
(29) 7-(5-{[(1S,2S,4S)-2-hydroxy-4-(trifluoromethoxy)cyclopentyl]carbamoyl}-2-(trifluoromethyl)phenyl)imidazo[1,5-a]pyridine-3-carboxamide,
(30) 5-{3-carbamoylimidazo[1,5-a]pyridin-7-yl}-N-[(2S)-1-hydroxy-4-(trifluoromethoxy)butan-2-yl]-6-(trifluoromethyl)pyridine-3-carboxamide,
(31) 7-(5-{[(2S)-1-hydroxy-4-(trifluoromethoxy)butan-2-yl]carbamoyl}-2-(trifluoromethyl)phenyl)imidazo[1,5-a]pyridine-3-carboxamide,
(32) N-(2-ethyl-5-{[(2S)-1-hydroxy-4-(trifluoromethoxy)butan-2-yl]carbamoyl}phenyl)-5-(3-fluorophenyl)pyridine-3-carboxamide,
(33) N-(2-ethyl-5-{[(1S,2S,4S)-2-hydroxy-4-(trifluoromethoxy)cyclopentyl]carbamoyl}phenyl)-5-(3-fluorophenyl)pyridine-3-carboxamide,
(34) 3-[(1Z)-2-(2-aminopyrimidin-5-yl)-2-fluoroethenyl]-4-(difluoromethoxy)-N-[(1S)-1-(3-fluorophenyl)-2-hydroxyethyl]benzamide,
(35) 3-[2-(2-aminopyrimidin-5-yl)ethynyl]-N-[(2S)-1-(4-chlorophenyl)-3-hydroxypropan-2-yl]-4-(difluoromethoxy)benzamide hydrochloride,
(36) 3-[2-(2-aminopyrimidin-5-yl)ethynyl]-4-(difluoromethoxy)-N-[(1S)-2-hydroxy-1-phenylethyl]benzamide hydrochloride,
(37) 3-[(1Z)-2-(2-aminopyrimidin-5-yl)-2-fluoroethenyl]-N-((1S,2S)-5,5-difluoro-2-hydroxycyclohexyl]-4-(difluoromethoxy)benzamide,
(38) 3-[(1Z)-2-(2-aminopyrimidin-5-yl)-2-fluoroethenyl]-4-(difluoromethoxy)-N-[(1S,2S)-2-hydroxycyclohexyl]benzamide,
(39) 3-[(1Z)-2-(2-aminopyrimidin-5-yl)-1-fluoroethenyl]-4-(difluoromethoxy)-N-((1S,2S,4S)-2-hydroxy-4-(trifluoromethoxy)cyclopentyl]benzamide,
(40) 3-[(1Z)-2-(2-aminopyrimidin-5-yl)-1-fluoroethenyl]-4-(difluoromethoxy)-N-[(5S,6S)-6-hydroxyspiro[2.4]heptan-5-yl]benzamide,
(41) 5-{3-carbamoylimidazo[1,5-a]pyridin-7-yl}-N-[(1S,2S)-5,5-difluoro-2-hydroxycyclohexyl]-6-(trifluoromethyl)pyridine-3-carboxamide,
(42) 5-(cyclopropylmethoxy)-N-(2-ethyl-5-{[(1S,2S,4S)-2-hydroxy-4-(trifluoromethoxy)cyclopentyl]carbamoyl}phenyl)pyridine-3-carboxamide,
(43) 5-(cyclopropylmethoxy)-N-(2-ethyl-5-{[(2S)-1-hydroxy-4-(trifluoromethoxy)butan-2-yl]carbamoyl}phenyl)pyridine-3-carboxamide,
(44) 2-[(cyclopropylmethyl)amino]-N-(2-ethyl-5-{[(2S)-1-hydroxy-4-(trifluoromethoxy)butan-2-yl]carbamoyl}phenyl)-1,3-thiazole-5-carboxamide,
(45) 2-[(cyclopropylmethyl)amino]-N-(2-ethyl-5-{[(1S,2S,4S)-2-hydroxy-4-(trifluoromethoxy)cyclopentyl]carbamoyl}phenyl)-1,3-thiazole-5-carboxamide,
(46) 2-cyclopropyl-N-(2-ethyl-5-{[(15,2S,4S)-2-hydroxy-4-(trifluoromethoxy)cyclopentyl]carbamoyl}phenyl)-1,3-thiazole-5-carboxamide, and
(47) 2-(cyclopropylmethyl)-N-(2-ethyl-5-{[(2S)-1-hydroxy-4-(trifluoromethoxy)butan-2-yl]carbamoyl}phenyl)-1,3-thiazole-5-carboxamide,
or a pharmaceutically acceptable salt thereof, or a solvate thereof.

7. A pharmaceutical composition containing the compound or a pharmaceutically acceptable salt thereof, or a solvate thereof, according to Claim 1, as an active ingredient.

8. A DDR1 kinase inhibiting agent containing the compound or a pharmaceutically acceptable salt thereof, or a solvate thereof, according to Claim 1, as an active ingredient.

9. A method for inhibiting DDR1 kinase, comprising administering the compound or a pharmaceutically acceptable salt thereof, or a solvate thereof, according to Claim 1, to a subject in need thereof.

10. A prophylactic or therapeutic agent for diseases in which DDR1 kinase is involved, containing the compound or a pharmaceutically acceptable salt thereof, or a solvate thereof, according to Claim 1.

11. A prophylactic or therapeutic agent for Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic renal failure, microvascular nephrotic syndrome, mesangial proliferative glomerulonephritis, membranous proliferative glomerulonephritis, semilunar glomerulonephritis, membranous nephropathy, pulmonary fibrosis, myelofibrosis, liver fibrosis, acute myeloid leukemia, chronic myeloid leukemia, acute lymphoid leukemia, chronic lymphoid leukemia, Hodgkin lymphoma, non-Hodgkin's lymphoma, glioma, non-small cell lung cancer, small cell lung cancer, breast cancer, ovarian cancer, prostate cancer, colon cancer or osteoarthritis, containing the compound or a pharmaceutically acceptable salt thereof, or a solvate thereof, according to Claim 1, as an active ingredient.
